# EUROPEAN PATENT APPLICATION

(11) **EP 4 397 748 A1**
(43) Date of publication of application: **10.07.2024**
(21) Application number: 22863447.3
(22) Date of filing: 30.08.2022
(51) Int. Cl.: C12N 1/20, C12N 15/62, C12N 7/01, C12N 5/10, C07K 16/10, A61K 39/42, A61P 31/18

(54) **GENE SEQUENCE CONSTRUCT FOR GENE THERAPY FOR HIV INFECTION**

(30) Priority: 30.08.2021 WO PCT/CN2021/115420
(71) Applicant: KANGLIN BIOTECHNOLOGY (HANGZHOU) CO., LTD., Hangzhou, Zhejiang 310018 (CN)
(72) Inventor: WU, Haoquan, Hangzhou, Zhejiang 310018 (CN); SUN, Baozhen, Hangzhou, Zhejiang 310018 (CN); DANG, Ying, Hangzhou, Zhejiang 310018 (CN)
(74) Representative: K&L Gates LLP
(86) International application number: PCT/CN2022/115822
(87) International publication number: WO 2023/030312

(57) **Abstract**

The present invention relates to a gene sequence construct for gene therapy for HIV infection. The gene sequence construct is constructed by means of sequentially connecting, via a coding sequence of a linker polypeptide, a gene coding sequence of a variable region scFv in each light chain and heavy chain of a monoclonal antibody against antigens at different binding sites involved in the different steps of HIV infection of a human CD4+ T cell, a gene coding sequence of a variable region scFv in each light chain and heavy chain of a monoclonal antibody bound to a human CD4 receptor site, a gene coding sequence of an Fc fragment in a human IgG constant region, and a gene coding sequence of a polypeptide for inhibiting the fusion of HIV and a CD4+ T cell membrane, and by placing the connected gene coding sequences downstream of a promoter and a secretion signal peptide coding sequence, thereby expressing a single-gene-encoded secretion-type antibody-like protein molecule. The recombinant single-gene construct can be conveniently introduced into a target tissue cell via a viral vector, and the expressed secretion-type antibody-like protein molecule has multi-antigen tropism, can realize the efficient and broad-spectrum blockade of the HIV infection process on a human CD4+ T cell by means of binding to multiple binding sites involved in the different steps of HIV infection of the human CD4+ T cell, and effectively avoids the loss of the ability to inhibit an HIV infection due to an HIV escape mutation, thereby achieving a long-term or even permanent treatment effect on HIV infection by means of a single injection.

## Description

This application claims priority of PCT patent application No. PCT/CN2021/115420 filed on August 30, 2021, the entire content of which is incorporated here as a part of this application.

### Technical field

The invention belongs to the field of gene therapy/biomedicine technology, and specifically relates to a gene sequence construct for gene therapy of HIV infection. The gene sequence construct can be used for gene therapy against HIV infection. The gene sequence construct can be used to express polytropic neutralizing antibody-like proteins with broad-spectrum and efficient HIV-neutralizing activity both in vivo and in vitro, and can be used for clinical research on gene therapy drugs for HIV infection delivered by recombinant viruses or non-viral vectors and for new drug research and development.

### Background of the Invention

AIDS (acquired immunodeficiency syndrome) caused by human immunodeficiency virus (HIV) infection is one of the most serious infectious diseases in the world today. Approximately 0.77 million of the 37.9 million HIV-infected people worldwide die of AIDS every year. With the development of anti-AIDS drugs, the expected survival period of actively treated HIV-infected patients has been greatly extended. However, the side effects and limitations of cocktail therapy, as well as the emerging drug-resistant strains of HIV, still causes long-term economic and social burdens, significant decline in the quality of life, and obvious suffering to the majority of AIDS patients.

Therefore, despite the progress that has been made in the anti-AIDS field, there is still a need to develop new drugs and methods for treating HIV infection.

### Summary of the invention

The present invention constructs a series of anti-HIV gene therapy constructs based on recombinant viral vectors. For example, single-chain antibody variable region fragments (scFv) of a variety of broad-spectrum neutralizing antibodies against HIV and a neutralizing antibody against human CD4 receptor are combined with the Fc fragment in the constant region of human antibodies and a HIV membrane fusion inhibitory polypeptide into a simple and efficient expression cassette.

Firstly, these gene sequence constructs for HIV infection gene therapy comprise one or more gene coding sequences of an antibody molecule with the ability to inhibit HIV infection and one or more gene coding sequences of a polypeptide (consisting of 2-50 amino acid residues) with the ability to inhibit HIV infection, to achieve the expression of a fusion protein molecule comprising the antibody molecule and the polypeptides against HIV infection encoded by a single gene, which has two or more than two target sites.

Specifically, the antibody molecule comprises a heavy chain constant region and/or a light chain constant region.

The heavy chain constant region comprises a heavy chain constant region of IgG1, IgG2, IgG3 or IgG4.

The light chain constant region comprises a light chain constant region of a kappa or lambda light chain.

The light chain variable region comprises the light chain variable region of a kappa or lambda light chain.

The above-mentioned gene sequence construct comprises two or more than two gene coding sequences of antibody molecules with the ability to inhibit HIV infection.

Alternatively, the gene sequence construct comprises three or more than three gene coding sequences of antibody molecules with the ability to inhibit HIV infection.

Alternatively, the gene sequence construct comprises four or more than four gene coding sequences of antibody molecules with the ability to inhibit HIV infection.

In addition, the above-mentioned gene sequence construct may comprise two or more than two gene coding sequences of polypeptides with the ability to inhibit HIV infection. These polypeptides consist of 2-50 amino acid residues.

Alternatively, the gene sequence construct comprises three or more than three gene coding sequences of polypeptides with the ability to inhibit HIV infection.

Alternatively, the gene sequence construct comprises four or more than four gene coding sequences of polypeptides with the ability to inhibit HIV infection.

In the gene sequence construct described in any one of the above, the fusion protein molecule comprising the antibody molecule and the polypeptide against HIV infection encoded by the single gene has three or more than three target sites.

Alternatively, the fusion protein molecule comprising the antibody molecule and the polypeptide against HIV infection encoded by the single gene has four or more than four targets.

Alternatively, the fusion protein molecule comprising the antibody molecule and the polypeptide against HIV infection encoded by the single gene has five or more than five targets.

Alternatively, the fusion protein molecule comprising the antibody molecule and the polypeptide against HIV infection encoded by the single gene has six or more than six targets.

The gene sequence construct described in any one of the above comprises two or more than two gene coding sequences of antibody molecules with the ability to inhibit HIV infection and one or more gene coding sequences of the polypeptide with the ability to inhibit HIV infection.

In the gene sequence construct described above, the fusion protein molecule comprising the antibody molecule and the polypeptide against HIV infection encoded by the single gene has three or more than three target sites.

In the gene sequence construct described in any one of the above, the gene coding sequence of a single-chain antibody molecule without a constant region with the ability to inhibit HIV infection and the gene coding sequence of the polypeptide with the ability to inhibit HIV infection are directly or indirectly concatenated via a coding sequence of a linker polypeptide.

The gene sequence construct described in any one of the above comprises two or more than two gene coding sequences of antibody molecules with the ability to inhibit HIV infection, and the gene coding sequences of the antibody molecules are directly or indirectly concatenated via a coding sequence of a linker polypeptide.

The gene sequence construct described in any one of the above comprises two or more than two gene coding sequences of polypeptides with the ability to inhibit HIV infection, and the gene coding sequences of the polypeptides are directly or indirectly concatenated via a coding sequence of a linker polypeptide.

In the gene sequence construct of any one of the above, the one or more gene coding sequence of the antibody molecule with the ability to inhibit HIV infection comprises a gene coding sequence of an anti-HIV-1-gp160 (including its cleavage products gp120 and gp41) antibody molecule.

In the gene sequence construct of any one of the above, the one or more gene coding sequence of the antibody molecule with the ability to inhibit HIV infection comprises a gene coding sequence of an antibody molecule that binds to human CD4 receptor site.

In the gene sequence construct of any one of the above, the one or more gene coding sequence of the polypeptide with the ability to inhibit HIV infection comprises a gene coding sequence of a polypeptide that inhibits the fusion of HIV and CD4+ T cell membranes.

The gene sequence construct described in any one of the above comprises two or more than two gene coding sequences of antibody molecules with the ability to inhibit HIV infection and one or more gene coding sequences of polypeptides with the ability to inhibit HIV infection, and the two or more than two gene coding sequences of the antibody molecules with the ability to inhibit HIV infection comprise a gene coding sequences of an antibody molecule against HIV-1-gp160 (including its cleavage products gp120 and gp41) and a gene coding sequence of an antibody molecule that binds to human CD4 receptor site, and the one or more gene coding sequences of polypeptides with the ability to inhibit HIV infection comprise a gene coding sequence of a polypeptide that inhibits the fusion of HIV and CD4+ T cell membranes.

The gene sequence construct described in any one of the above comprises (i) gene coding sequences of light chain complementarity determining regions (LCDR1, LCDR2 and LCDR3) and heavy chain complementarity determining regions (HCDR1, HCDR2 and HCDR3) of an anti-HIV-1-gp160 (including its cleavage products gp120 and gp41) monoclonal antibody, (ii) gene coding sequences of light chain complementarity determining regions (LCDR1, LCDR2 and LCDR3) and heavy chain complementarity determining regions (HCDR1, HCDR2 and HCDR3) of a monoclonal antibody that binds to human CD4 receptor site, (iii) a gene coding sequence of human IgG constant region Fc fragment, and (iv) a gene coding sequence of a short peptide that inhibits the fusion of HIV and CD4+ T cell membranes, in which the coding sequences of the light chain and the heavy chain of the antibodies can be directly or indirectly concatenated via a coding sequence of a linker polypeptide in any order.

The gene sequence construct described in any one of the above comprises (i) gene coding sequences of light chain variable region (VL) and heavy chain variable region (VH) of an anti-HIV-1-gp160 (including its cleavage products gp120 and gp41) monoclonal antibody, (ii) gene coding sequences of light chain variable region (VL) and heavy chain variable region (VH) of a monoclonal antibody that binds to human CD4 receptor site, (iii) a gene coding sequence of human IgG constant region Fc fragment, and (iv) a gene coding sequence of a short peptide that inhibits the fusion of HIV and CD4+ T cell membranes, in which the coding sequences of the light chain variable region (VL) and the heavy chain variable region (VH) of the antibodies can be directly or indirectly concatenated via a coding sequence of a linker polypeptide in any order.

The gene sequence construct described in any one of the above further comprises a promoter located upstream of the gene coding sequence of the antibody molecule with the ability to inhibit HIV infection and the gene coding sequence of the polypeptide with the ability to inhibit HIV infection.

The gene sequence construct described in any one of the above further comprises a secretion signal peptide coding sequence located upstream of the gene coding sequence of the antibody molecule with the ability to inhibit HIV infection and the gene coding sequence of the polypeptide with the ability to inhibit HIV infection.

The gene sequence construct described in any one of the above comprises a first gene coding sequence of an antibody molecule with the ability to inhibit HIV infection, a second gene coding sequence of an antibody molecule with the ability to inhibit HIV infection, and a gene coding sequence of a polypeptide with the ability to inhibit HIV infection, which is selected from:
VL2-linker-VH2-linker-VL1-linker-VH1-linker-CH2-CH3; or
VL2-linker-VH2-linker-VL1-linker-VH1-linker-CH2-CH3-linker-peptide inhibitor; or
other constructs that are constructed by arranging the combination of VL2 and VH2 or VL1 and VH1 in different orders in a construct.

VL2 and VH2 are the variable region fragments of light chain and heavy chain of the first antibody molecule respectively; VL1 and VH1 are the variable region fragments of light chain and heavy chain of the second antibody molecule respectively; CH2-CH3 is Fc fragment of human IgG constant region, and the linker is a linker polypeptide; the peptide inhibitor is a polypeptide that inhibits HIV infection (for example, a polypeptide that inhibits the fusion of HIV and CD4+ T cell membranes).

The gene sequence construct described above is VL2-linker-VH2-linker-VL1-linker-VH1-linker-CH2-CH3, or a construct in which the combinations of VL2 and VH2 or VL1 and VH1 are arranged in different orders.

The gene sequence construct described above is VL2-linker-VH2-linker-VL1-linker-VH1-linker-CH2-CH3-linker-peptide inhibitor, or a construct in which the combinations of VL2 and VH2 or VL1 and VH1 are arranged in different orders.

In the gene sequence construct described above, the protein sequences of VL2 and VH2 include SEQ ID NO: 2, or a functional fragment thereof, or a homologous sequence that is at least 75%, 80%, 85%, 90%, 95%, 98%, or 99% identical thereto.

In the gene sequence construct described above, the protein sequences of VL1 and VH1 include SEQ ID NO: 3, or a functional fragment thereof, or a homologous sequence that is at least 75%, 80%, 85%, 90%, 95%, 98%, or 99% identical thereto.

In the gene sequence construct described above, the sequence of the linker polypeptide (linker) is selected from: GGGGS, (GGGGS)₂, (GGGGS)₃, (GGGGS)₄, (GGGGS)₅, (GGGGS)₆ and (GGGGS)₇, or other optional linker polypeptide sequences.

In the gene sequence construct described above, the polypeptide (peptide inhibitor) that inhibits the fusion of HIV and CD4+ T cell membranes can be selected from the group consisting of membrane fusion inhibitory polypeptides P52, C34, T20, etc.

The sequence of the membrane fusion inhibitory polypeptide P52 includes SEQ ID NO: 5 or a homologous sequence that is at least 75%, 80%, 85%, 90%, 95%, 98%, or 99% identical thereto; the polypeptide sequence of C34 includes SEQ ID NO: 6 or a homologous sequence that is at least 75%, 80%, 85%, 90%, 95%, 98%, or 99% identical thereto; the polypeptide sequence of T20 includes SEQ ID NO: 7 or a homologous sequence that is at least 75%, 80%, 85%, 90%, 95%, 98%, or 99% identical thereto.

Furthermore, the present invention also provides a viral vector genome comprising any of the constructs described above and a corresponding viral vector system comprising the genome.

The viral vector system described above can be a lentiviral vector system or an adeno-associated virus vector system.

Among them, the lentiviral vector system can include the viral vector genome of any of the above constructs and other nucleotide sequences encoding and expressing packaging components required for the production of lentivirus, which are introduced into production cells to produce a lentiviral particle containing the genome of the construct described above.

The adeno-associated virus vector system can include the viral vector genome of any of the above constructs and other nucleotide sequences encoding and expressing packaging components required for the production of adeno-associated virus, which are introduced into production cells to produce an adeno-associated virus particle containing the genome of the construct described above.

Any virus particle produced above, which comprises the genome of any of the constructs described above, can express anti-HIV neutralizing antibody-like molecules after transducing cells, and can be used to administer to a patient to inhibit or prevent HIV infection.

The present invention also provides a pharmaceutical composition comprising the viral particle described above, and a pharmaceutically acceptable carrier or diluent, or cells transduced by the lentiviral particle described above in vitro, including but not limited to transduced muscle cells, liver cells, or CD4+ T cells.

The above-mentioned virus particle or the pharmaceutical composition containing the above virus particle can be used for injection into the body to express an antibody molecule protein with two or more than two target sites, and the mature molecule is a dimer formed by disulfide bonds, which can effectively and broadly block infection of HIV against human CD4+ T cells by binding to multiple binding sites involved in different steps of HIV infection against human CD4+ T cells, and can be used for gene therapy of HIV infection, thereby achieving long-term treatment for a HIV-infected individual.

The present invention provides a method for inhibiting HIV infection, comprising administering the viral particle or the pharmaceutical composition described above to cells.

The cells include muscle cells, liver cells, or CD4+ T cells.

The above-mentioned cells can be transduced in vitro or in vivo using one of the above-mentioned viral particles or pharmaceutical compositions.

The present invention provides a method of treating HIV infection in a subject in need thereof, comprising administering to the subject a therapeutically effective dose of the viral particle or the pharmaceutical composition as described above.

The subjects described therein include an early-stage HIV infector, a HIV-infected individual who is already received cocktail drug therapy, or a HIV-infected individual who is resistant to cocktail drug therapy.

The mode of administering drugs to the above-mentioned subject is intramuscular injection of one of the above-mentioned virus particles or pharmaceutical compositions.

Alternatively, one of the above-mentioned viral particles or pharmaceutical compositions or CD4+ T cells transduced thereby is injected intravenously.

The virus particles and pharmaceutical compositions injected into the body through the above-mentioned methods can express anti-HIV protein molecules with multiple targets sites and secrete them into blood, which can act on multiple nodes of HIV infection, and can effectively block HIV infection path and effectively avoid the loss of the ability to inhibit HIV infection due to escape mutations of HIV, thereby achieving a long-term or even permanent therapeutic effect on HIV infection with a single injection.

Antibody-like molecules composed of multiple single-chain antibody variable region fragments (scFv) based on the above expression cassettes were delivered to mice via lentivirus and adeno-associated virus vectors, and showed effective blood concentration, strong in vitro cytological HIV-1 virus neutralizing activity, and broad-spectrum neutralizing ability against both CXCR4 and CCR5 tropic viruses, suggesting a highly potential technical route for anti-HIV broad-spectrum neutralizing antibody gene therapy drugs.

The present invention can be used for various forms of anti-HIV gene therapy based on the genetic expression of broad-spectrum neutralizing antibodies.

### Description of the drawings

The drawings of the present invention are described in detail.
Figure 1. Schematic representation of the mature molecular structure of an expected tritropic anti-HIV neutralizing antibody.
Figure 2. Schematic representation of the gene sequence constituent of a tritropic anti-HIV neutralizing antibody.
Figure 3. Schematic representation of the mature molecular structure of an expected amphotropic anti-HIV neutralizing antibody.
Figure 4. Schematic representation of the gene sequence constituent of an amphotropic anti-HIV neutralizing antibody.
Figure 5. Schematic representation of a gene construct for cloning the gene sequence of an amphotropic (KL-BsHIV01) or tritropic (KL-BsHIV01-003) anti-HIV neutralizing antibody into a lentiviral vector.
Figure 6. Profile of the latest-generation lentiviral vector pKL-kan-lenti-EF1α-WPRE used in the present invention.
Figure 7. Profile of the latest-generation adeno-associated virus vector pAAV-MCS-CMV-EGFP (anti) used in the present invention.
Figure 8. Schematic representation of a gene construct for cloning the gene sequence of an amphotropic (KL-BsHIV01) or tritropic (KL-BsHIV01-003) anti-HIV neutralizing antibody into an adeno-associated virus vector.

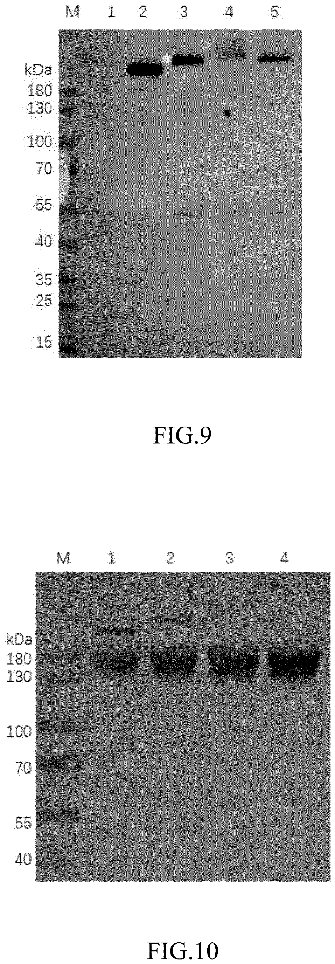

Figure 9. Expression of anti-HIV neutralizing antibody-like molecule in 293T cells detected by Western blot analysis. After transfecting 293T cells with a plasmid of a gene construct of the anti-HIV neutralizing antibody-like gene sequence molecule cloned into an adeno-associated virus vector, the same volume of 293T cell culture supernatant was collected as samples for Western blot analysis under non-reducing conditions, anti-HIV neutralizing antibody-like proteins were detected using goat anti-human IgG1 Fc fragment antibodies. M, pre-stained protein size marker; 1, blank plasmid transfection control; 2, KL-BsHIV01; 3, KL-BsHIV01-003; 4, KL-BsHIV01-C34; 5, KL-BsHIV01-T20. The larger molecular bands (much larger than 180 kDa) in the Western blot are anti-HIV neutralizing antibody-like molecules expected to exist in the form of dimers (under non-reducing conditions).
Figure 10. Expression of anti-HIV neutralizing antibody-like in C2C12 myotube cells detected by Western blot analysis. The recombinant adeno-associated virus is packaged after transfecting 293T cells with the plasmid of the gene construct of the anti-HIV neutralizing antibody-like gene sequence cloned into the adeno-associated virus vector and other helper plasmids. After isolation and purification, the transduced C2C12 cells were infected with the same infection coefficient. The same volume of C2C12 cell culture supernatant was collected as samples for Western blot analysis under non-reducing conditions, anti-HIV neutralizing antibody-like proteins were detected using goat anti-human IgG1 Fc fragment antibodies. M, pre-stained protein size markers; 1, KL-BsHIV01; 2, KL-BsHIV01-003; 3, KL-BsHIV01-C34; 4, KL-BsHIV01-T20. The larger molecular bands (much larger than 180 kDa) in the Western blot are anti-HIV neutralizing antibody-like molecules expected to exist in the form of dimers (under non-reducing conditions). The band at the equivalent position of 180 kDa is a non-specific band.
Figure 11. Neutralizing activity of the culture supernatant of 293T cells transduced with the HIV-neutralizing antibody lentiviral gene therapy vector against HIV. VCN, copy number of lentiviral vector after transduction of 293T cells.
Figure 12. Neutralizing activity of culture supernatant of C2C12 cells transduced with the HIV neutralizing antibody adeno-associated virus gene therapy vector against the virus.
Figure 13. Expression of the anti-HIV neutralizing antibody adeno-associated virus gene therapy vector in BALB/c mice after intramuscular injection. The levels of anti-HIV neutralizing antibodies secreted into mouse serum were determined by ELISA.
Figure 14. Neutralizing activity against HIV strains of amphotropic and tritropic HIV-neutralizing antibody-like secreted into mouse serum at different concentrations. The data in the figure are the mean ± SD of three parallel measurements for each sample. This experiment was repeated more than two times.

### Detailed description of the invention

Broadly neutralizing antibodies (bNAb) with HIV-1 neutralizing activity are produced in a small number of elite infected individuals over several years, which can efficiently and broadly bind to virus surface glycoproteins of HIV and neutralize the infection activity of HIV against CD4+ T cells, thereby representing a promising method for preventing or resisting HIV-1 infection. However, the specific mechanism for production of the bNAb is not clear. Most individuals infected with HIV-1 can only produce non-neutralizing antibodies. Currently, there is no research has successfully induced the production of broad-spectrum neutralizing antibodies in healthy subjects through standard immunization methods.

Without wishing to be bound by theory, it is believed that in some embodiments, recombinantly derived broad-spectrum neutralizing antibodies against HIV-1 can effectively reduce the viral load in a patient, and even if they cannot completely eliminate HIV from the body, they can help control the progression from HIV infection to AIDS. Compared with small molecular anti-HIV drugs, recombinantly derived neutralizing antibodies can also greatly reduce side effects and increase patient compliance. Clinically, recombinant broad-spectrum neutralizing antibodies can be used as vaccine replacement products for preventing HIV infection in specific situations, or as antiviral drugs at different disease stages.

However, like highly effective anti-HIV small molecular drugs, specific broad-spectrum neutralizing antibodies only target a single epitope of a single viral protein (HIV-gp160). The escape phenomenon caused by viral mutations is still difficult to avoid. Since the development, production, and use costs of broad-spectrum neutralizing antibodies are much higher than that of small molecular anti-HIV drugs, combined use of drugs has no advantages. Therefore, a large number of anti-HIV broad-spectrum neutralizing antibody drugs under development are difficult to use clinically to date.

In response to the above problems, the research and development of broad-spectrum neutralizing antibody drugs against HIV mainly requires breakthroughs in the following two aspects:
1. Development of amphotropic or polytropic neutralizing antibodies or antibody-like macromolecules to cover multiple targets to avoid escape caused by viral mutations.
2. Geneticization of broad-spectrum neutralizing antibodies or antibody-like macromolecules. Anti-HIV infection gene therapy drugs delivered by recombinant viruses or non-viral vectors stably express neutralizing antibodies or antibody-like macromolecules in the body in a genome-integrated or non-integrated manner for a long time. A single treatment is effective for a long time or even lifelong, greatly reducing the production and use costs of macromolecular antibody drugs.

The present invention adopts a polytropic antibody molecular structure, that is, the antigen-binding region consists of two or more than two single-chain variable region fragments (scFv) of monoclonal broad-spectrum neutralizing antibodies concatenated by a linker polypeptide (linker).

Specific embodiments include constructing a series of antibody molecule gene constructs containing amphotropic/tritropic antibody single chain variable region fragments (scFv) and cloning them into recombinant adeno-associated virus and recombinant lentiviral vectors. Then the corresponding adeno-associated virus and lentivirus are packaged in 293T cells, and the 293T cells and differentiated and undifferentiated muscle cell lines are infected with a certain biological titer of the virus. The antibody molecules produced in the cell supernatant were tested quantitatively and qualitatively, and their neutralizing activity against the HIV-1 wild-type virus strain was tested in the infection activity analysis of the HIV-1 wild-type virus strain and the virus-sensitive reporter cell line TZM-bl.

The structure of the anti-HIV neutralizing antibody of the present invention, the constituent of the gene sequence construct, and the special terms involved will be further described in detail below in conjunction with the examples.

**Antibody** can be an immunoglobulin, an antigen-binding fragment, or a protein molecule derived therefrom that specifically recognizes and binds to an antigen (e.g., HIV-1 gp41 antigen). "Antibody" in the present invention is a broad definition covering various antibody structures, including but not limited to monoclonal antibodies, polyclonal antibodies, polytropic antibodies (e.g., amphotropic antibodies, tritropic antibodies), and antibody fragments, provided they possess specific antigen-binding activity. Examples of specific antibodies include intact immunoglobulins as well as antibody variants and fragments that retain binding affinity for the antigen. Examples of antibody fragments include, but are not limited to, variable region fragments (Fv), antigen-binding fragments (e.g., Fab, Fab', Fab'-SH, or F(ab')2 produced after proteolysis), single-chain antibodies molecules (eg, scFv), diabodies, nanobodies, and polytropic antibodies formed from combinations of antibody fragments. Antibody fragments include antigen-binding fragments produced by modification of intact antibodies or antigen-binding fragments synthesized de novo using recombinant DNA technology.

Single chain antibodies (scFv) are molecules obtained through genetic engineering and contain the light chain variable region (VL) and heavy chain variable region (VH) of one or more antibodies, each fragment is concatenated by a suitable linker polypeptide to form a fused single-chain molecule. The concatenation orders of VL and VH in a single-chain antibody molecule usually does not affect its antigen-binding function, thus both single-chain antibodies composed of two concatenation manners (VL-VH or VH-VL) will be used.

An antibody can have one or more antigen-binding sites. In the case of more than one antigen binding site, these binding sites may be the same or different. For example, a naturally occurring immunoglobulin has two identical antigen-binding sites, while a Fab fragment produced from an immunoglobulin by papain hydrolysis has only one antigen-binding site, and an amphotropic single-chain antibody (scFv) has two different antigen-binding sites.

Normally, a naturally occurring immunoglobulin consists of a light chain and a heavy chain linked by disulfide bonds. Immunoglobulin genes include γ, α, δ, ε, µ, λ, and κ constant region genes as well as numerous immunoglobulin variable region genes. Light chains are of two types, λ and κ. There are five main types of heavy chains (γ, α, δ, ε, µ), which determine the functional classification of antibody molecules, namely IgG, IgA, IgD, IgE, and IgM.

Each heavy and light chain contains a constant region and a variable region. VH represents the variable region of the antibody heavy chain, including the heavy chain variable region of the antigen-binding fragment Fv, scFv, or Fab. VL represents the variable region of an antibody light chain, including the light chain variable region of an antigen-binding fragment Fv, scFv, or Fab. In the following examples, VH and VL work together to specifically recognize and bind antigens.

VH and VL contain three separated highly variable regions (also called complementarity determining regions (CDRs)) and a framework region. The sequences of the backbone regions of different light and heavy chains are relatively conserved within the same species. The backbone region of an antibody determines the position of the complementarity-determining regions in the three-dimensional structure. The complementarity determining region is mainly responsible for binding to the epitope of an antigen. The three complementarity determining regions on the light chain are labeled LCDR1, LCDR2, and LCDR3 from N-terminus to C-terminus, respectively. The three complementarity determining regions on the heavy chain are labeled HCDR1, HCDR2, and HCDR3 from N-terminus to C-terminus, respectively.

The protein sequences of VH and VL in the present invention include, in addition to the sequences disclosed in the following examples, any other sequences that carry functional fragments thereof, or a homologous sequence that is at least 75%, 80%, 85%, 90%, 95%, 98%, or 99% identical thereto.

**Constant region fragment of an antibody** is a region of immunoglobulin with a relatively stable amino acid sequence except for the variable region near the N-terminus where the amino acid sequence changes greatly, including the constant domain in the antigen-binding fragment (located at the N-terminus of the hinge region, including the light chain constant domain and the heavy chain constant domain) and the constant domain of the crystallizable fragment of the heavy chain (called the Fc fragment, located at the C-terminal of the hinge region). The Fc fragment usually refers to the last two constant region domains of immunoglobulins IgA, IgD, and IgG or the last three constant region domains of IgE and IgM. The Fc fragment may also include part or all of the hinge region sequence located at its N-terminus.

**Anti-HIV-1 neutralizing antibody or antigen-binding fragment** specifically binds to the HIV-1 envelope protein (e.g., binds to gp41), thereby inhibiting biological functions associated with the HIV-1 envelope (e.g., the ability to bind to target receptors). In the following examples, anti-HIV-1 neutralizing antibodies or antigen-binding fragments reduce the infection titers of HIV-1 strains with different tropisms against cells.

**Amphotropic or polytropic antibody** is a recombinant molecule composed of two or more than two different antigen-binding domains, and therefore can bind two or more than two different antigenic determinants. Amphotropic or polytropic antibodies include molecules composed of two or more than two different antigen-binding domains linked through chemical synthesis or genetic engineering. The antigen-binding domains can be linked via a linker polypeptide. The antigen-binding domain can be a monoclonal antibody, an antigen-binding fragment (e.g., scFv or Fab), or a combination of antigen-binding domains from different sources.

**Linker polypeptide** is used to connect two protein molecules or fragments into a continuous single fusion molecule. For example, in the following examples, two or more antibody molecules or antigen-binding fragments (e.g., scFv) are connected to form a polytropic antibody molecule with two or more antigen-binding sites; or the light chain variable region (VL) and heavy chain variable region (VH) of an antibody are connected to form a single-chain antigen-binding sequence; or the antibody molecule or antigen-binding fragment is connected with other effector molecules, for example, the antigen-binding fragment scFv is connected to an HIV-1 membrane fusion inhibitory polypeptide to form a fusion protein. Linker polypeptides are typically rich in glycine (Gly or G) to increase linker flexibility, and rich in serine (Ser or S) or threonine (Thr or T) to increase solubility, for example, the (GGGGS)ₙ linkers with different lengths used in some of the following examples, n may be 1 or more than 1. However, the linker polypeptide sequence used in the examples is not limited to this, and also includes other optional linker polypeptide sequences.

**Antigenic determinant** is a specific chemical group or polypeptide sequence on a molecule that has antigenicity, that is, it can stimulate a specific immune response of the host. An antibody specifically binds to a specific epitope on a polypeptide, such as in the following examples, an antibody that specifically binds to an epitope on gp41.

**HIV-1 envelope protein** is first synthesized as a precursor protein with a size of 845-870 amino acid residues, called HIV gp160. gp160 forms a homotrimer in the host cell, which is glycosylated, cleaved to remove the signal peptide, and then cleaved by an intracellular protease at amino acid residues 511/512 to produce gp120 and gp41 polypeptide chains. gp120 and gp41 remain associated together in the homotrimer as the gp120/gp41 protomer. Mature gp120 consists of amino acid residues 31-511 of the HIV-1 envelope protein and is a highly N-glycosylated protein, constituting the majority of the domain of the HIV-1 envelope protein trimer exposed on the envelope surface. gp120 is responsible for binding to the human CD4 cell receptor as well as coreceptors (e.g., chemokine receptors CCR5 or CXCR4). gp41 consists of amino acid residues 512-860 of the HIV-1 envelope protein, including the intra-envelope domain, the transmembrane domain, and the extra-envelope domain. The extra-envelope domain of gp41 includes amino acid residues 512-644, which combines with gp120 to form a protomer, which together constitute the HIV-1 envelope protein homotrimer. The protruding extra-envelope domain of the HIV-1 envelope protein trimer undergoes several structural rearrangements, from a closed structure before fusion with the host cell membrane that can escape antibody recognition, to an intermediate structure that bind human CD4 cell receptors and coreceptors, and a post-membrane fusion structure.

**HIV membrane fusion inhibitory peptide:** Membrane fusion of virus and host cells is a key step in HIV infection against cells. After binding of the glycoprotein gp120/gp41 homotrimer on the HIV envelope to the human CD4 cell receptor and co-receptor, the structure undergoes several changes. Finally, the gp41 trimer integrated on the HIV envelope is inserted into the host cell membrane, completing the fusion of the virus and host cell membranes, and resulting in entering of HIV genetic material into the cells. HIV membrane fusion inhibitory polypeptide binds to the envelope protein of the virus, thereby preventing it from undergoing the structural changes necessary for the fusion of the virus and the host CD4 cell membrane, and preventing HIV from infecting CD4 cells. HIV membrane fusion inhibitory polypeptides used in the present invention include the P52, C34, T20 sequences disclosed in the following examples or homologous sequences that are at least 75%, 80%, 85%, 90%, 95%, 98%, or 99% identical thereto.

**Gene sequence construct** is a vector composed of a recombinant polynucleotide sequence, which is composed of an expression control sequence connected to the nucleic acid sequence to be expressed. The expression vector contains sufficient cis-acting elements and other expression elements are provided by the host cell. Expression vectors include all vectors of the present invention, such as plasmids and viruses carrying integrated recombinant polynucleotide sequences (e.g., recombinant lentivirus and recombinant adeno-associated virus). The vector carries a nucleic acid sequence (DNA or RNA) that allows it to replicate in a host cell, such as a replication initiation site, one or more selectable marker genes, and other genetic structures disclosed in the present invention. Viral vectors are recombinant nucleic acid vectors that carry at least part of the nucleic acid sequence from one or more viruses. In certain examples below, a viral vector contains one or more nucleic acid sequences encoding a disclosed antibody or antigen-binding fragment that specifically binds to HIV-1 gpl60 and thereby neutralizes HIV-1. In some examples, the viral vector may be a recombinant adeno-associated virus (AAV) vector or a recombinant lentiviral vector. These viral vectors are replicationdefective vectors and require other helper plasmids or vectors carrying gene functions or components necessary for viral replication to be amplified and packaged in cells to produce viral particles. The purified and prepared viral vectors or viral particles will not replicate and amplify in host cells when used to treat patients.

**Treatment of HIV infection:** HIV is a virus that attacks the body's immune system. It mainly targets the most important CD4 T lymphocytes in the human immune system, substantially destroys these cells, leading to the loss of immune function. Many patients with acute HIV infection experience flu-like symptoms 2 to 4 weeks after infection, which may last from days to weeks. Patients in the acute infection stage have a large amount of HIV in their blood and are highly infectious. Afterwards, the patient enters the asymptomatic chronic infection period, also known as the HIV latent period. However, the HIV in the patient's is still active, continues to proliferate, and can be spread. The average incubation period of HIV in the human is 8 to 9 years. During the incubation period of the HIV, HIV-infected people can live and work for many years without any symptoms. However, if people infected with HIV do not receive any anti-HIV infection treatment, they will develop into the most serious stage of HIV infection, that is, the AIDS stage after the incubation period. AIDS patients have a high viral load and can easily transmit HIV to others. Their immune systems are severely damaged and their bodies are susceptible to various diseases and malignant tumors, with a high mortality rate.

Currently, there is still a lack of effective drugs to cure HIV infection worldwide. The current treatment goals are: maximally and sustainably reducing viral load; rebuilding acquired immune function and maintaining immune function; improving quality of life; and reducing HIV-related morbidity and mortality. The currently commonly used anti-HIV infection method is a combined antiretroviral therapy (i.e., cocktail therapy), which greatly improves the efficacy of anti-HIV and significantly improves the quality of life and prognosis of patients. The earlier the cocktail therapy is started, the better the results. For example, in the acute phase of HIV infection, i.e., in the first few months after infection, treatment should be started as soon as HIV infection is diagnosed. However, cocktail therapy has side effects and limitations, such as the need of maintaining long-term compliance with continuous medication, as well as the emerging drug-resistant strains of HIV, which still causes long-term economic and social burdens, a significant decline in the quality of life, and obvious suffering to the majority of AIDS patients.

Broad-spectrum neutralizing antibodies with HIV-1 neutralizing activity identified and isolated from a small number of elite infected individuals can efficiently and broadly bind to virus surface glycoproteins of HIV and neutralize the infection activity of HIV against human CD4+ T cells, thereby representing a promising method for preventing or resisting HIV-1 infection. Compared with small molecular anti-HIV drugs, recombinantly derived neutralizing antibodies can also greatly reduce side effects and increase patient compliance. The only anti-HIV neutralizing antibody currently approved for clinical use is Ibalizumab, which targets the CD4 receptor on the surface of human T cells. It interferes with the binding of virus surface glycoproteins of HIV to CD4 receptors by binding to CD4 receptors, thereby neutralizing the infection activity of the virus against CD4+ T cells and showing excellent efficacy in patients with multi-drug resistance to HIV infection.

However, specific broad-spectrum neutralizing antibodies only target a single epitope of a single viral protein (HIV-gp160). The escape phenomenon caused by viral mutations is still difficult to avoid. Since the development, production, and use costs of broad-spectrum neutralizing antibodies are much higher than that of small molecular anti-HIV drugs, combined use of drugs has no advantages. The present invention develops amphotropic and polytropic neutralizing antibodies or antibody-like macromolecules, which cover multiple targets to avoid escape caused by viral mutations, and geneticizes the broad-spectrum neutralizing antibodies or antibody-like macromolecules, resulting in breakthrough in the development of anti-HIV broad-spectrum neutralizing antibody drugs. Anti-HIV infection gene therapy drugs delivered by recombinant viruses or non-viral vectors stably express neutralizing antibodies or antibody-like macromolecules in the body in a genome-integrated or non-integrated manner for a long time. A single treatment is effective for a long time (for example, therapeutically effective lasts for one year or years) or even lifelong, greatly reducing the production and use costs of macromolecular antibody drugs.

The following examples explain the present invention and the present invention is not limited to the following examples.

### Examples

### I. Design of structure of HIV neutralizing antibodies

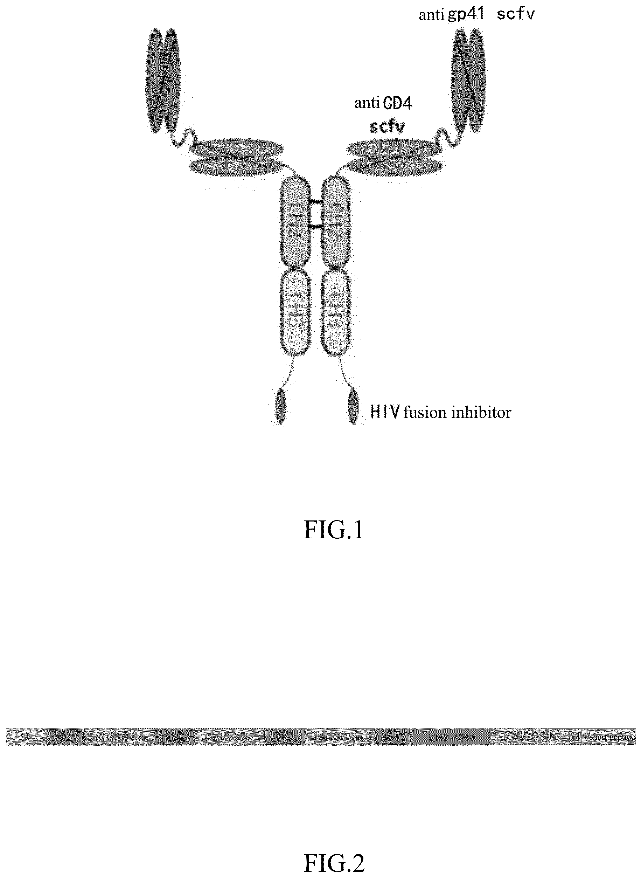

The structure of the anti-HIV neutralizing antibody and the antibody-like molecule used in the present invention is a tritropic anti-HIV neutralizing antibody-like molecule, which, from the N-terminal to the C-terminal of the protein molecule consists of: signal peptide - scFv of anti-HIV-gp41 broad-spectrum neutralizing antibody (VH-(ggggs)ₙ linker-VL)-(ggggs)ₙ linker- scFv of anti-human CD4 antibody (VH-(ggggs)ₙ linker-VL)-human IgG1 CH2- CH3-(ggggs)ₙ linker-HIV membrane fusion inhibitory short peptide. The coding sequence is expressed in cells and is translated into a protein, which forms a dimer and is secreted out of the cells. The expected structure of the mature anti-HIV neutralizing antibody-like molecule is shown in Figure 1, and the gene structure is shown in Figure 2.

### II. Gene sequences expressing HIV neutralizing antibodies

The sequences of anti-HIV-gp41 broad-spectrum neutralizing antibody scFv are: signal peptide (the protein sequence is as set forth in SEQ ID NO: 1); anti-HIV-1-gp41-MPER monoclonal antibody (10E8v4- 5R+100cF)-scFv (the protein sequence is as set forth in SEQ ID NO: 2); scFv of anti-human CD4 monoclonal antibody (Ibalizumab) (the protein sequence is as set forth in SEQ ID NO: 3), human IgG1 Fc fragment (the protein sequence is as set forth in SEQ ID NO: 4), HIV membrane fusion inhibitory short peptide P52 (the protein sequence is as set forth in SEQ ID NO: 5), HIV membrane fusion inhibitory short peptide C34 (the protein sequence is as set forth in SEQ ID NO: 6), HIV membrane fusion inhibitory short peptide T20 (the protein sequence is as set forth in SEQ ID NO: 7).

### III. A total of four gene expression cassettes for anti-HIV neutralizing antibody were constructed, which are:

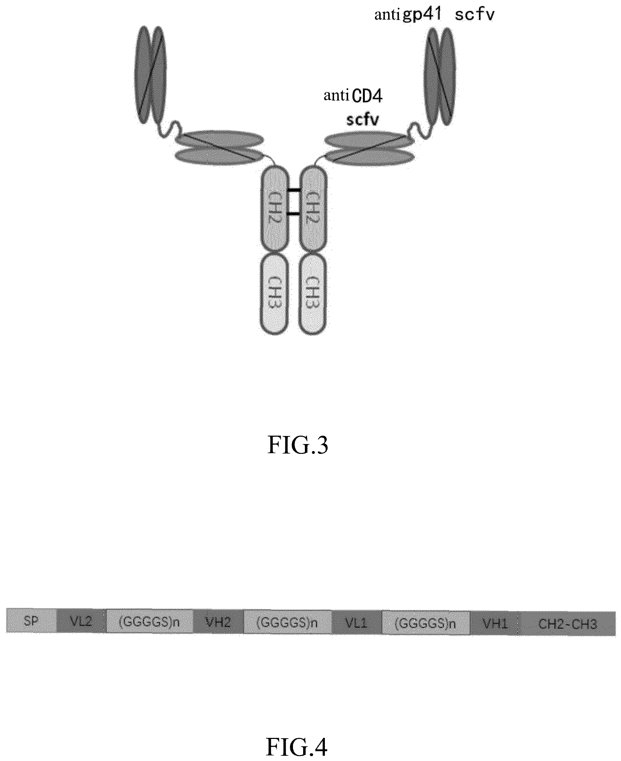

1. Anti-HIV amphotropic neutralizing antibody-like molecule KL-BsHIV01 containing anti-HIV neutralizing antibody 10E8v4-5R+100cF-scFv (the protein sequence is as set forth in SEQ ID NO: 8) (the DNA sequence is as set forth in SEQ ID NO: 9) (as shown in Figure 3 and Figure 4);
2. Anti-HIV tritropic neutralizing antibody-like molecule KL-BsHIV01-003 containing anti-HIV neutralizing antibody 10E8v4-5R+100cF-scFv (the protein sequence is as set forth in SEQ ID NO: 10) (the DNA sequence is as set forth in SEQ ID NO: 11) (as shown in Figure 1 and Figure 2);
3. Anti-HIV tritropic neutralizing antibody-like molecule KL-BsHIV01-C34 containing anti-HIV neutralizing antibody 10E8v4-5R+100cF-scFv (the protein sequence is as set forth in SEQ ID NO: 12) (the DNA sequence is as set forth in SEQ ID NO: 13) (as shown in Figure 1 and Figure 2);
4. Anti-HIV tritropic neutralizing antibody-like molecule KL-BsHIV01-T20 containing anti-HIV neutralizing antibody 10E8v4-5R+100cF-scFv (the protein sequence is as set forth in SEQ ID NO: 14) (the DNA sequence is as set forth in SEQ ID NO: 15) (as shown in Figure 1 and Figure 2).

### IV. Examples of gene therapy vector constructs for HIV neutralizing antibodies

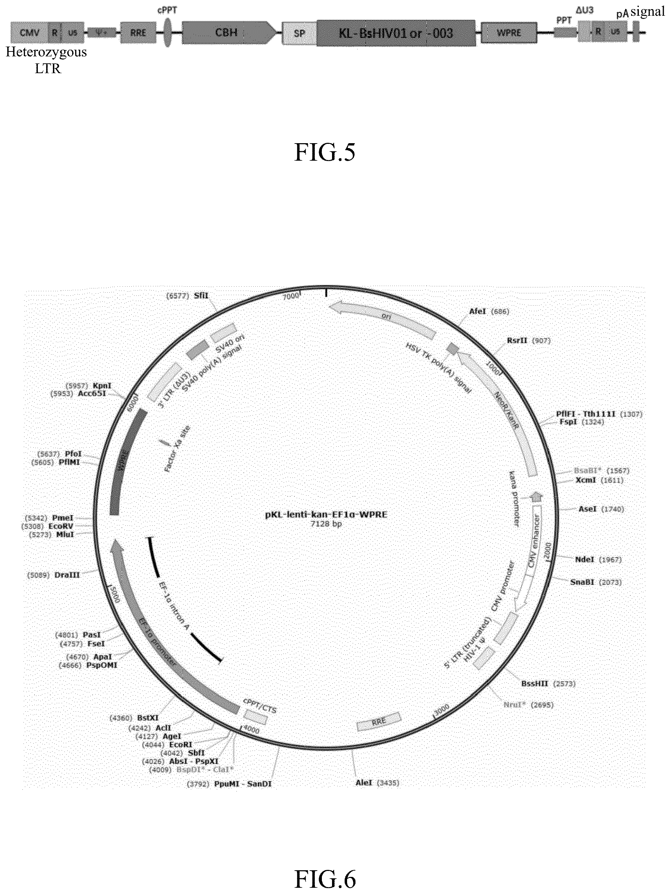

As shown in Figure 5, the above gene expression cassette for monoclonal antibody molecule was cloned into the latest generation lentiviral vector currently used, pKL-kan-lenti-EF1α-WPRE (Figure 6) (the DNA sequence is as set forth in SEQ ID NO: 16) by multi-fragment recombinant ligation. The lentiviral vector includes: 5'LTR, in which the promoter region of the LTR is replaced with a CMV promoter; ψ packaging signal; retroviral export element RRE; cPPT; a promoter CBH; a polynucleotide encoding a polypeptide of an HIV neutralizing antibody fragment; the post-transcriptional regulatory element WPRE; PPT; ΔU3 3'LTR; and a poly(A) signal. The gene expression cassette for neutralizing antibody (Figure 4) designed in this example was synthesized by Nanjing Genscript Biotechnology Co., Ltd., which together with the CBH promoter (the sequence is as set forth in SEQ ID NO: 17) were cloned between the multiple cloning sites EcoRI/EcoRV on the lentiviral vector backbone pKL-kan-lenti-EF1α-WPRE by homologous recombination methods well known in the art (Figure 6). After cloning was completed, the sequence information was confirmed by sequencing, and the plasmid was named as pKL-Kan-lenti-CBH-KL-BsHIV01 (the sequence is as set forth in SEQ ID NO: 18) (Figure 5). The coding sequence of the HIV fusion inhibitory short peptide P52 or C34 or T20 was cloned between the multiple cloning sites EcoRV on the lentiviral vector backbone pKL-kan-lenti-EF1α-WPRE by homologous recombination methods well known in the art. After cloning was completed, the sequence information was confirmed by sequencing, and the plasmid was named as pKL-Kan-lenti-CBH-KL-BsHIV01-003 (the sequence is as set forth in SEQ ID NO: 19) (Figure 5).

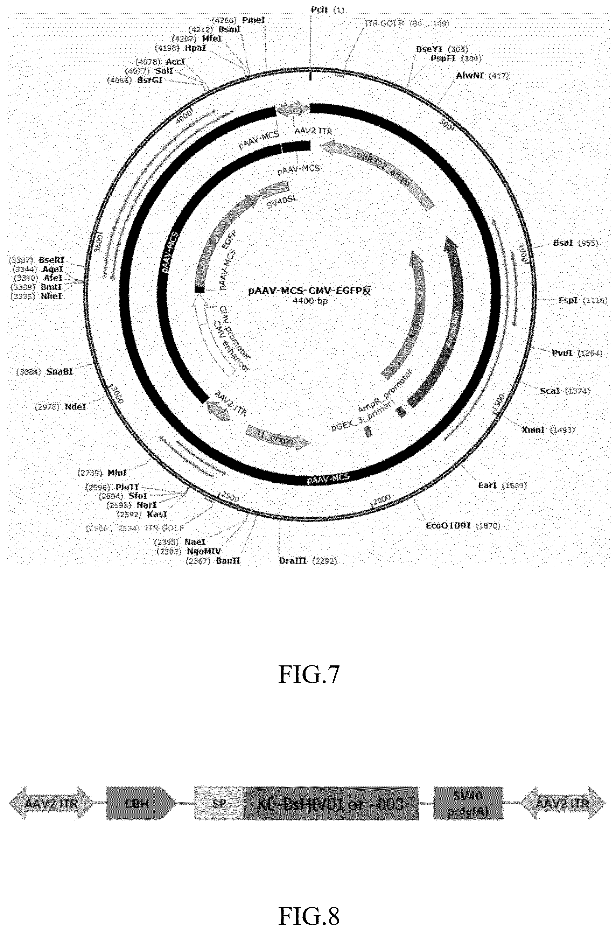

As shown in Figure 5, the HIV neutralizing antibody gene expression cassettes CBH-KL-BsHIV01, CBH-KL-BsHIV01-003, CBH-KL-BsHIV01-C34, CBH-KL-BsHIV01-T20 present in pKL-Kan-lenti-CBH-KL-BsHIV01, together with WPRE, were cloned between the multi-cloning sites MluI/SalI on the currently used latest generation adeno-associated virus vector pAAV-MCS-CMV-EGFP (anti) (the sequence is as set forth in SEQ ID NO: 20) through multi-fragment recombination ligation (see Figure 7). The adeno-associated virus vector includes: AAV2 ITR; a promoter CBH; a polynucleotide encoding HIV neutralizing antibody fragment; WPRE and SV40 poly(A) signal; AAV2 ITR. The plasmids were named as pAAV-CBH-KL-BsHIV01-WPRE (the sequence is as set forth in SEQ ID NO: 21), pAAV-CBH-KL-BsHIV01-003-WPRE (the sequence is as set forth in SEQ ID NO: 22), pAAV-CBH-KL-BsHIV01 -C34-WPRE (the sequence is as set forth in SEQ ID NO: 23), pAAV-CBH-KL-BsHIV01-T20-WPRE (the sequence is as set forth in SEQ ID NO: 24) (Figure 8).

### V. Packaging and purification of viruses expressing HIV neutralizing antibodies

Lentiviral vectors (pKL-Kan-lenti-CBH-KL-BsHIV01 and pKL-Kan-lenti-CBH-KL-BsHIV01-003) were used to package lentiviral vectors for antibody gene therapy in the 293T cell line. The antibody gene lentiviral vectors (pKL-Kan-lenti-CBH-KL-BsHIV01 and pKL-Kan-lenti-CBH-KL-BsHIV01-003) constructed in the examples, envelope plasmid (pKL-Kan-Vsvg, the nucleotide sequence is shown in SEQ ID NO: 25) and the packaging plasmids (pKL-Kan-Rev, the nucleotide sequence is shown in SEQ ID NO: 26; pKL-Kan-GagPol, the nucleotide sequence is shown in SEQ ID NO: 27) were mixed and co-transfected into 293T cells (purchased from the American Type Culture Collection Center (ATCC), ATCC deposit number: CRL-3216) at the same time, to perform the package of the HIV neutralizing antibody gene therapy lentivirus in the 293T cell line. The transfection method was transient transfection of eukaryotic cells mediated by PEI cationic polymer. The PEI cationic polymer is the PEI-Max transfection reagent purchased from Polysciences (Cat. No.: 24765-1). The transfection operation was carried out according to the standardized operation recommended by the manufacturer, and the transfection scale was 15cm cell culture dish. At 48 hours after the transfection was completed, the lentiviral vector (transfected cell culture supernatant) was harvested. The supernatant was firstly centrifuged in a desktop bucket centrifuge at 4000 rpm at room temperature for 5 minutes to remove cell debris, and then centrifuged at 10000g at 4°C for 4 hours to obtain virus particle pellet. After removing the centrifugation supernatant, 1 mL DMEM complete medium was added to the virus particle pellet, the virus particles were resuspended with a microinjector, and the prepared virus resuspension was aliquoted and frozen at -80°C for later use.

The AAV expression vectors (pAAV-CBH-KL-BsHIV01-WPRE, pAAV-CBH-KL-BsHIV01-003-WPRE, pAAV-CBH-KL-BsHIV01-C34-WPRE, and pAAV-CBH-KL-BsHIV01-T20-WPRE ) were used to package the antibody gene therapy AAV vector in the 293T cell line. The antibody gene AAV vector constructed in the Example, capsid plasmid (AAV2/8, the nucleotide sequence is shown in SEQ ID NO: 28) and packaging plasmid (pHelper, the nucleotide sequence is shown in SEQ ID NO: 29) were mixed and co-transfected into 293T cells at the same time, to perform the package of the HIV neutralizing antibody gene therapy vector AAV in the 293T cell line. The transfection method was transient transfection of eukaryotic cells mediated by PEI cationic polymer. The PEI cationic polymer is the PEI-Max transfection reagent purchased from Polysciences (Cat. No.: 24765-1). The transfection operation was carried out according to the standardized operation recommended by the manufacturer, and the transfection scale was 15cm cell culture dish. The supernatant was discarded after 7 hours after transfection and replaced with 25 ml of toxin-producing medium. At 120 hours after the transfection is completed, the supernatant and cells were collected, centrifuged at 4200 rpm for 10 minutes, and then the supernatant and cells were separated. Lysis solution and ribozyme were added to the cells, lysed and digested for 1 hour, centrifuged at 10000g for 10 minutes and the lysate supernatant was obtained. The lysate supernatant and culture medium supernatant were purified by affinity chromatography and then aliquoted and frozen at -80°C for later use.

### VI. Functional verification of HIV neutralizing antibodies expressed in the supernatant of cells transduced with lentiviral gene therapy vectors

The packaged lentiviral vectors pKL-Kan-lenti-CBH-KL-BsHIV01 and pKL-Kan-lenti-CBH-BsHIV01-003 were used to infect 293T cells at different MOIs. After 48 hours, the supernatant and some cells were collected. The infection copy number of lentiviral vectors was detected by probe method.

1. 293T cells infected with lentiviral vectors were collected, washed with PBS, centrifuged at 4200rpm for 5 minutes to collect the cells, resuspended in 20µl QE DNA Extraction Solution and subjected to the following program on a PCR machine to lyse the cells and extract the total DNA.

### Cell Lysis PCR Procedure:

| Temperature | Time |
|---|---|
| 65°C | 15 min |
| 68°C | 15 min |
| 95°C | 10 min |

Quantitative PCR was used to calculate the infected lentivirus copy number (VCN) of 293T cells using methods well known in the art.

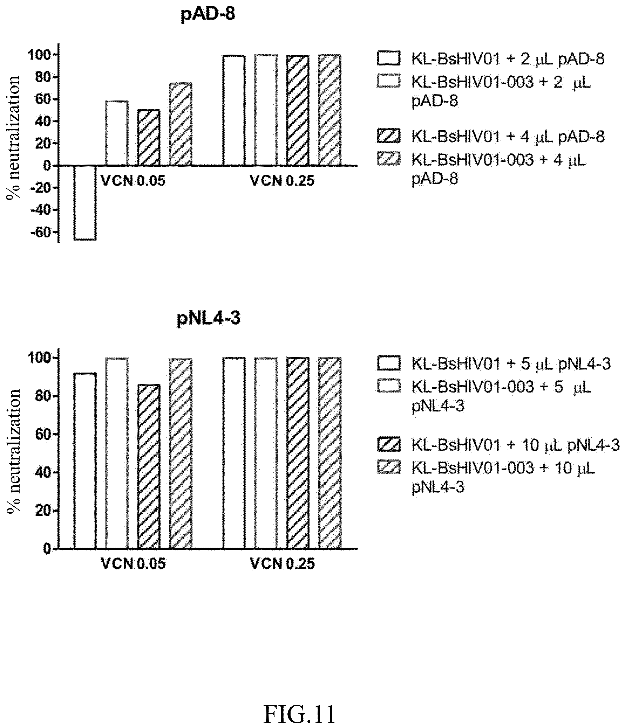

2. TZM-bl cells at 2E4 cells/well were plated. 50 µL of cell supernatant with antibody expression with VCN of 0.05 and 0.25 was mixed with 50 µL of HIV pAD-8 and pNL4-3 respectively, incubated at 37°C for 30 minutes, and added to TZM-bl cells. The wells where only pAD-8 or pNL4-3 was added were negative controls, and the well where HIV was not added was blank control. At 24 hours, the supernatant was discarded, and 100 µL cell lysis solution was added. After 10 minutes, the lysed cells were collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed (Figure 11) that in *in vitro* cell experiments, at the same infection copy number (VCN), 293T cell culture supernatant HIV transduced with the neutralizing antibody KL-BsHIV01-003 lentiviral gene therapy vector contained antibodies that completely neutralize HIV, and the neutralizing effect was significantly better than the HIV bispecific neutralizing antibody KL-BsHIV01 lentiviral gene therapy vector.

### VII. Functional verification of HIV neutralizing antibodies expressed in the supernatant of cells transduced with adeno-associated virus gene therapy vectors

C2C12 mouse myoblasts were plated in 24-well cell culture plate at 1E5 cells per well, and differentiated into myotube cells using 2% horse serum medium. The packaged adeno-associated viruses pAAV-CBH-KL-BsHIV01, pAAV-CBH-KL-BsHIV0-003, pAAV-CBH-KL-BsHIV01-C34, andpAAV-CBH-KL-BsHIV01-T20 were used to infect differentiated C2C12 cells at different MOIs. The supernatant was collected after 96 hours.
1. The enzyme plate (Corning, Cat. No.: 42592) was coated with synthetic HIV MPER peptide. The expression supernatant and the purified and quantified KL-BsHIV01 standard were used as the primary antibody, HRP-labeled goat anti-human IgG Fc (KPL, Cat. No.: 04-10-20) was used as the secondary antibody, developed with TMB, and the OD value was detected at 450 nm with a microplate reader. ELISA results (Table 1) showed that the anti-HIV neutralizing antibody adeno-associated virus vector could effectively express HIV neutralizing antibodies after transducing cells in vitro and secreted mature HIV neutralizing antibody proteins into the cell culture supernatant. At the same MOI, the expression level of KL-BsHIV01 in the supernatant was the highest, and the expression level decreased after adding HIV fusion inhibitory short peptide (see also Figure 9 and Figure 10).

**Table 1. ELISA detection of antibody expression level in culture supernatant of C2C12 cells transduced with HIV neutralizing antibody adeno-associated virus gene therapy vectors**

| AAV expression construct | HIV neutralizing antibody concentration (ng/mL) | | |
|---|---|---|---|
| | MOI 8.00E+04 | MOI 1.60E+05 | MOI 3.20E+05 |
| KL-BsHIV01 | 675.00 | 460.40 | 423.70 |
| KL-BsHIV01-003 | 31.82 | 62.42 | 122.80 |
| KL-BsHIV01-C34 | 43.05 | 43.43 | 60.49 |
| KL-BsHIV01-T20 | 11.67 | 20.91 | 31.43 |

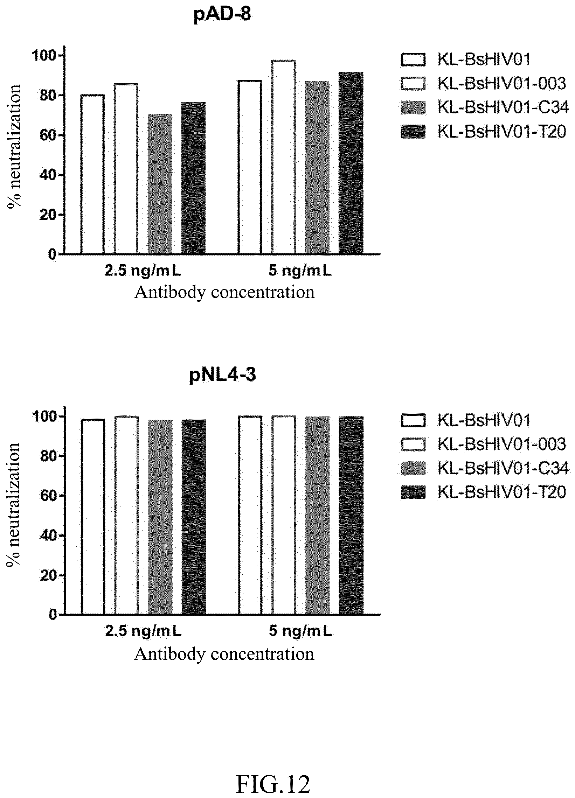

2. TZM-bl cells at 2E4 cells/well were plated. the cell expression supernatants containing the same amount of antibodies were mixed with 50 µL of HIV pAD-8 or pNL4-3, incubated at 37°C for 30 minutes, and added to TZM-bl cells. The wells where only pAD-8 or pNL4-3 was added were negative controls, and the well where HIV was not added was blank control. At 24 hours, the supernatant was discarded, and 100 µL cell lysis solution was added. After 10 minutes, the lysed cells were collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed (Figure 12) that in *in vitro* cell experiments, when the same amount of HIV neutralizing antibodies was added, the tritropic HIV neutralizing antibody in which HIV fusion inhibitory short peptide is added to KL-BsHIV01 had the neutralizing effect that was not weaker than the original antibody, and the neutralizing effect of KL-BsHIV01-003 on pAD-8 and pNL-3 was significantly better than that of the HIV bispecific neutralizing antibody KL-BsHIV01.

### VIII. Expression of adeno-associated virus gene therapy vector in BALB/c mice after intramuscular injection

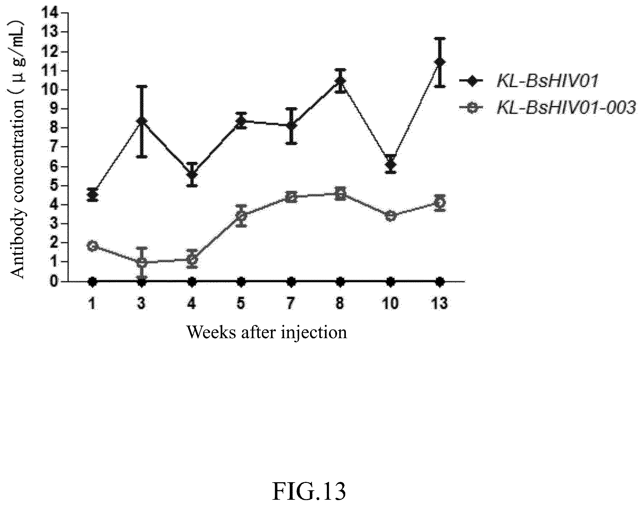

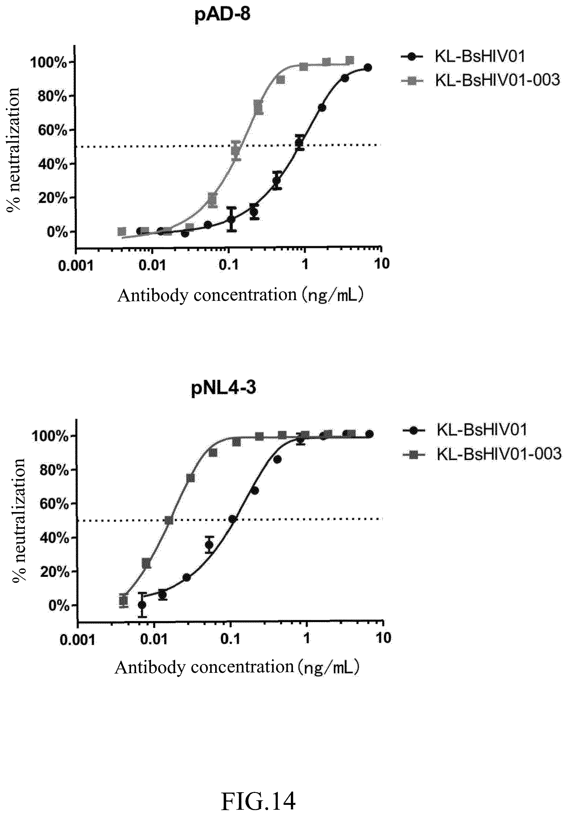

The AAV gene therapy vectors (pAAV-CBH-KL-BsHIV01-WPRE and pAAV-CBH-KL-BsHIV01-003-WPRE) were injected intramuscularly into the thigh muscles of the hind limbs of mice. Blood was collected every 1 week, the serum was separated, and the concentration of antibodies expressed in the serum was detected by ELISA.
1. The enzyme plate (Corning, Cat. No.: 42592) was coated with synthetic HIV MPER peptide. The expression supernatant and the purified and quantified KL-BsHIV01 standard were used as the primary antibody, HRP-labeled goat anti-human IgG Fc (KPL, Cat. No.: 04-10-20) was used as the secondary antibody, developed with TMB, and the OD value was detected at 450 nm with a microplate reader. The ELISA results (Figure 13) showed that the anti-HIV neutralizing antibody adeno-associated virus vector could continuously and effectively express HIV neutralizing antibodies in mice.
2. TZM-bl cells at 2E4 cells/well were plated. The serum dilutions containing the same amount of antibodies were mixed with 50 µL of HIV pAD-8 or pNL4-3, incubated at 37°C for 30 minutes, and added to TZM-bl cells. The wells where only pAD-8 or pNL4-3 was added were negative controls, and the well where HIV was not added was blank control. After overnight culture, the supernatant was discarded, and 100 µL cell lysis solution was added. After 10 minutes, the lysed cells were collected, and centrifuged at 8000 rpm for 5 minutes. 100 µL of firefly luciferase detection reagent was added to 50 µL of lysed cells. RLU (relative light unit) was measured using a multifunctional microplate reader with chemiluminescence function. The results showed that in cell experiments, both anti-HIV amphotropic (KL-BsHIV01) and tritropic (KL-BsHIV01-003) neutralizing antibodies had strong activity of neutralizing HIV (Figure 14), with the IC50s against HIV strain pAD-8 of 0.878 ng/mL and 0.150 ng/mL respectively, and the IC50s against HIV strain pNL4-3 of 0.117 ng/mL and 0.017 ng/mL respectively. It is obvious that after adding the same amount of HIV neutralizing antibody, the tritropic HIV neutralizing antibody KL-BsHIV01-003 had better neutralizing effect on pAD-8 and pNL4-3 than the amphotropic HIV neutralizing antibody KL-BsHIV01 (Figure 14). The two HIV neutralizing antibodies have stronger neutralizing activity against HIV than the reported HIV broad-spectrum neutralizing antibodies.
   SEQ ID NO: 1
      MARPLCTLLLLMATLAGALA
   SEQ ID NO: 2 
SELTQDPAVSVALKQTVTITCRGDSLRSHYASWYQKKPGQAPVLL
FYGKNNRPSGIPDRFSGSASGNRASLTITGAQAEDEADYYCSSRDK
SGSRLSVFGGGTKLTVLGGGGSGGGGSGGGGSEVRLRESGGGLVK
PGGSLRLSCSASGFDFDNAWMTWVRQPPGKGLEWVGRITGPGEG
WSVDYAESVKGRFTISRDNTKNTLYLEMNNVRTEDTGYYFCARTG
KYYDFWFGYPPGEEYFQDWGQGTLVIVSS
   SEQ ID NO: 3 
DIVMTQSPDSLAVSLGERVTMNCKSSQSLLYSTNQKNYLAWYQQ
KPGQSPKLLIYWASTRESGVPDRFSGSGSGTDFTLTISSVQAEDVA
VYYCQQYYSYRTFGGGTKLEIKRTVAGGGGSGGGGSGGGGSQVQ
LQQSGPEVVKPGASVKMSCKASGYTFTSYVIHWVRQKPGQGLDWI
GYINPYNDGTDYDEKFKGKATLTSDTSTSTAYMELSSLRSEDTAV
YYCAREKDNYATGAWFAYWGQGTLVTVSS
   SEQ ID NO: 4 
EPKSCDKTHTCPPCPAPELLGGPSVFLFPPKPKDTLMISRTPEVTCV
VVDVSHEDPEVKFNWYVDGVEVHNAKTKPREEQYNSTYRVVSVL
TVLHQDWLNGKEYKCKVSNKALPAPIEKTISKAKGQPREPQVYTL
PPSRDELTKNQVSLTCLVKGFYPSDIAVEWESNGQPENNYKTTPPV
LDSDGSFFLYSKLTVDKSRWQQGNVFSCSVMHEALHNHYTQKSLS
LSPGK
   SEQ ID NO: 5
      WEQKIEELLKKAEEQQKKNEEELKKLEK
   SEQ ID NO: 6
      YTSLIHSLIEESQNQQEKNEQELLELDKWASLWNWF
   SEQ ID NO: 7
      LLEQENKEQQNQSEEILSHILSTYNNIERDWEMW
   SEQ ID NO: 8 
MARPLCTLLLLMATLAGALASELTQDPAVSVALKQTVTITCRGDS
LRSHYASWYQKKPGQAPVLLFYGKNNRPSGIPDRFSGSASGNRAS
LTITGAQAEDEADYYCSSRDKSGSRLSVFGGGTKLTVLGGGGSGG
GGSGGGGSEVRLRESGGGLVKPGGSLRLSCSASGFDFDNAWMTW
VRQPPGKGLEWVGRITGPGEGWSVDYAESVKGRFTISRDNTKNTL
YLEMNNVRTEDTGYYFCARTGKYYDFWFGYPPGEEYFQDWGQGT
LVIVSSGGGGSGGGGSGGGGSGGGGSGGGGSDIVMTQSPDSLAVS
LGERVTMNCKSSQSLLYSTNQKNYLAWYQQKPGQSPKLLIYWAST
RESGVPDRFSGSGSGTDFTLTISSVQAEDVAVYYCQQYYSYRTFGG
GTKLEIKRTVAGGGGSGGGGSGGGGSQVQLQQSGPEVVKPGASV
 
KMSCKASGYTFTSYVIHWVRQKPGQGLDWIGYINPYNDGTDYDE
KFKGKATLTSDTSTSTAYMELSSLRSEDTAVYYCAREKDNYATGA
WFAYWGQGTLVTVSSEPKSCDKTHTCPPCPAPELLGGPSVFLFPPK
PKDTLMISRTPEVTCVVVDVSHEDPEVKFNWYVDGVEVHNAKTK
PREEQYNSTYRVVSVLTVLHQDWLNGKEYKCKVSNKALPAPIEKT
ISKAKGQPREPQVYTLPPSRDELTKNQVSLTCLVKGFYPSDIAVEW
ESNGQPENNYKTTPPVLDSDGSFFLYSKLTVDKSRWQQGNVFSCS
VMHEALHNHYTQKSLSLSPGK
   SEQ ID NO: 9 
ATGGCGAGACCCCTGTGCACATTACTTCTGTTGATGGCTACCCT
GGCAGGCGCCCTCGCCAGCGAGCTGACACAGGACCCTGCCGTGT
CCGTGGCCCTGAAGCAGACCGTGACAATCACCTGCAGAGGCGA
TTCCCTGAGATCCCACTACGCCTCCTGGTACCAGAAGAAGCCTG
GCCAGGCCCCCGTGCTGCTGTTTTACGGCAAGAATAACCGCCCC
AGCGGCATCCCCGATAGATTTTCCGGCAGCGCCTCCGGCAACAG
AGCCAGCCTGACAATCACCGGCGCCCAGGCCGAGGACGAGGCT
GATTACTACTGCAGCTCCAGAGATAAGAGCGGCAGCAGACTGTC
CGTGTTTGGCGGCGGCACCAAGCTGACCGTGCTCGGAGGAGGA
GGAAGCGGAGGAGGAGGCTCAGGCGGCGGCGGCTCTGAGGTGA
GGCTGAGAGAGTCCGGCGGCGGCCTGGTGAAGCCCGGAGGATC
TCTGAGGCTGTCCTGCTCCGCCTCCGGCTTCGATTTTGACAATGC
CTGGATGACCTGGGTGAGACAGCCCCCTGGCAAGGGCCTGGAG
TGGGTGGGAAGGATCACAGGCCCCGGCGAGGGCTGGTCCGTGG
ATTACGCCGAGTCCGTGAAGGGCAGGTTCACAATCTCCAGGGAT
AACACCAAGAACACCCTGTACCTGGAGATGAACAACGTGAGGA
CAGAGGATACCGGCTACTACTTTTGCGCCAGAACAGGCAAGTAC
TACGACTTTTGGTTCGGCTACCCCCCTGGCGAGGAGTACTTCCA
GGATTGGGGCCAGGGCACCCTGGTCATTGTGTCCAGCGGCGGCG
GCGGCAGTGGCGGCGGCGGAAGCGGCGGCGGCGGCTCTGGCGG
CGGCGGCAGCGGCGGCGGCGGCTCCGACATCGTGATGACCCAG
TCTCCTGATAGCCTGGCCGTGAGCCTGGGCGAGAGAGTGACAAT
GAACTGTAAGTCTAGCCAGAGCCTGCTGTACTCCACCAACCAGA
AGAATTACCTGGCCTGGTATCAGCAGAAGCCTGGCCAGTCCCCA
AAGCTGCTGATCTATTGGGCATCTACAAGGGAGAGCGGAGTGCC
AGACAGATTCAGCGGATCCGGATCTGGAACCGACTTCACCCTGA
CAATCTCCTCTGTGCAGGCCGAGGACGTGGCCGTGTACTATTGC
CAGCAGTACTATAGCTACAGGACATTCGGCGGCGGCACCAAGCT
GGAGATCAAGCGCACCGTGGCCGGAGGAGGAGGATCTGGCGGA
GGAGGGTCCGGCGGCGGCGGCTCCCAGGTGCAGCTGCAGCAGA
GCGGACCAGAGGTGGTGAAGCCTGGAGCCTCCGTGAAGATGTC
 
TTGTAAGGCCAGCGGCTACACCTTCACATCCTATGTGATCCACT
GGGTGAGGCAGAAGCCAGGACAGGGACTGGACTGGATCGGCTA
CATCAACCCTTATAATGATGGCACCGACTACGATGAGAAGTTTA
AGGGCAAGGCCACCCTGACATCCGATACCAGCACATCCACCGCC
TATATGGAGCTGAGCTCCCTGCGGTCTGAGGACACAGCCGTGTA
CTATTGCGCCAGAGAGAAGGATAACTACGCAACCGGAGCATGG
TTCGCATATTGGGGACAGGGTACCCTGGTCACCGTGTCTAGCGA
GCCCAAGAGCTGTGACAAGACACACACCTGCCCTCCATGTCCAG
CACCAGAGCTGCTGGGAGGGCCCTCCGTGTTCCTGTTCCCCCCT
AAGCCAAAGGATACACTGATGATCTCCAGGACACCAGAGGTGA
CCTGCGTGGTGGTGGACGTGTCTCACGAGGATCCTGAGGTGAAG
TTTAACTGGTACGTGGACGGCGTGGAGGTGCACAATGCCAAGAC
CAAGCCTAGGGAGGAGCAGTACAATTCTACATATCGCGTGGTGA
GCGTGCTGACCGTGCTGCACCAGGATTGGCTGAACGGCAAGGA
GTATAAGTGCAAGGTGAGCAATAAGGCCCTGCCTGCCCCAATCG
AGAAGACAATCTCCAAGGCAAAGGGACAGCCAAGGGAGCCTCA
GGTGTACACCCTGCCACCCTCTCGCGACGAGCTGACAAAGAACC
AGGTGAGCCTGACCTGTCTGGTGAAGGGCTTCTATCCTAGCGAT
ATTGCCGTGGAGTGGGAGTCCAATGGCCAGCCAGAGAACAATT
ACAAGACCACACCTCCAGTGCTGGACTCCGATGGCTCTTTCTTT
CTGTATTCCAAGCTGACAGTGGACAAGTCTAGATGGCAGCAGGG
CAACGTGTTTTCTTGTAGCGTGATGCACGAGGCCCTGCACAATC
ACTACACCCAGAAGTCCCTGTCTCTGAGCCCCGGCAAGTAG
   SEQ ID NO: 10 
MARPLCTLLLLMATLAGALASELTQDPAVSVALKQTVTITCRGDS
LRSHYASWYQKKPGQAPVLLFYGKNNRPSGIPDRFSGSASGNRAS
LTITGAQAEDEADYYCSSRDKSGSRLSVFGGGTKLTVLGGGGSGG
GGSGGGGSEVRLRESGGGLVKPGGSLRLSCSASGFDFDNAWMTW
VRQPPGKGLEWVGRITGPGEGWSVDYAESVKGRFTISRDNTKNTL
YLEMNNVRTEDTGYYFCARTGKYYDFWFGYPPGEEYFQDWGQGT
LVIVSSGGGGSGGGGSGGGGSGGGGSGGGGSDIVMTQSPDSLAVS
LGERVTMNCKSSQSLLYSTNQKNYLAWYQQKPGQSPKLLIYWAST
RESGVPDRFSGSGSGTDFTLTISSVQAEDVAVYYCQQYYSYRTFGG
GTKLEIKRTVAGGGGSGGGGSGGGGSQVQLQQSGPEVVKPGASV
KMSCKASGYTFTSYVIHWVRQKPGQGLDWIGYINPYNDGTDYDE
KFKGKATLTSDTSTSTAYMELSSLRSEDTAVYYCAREKDNYATGA
WFAYWGQGTLVTVSSEPKSCDKTHTCPPCPAPELLGGPSVFLFPPK
PKDTLMISRTPEVTCVVVDVSHEDPEVKFNWYVDGVEVHNAKTK
PREEQYNSTYRVVSVLTVLHQDWLNGKEYKCKVSNKALPAPIEKT
ISKAKGQPREPQVYTLPPSRDELTKNQVSLTCLVKGFYPSDIAVEW
 
ESNGQPENNYKTTPPVLDSDGSFFLYSKLTVDKSRWQQGNVFSCS
VMHEALHNHYTQKSLSLSPGKGGGGSGGGGSGGGGSGGGGSGGG
GSWEQKIEELLKKAEEQQKKNEEELKKLEK
   SEQ ID NO: 11 
ATGGCGAGACCCCTGTGCACATTACTTCTGTTGATGGCTACCCT
GGCAGGCGCCCTCGCCAGCGAGCTGACACAGGACCCTGCCGTGT
CCGTGGCCCTGAAGCAGACCGTGACAATCACCTGCAGAGGCGA
TTCCCTGAGATCCCACTACGCCTCCTGGTACCAGAAGAAGCCTG
GCCAGGCCCCCGTGCTGCTGTTTTACGGCAAGAATAACCGCCCC
AGCGGCATCCCCGATAGATTTTCCGGCAGCGCCTCCGGCAACAG
AGCCAGCCTGACAATCACCGGCGCCCAGGCCGAGGACGAGGCT
GATTACTACTGCAGCTCCAGAGATAAGAGCGGCAGCAGACTGTC
CGTGTTTGGCGGCGGCACCAAGCTGACCGTGCTCGGAGGAGGA
GGAAGCGGAGGAGGAGGCTCAGGCGGCGGCTCTGAGGTGA
GGCTGAGAGAGTCCGGCGGCGGCCTGGTGAAGCCCGGAGGATC
TCTGAGGCTGTCCTGCTCCGCCTCCGGCTTCGATTTTGACAATGC
CTGGATGACCTGGGTGAGACAGCCCCCTGGCAAGGGCCTGGAG
TGGGTGGGAAGGATCACAGGCCCCGGCGAGGGCTGGTCCGTGG
ATTACGCCGAGTCCGTGAAGGGCAGGTTCACAATCTCCAGGGAT
AACACCAAGAACACCCTGTACCTGGAGATGAACAACGTGAGGA
CAGAGGATACCGGCTACTACTTTTGCGCCAGAACAGGCAAGTAC
TACGACTTTTGGTTCGGCTACCCCCCTGGCGAGGAGTACTTCCA
GGATTGGGGCCAGGGCACCCTGGTCATTGTGTCCAGCGGCGGCG
GCGGCAGTGGCGGCGGCGGAAGCGGCGGCGGCGGCTCTGGCGG
CGGCGGCAGCGGCGGCGGCGGCTCCGACATCGTGATGACCCAG
TCTCCTGATAGCCTGGCCGTGAGCCTGGGCGAGAGAGTGACAAT
GAACTGTAAGTCTAGCCAGAGCCTGCTGTACTCCACCAACCAGA
AGAATTACCTGGCCTGGTATCAGCAGAAGCCTGGCCAGTCCCCA
AAGCTGCTGATCTATTGGGCATCTACAAGGGAGAGCGGAGTGCC
AGACAGATTCAGCGGATCCGGATCTGGAACCGACTTCACCCTGA
CAATCTCCTCTGTGCAGGCCGAGGACGTGGCCGTGTACTATTGC
CAGCAGTACTATAGCTACAGGACATTCGGCGGCGGCACCAAGCT
GGAGATCAAGCGCACCGTGGCCGGAGGAGGAGGATCTGGCGGA
GGAGGGTCCGGCGGCGGCGGCTCCCAGGTGCAGCTGCAGCAGA
GCGGACCAGAGGTGGTGAAGCCTGGAGCCTCCGTGAAGATGTC
TTGTAAGGCCAGCGGCTACACCTTCACATCCTATGTGATCCACT
GGGTGAGGCAGAAGCCAGGACAGGGACTGGACTGGATCGGCTA
CATCAACCCTTATAATGATGGCACCGACTACGATGAGAAGTTTA
AGGGCAAGGCCACCCTGACATCCGATACCAGCACATCCACCGCC
TATATGGAGCTGAGCTCCCTGCGGTCTGAGGACACAGCCGTGTA
 
CTATTGCGCCAGAGAGAAGGATAACTACGCAACCGGAGCATGG
TTCGCATATTGGGGACAGGGTACCCTGGTCACCGTGTCTAGCGA
GCCCAAGAGCTGTGACAAGACACACACCTGCCCTCCATGTCCAG
CACCAGAGCTGCTGGGAGGGCCCTCCGTGTTCCTGTTTCCCCCT
AAGCCAAAGGATACACTGATGATCTCCAGGACACCAGAGGTGA
CCTGCGTGGTGGTGGACGTGTCTCACGAGGATCCTGAGGTGAAG
TTTAACTGGTACGTGGACGGCGTGGAGGTGCACAATGCCAAGAC
CAAGCCTAGGGAGGAGCAGTACAATTCTACATATCGCGTGGTGA
GCGTGCTGACCGTGCTGCACCAGGATTGGCTGAACGGCAAGGA
GTATAAGTGCAAGGTGAGCAATAAGGCCCTGCCTGCCCCAATCG
AGAAGACAATCTCCAAGGCAAAGGGACAGCCAAGGGAGCCTCA
GGTGTACACCCTGCCACCCTCTCGCGACGAGCTGACAAAGAACC
AGGTGAGCCTGACCTGTCTGGTGAAGGGCTTCTATCCTAGCGAT
ATTGCCGTGGAGTGGGAGTCCAATGGCCAGCCAGAGAACAATT
ACAAGACCACACCTCCAGTGCTGGACTCCGATGGCTCTTTCTTT
CTGTATTCCAAGCTGACAGTGGACAAGTCTAGATGGCAGCAGGG
CAACGTGTTTTCTTGTAGCGTGATGCACGAGGCCCTGCACAATC
ACTACACCCAGAAGTCCCTGTCTCTGAGCCCCGGCAAGGGCGGA
GGAGGAAGCGGAGGAGGCGGCTCTGGCGGAGGAGGTTCCGGAG
GAGGCGGAAGCGGCGGAGGAGGCTCTTGGGAGCAGAAGATCGA
GGAGCTGCTGAAGAAGGCCGAGGAGCAGCAGAAGAAGAATGA
GGAGGAGCTGAAGAAGCTGGAGAAGTAG
   SEQ ID NO: 12 
MARPLCTLLLLMATLAGALASELTQDPAVSVALKQTVTITCRGDS
LRSHYASWYQKKPGQAPVLLFYGKNNRPSGIPDRFSGSASGNRAS
LTITGAQAEDEADYYCSSRDKSGSRLSVFGGGTKLTVLGGGGSGG
GGSGGGGSEVRLRESGGGLVKPGGSLRLSCSASGFDFDNAWMTW
VRQPPGKGLEWVGRITGPGEGWSVDYAESVKGRFTISRDNTKNTL
YLEMNNVRTEDTGYYFCARTGKYYDFWFGYPPGEEYFQDWGQGT
LVIVSSGGGGSGGGGSGGGGSGGGGSGGGGSDIVMTQSPDSLAVS
LGERVTMNCKSSQSLLYSTNQKNYLAWYQQKPGQSPKLLIYWAST
RESGVPDRFSGSGSGTDFTLTISSVQAEDVAVYYCQQYYSYRTFGG
GTKLEIKRTVAGGGGSGGGGSGGGGSQVQLQQSGPEVVKPGASV
KMSCKASGYTFTSYVIHWVRQKPGQGLDWIGYINPYNDGTDYDE
KFKGKATLTSDTSTSTAYMELSSLRSEDTAVYYCAREKDNYATGA
WFAYWGQGTLVTVSSEPKSCDKTHTCPPCPAPELLGGPSVFLFPPK
PKDTLMISRTPEVTCVVVDVSHEDPEVKFNWYVDGVEVHNAKTK
PREEQYNSTYRVVSVLTVLHQDWLNGKEYKCKVSNKALPAPIEKT
ISKAKGQPREPQVYTLPPSRDELTKNQVSLTCLVKGFYPSDIAVEW
ESNGQPENNYKTTPPVLDSDGSFFLYSKLTVDKSRWQQGNVFSCS
 
VMHEALHNHYTQKSLSLSPGKGGGGSGGGGSGGGGSGGGGSGGG
GS YTSLIHSLIEESQNQQEKNEQELLELDKWASLWNWF
   SEQ ID NO: 13 
ATGGCGAGACCCCTGTGCACATTACTTCTGTTGATGGCTACCCT
GGCAGGCGCCCTCGCCTCCGAGCTGACCCAGGACCCTGCCGTCA
GCGTGGCCTTGAAACAGACCGTAACTATAACGTGCAGAGGTGAT
AGCTTAAGATCCCACTACGCCAGCTGGTATCAAAAGAAGCCAG
GTCAGGCTCCCGTGCTCCTGTTCTACGGGAAGAATAACCGCCCA
TCCGGCATTCCGGATCGGTTCTCCGGAAGCGCAAGTGGAAATCG
CGCCTCGCTGACAATAACAGGAGCGCAGGCAGAGGACGAAGCA
GACTACTATTGTTCAAGTAGGGATAAGAGCGGATCCAGGCTGAG
CGTGTTCGGCGGAGGCACCAAGCTGACAGTGCTTGGTGGGGGG
GGCTCCGGCGGCGGCGGCAGCGGAGGAGGGGGAAGTGAGGTTA
GACTGAGGGAGTCCGGCGGGGGGTTAGTCAAGCCAGGGGGCAG
TCTCCGGCTTTCCTGCTCAGCTAGTGGCTTCGATTTCGATAACGC
GTGGATGACTTGGGTCCGCCAGCCTCCCGGGAAAGGGCTCGAGT
GGGTGGGAAGAATCACAGGCCCCGGCGAGGGATGGTCCGTTGA
CTATGCAGAGTCTGTCAAGGGTCGGTTCACTATCAGCCGAGATA
ATACCAAGAACACTCTGTACTTGGAGATGAATAACGTGAGAAC
AGAAGACACAGGATACTACTTCTGTGCTCGTACTGGCAAATATT
ATGACTTTTGGTTCGGGTACCCACCGGGAGAGGAGTATTTCCAG
GACTGGGGCCAGGGTACACTGGTCATTGTGTCTTCTGGTGGCGG
AGGGTCTGGCGGAGGCGGATCCGGCGGAGGTGGCTCAGGCGGA
GGAGGGAGTGGCGGCGGCGGTTCTGACATCGTAATGACTCAGTC
ACCAGACTCTCTGGCCGTATCCTTAGGAGAGCGCGTGACGATGA
ACTGCAAGTCTTCTCAGTCACTGCTTTACAGCACAAACCAAAAA
AACTACCTGGCTTGGTACCAGCAGAAGCCTGGACAGAGCCCCA
AGCTACTGATCTATTGGGCCTCGACTAGAGAAAGTGGGGTGCCC
GACAGATTTTCGGGGAGTGGCAGCGGTACAGATTTTACACTCAC
CATTTCCTCAGTGCAGGCAGAAGATGTGGCCGTGTACTATTGCC
AACAGTATTACAGCTATCGGACATTTGGTGGTGGCACAAAGCTA
GAGATTAAGAGGACCGTGGCCGGAGGTGGCGGCAGTGGAGGGG
GCGGGAGTGGGGGCGGAGGTTCACAGGTGCAGCTGCAACAGTC
CGGGCCTGAAGTGGTTAAACCCGGAGCGAGCGTAAAGATGTCTT
GCAAGGCTTCCGGTTATACATTCACATCATATGTCATTCACTGG
GTGAGGCAAAAGCCAGGGCAAGGTCTTGATTGGATAGGGTACA
TCAATCCTTACAATGATGGGACTGACTACGATGAGAAGTTCAAG
GGAAAGGCAACCCTGACTTCTGATACCTCCACGTCTACCGCTTA
CATGGAACTAAGCTCCCTGCGTTCAGAGGACACTGCCGTGTACT
ATTGTGCTCGGGAGAAAGATAACTATGCCACCGGCGCCTGGTTT
 
GCCTACTGGGGCCAAGGCACCTTGGTGACCGTTAGCTCCGAGCC
GAAATCGTGCGACAAGACCCACACCTGTCCCCCCTGTCCTGCAC
CTGAGCTCCTGGGTGGGCCCTCTGTATTCCTCTTCCCTCCAAAAC
CAAAAGACACGTTGATGATTAGCAGGACCCCAGAAGTAACATG
CGTTGTGGTCGACGTCTCACATGAAGACCCAGAGGTGAAATTTA
ATTGGTACGTCGATGGGGTTGAAGTTCATAACGCCAAAACTAAG
CCGAGGGAGGAACAGTATAATAGCACTTATCGAGTGGTTTCAGT
TTTGACCGTACTTCATCAAGACTGGCTGAACGGGAAGGAATATA
AGTGCAAGGTGAGTAATAAAGCACTGCCTGCGCCCATCGAGAA
AACGATCAGTAAAGCCAAGGGACAGCCTCGCGAACCCCAGGTG
TACACTCTTCCGCCCTCCCGGGACGAGCTCACCAAAAACCAGGT
GTCCTTGACCTGTCTCGTCAAGGGCTTTTACCCATCAGATATCGC
TGTCGAATGGGAAAGTAACGGGCAGCCTGAAAACAATTACAAA
ACAACCCCACCCGTCCTCGATTCAGATGGCAGCTTTTTTTTATAC
TCTAAACTTACGGTGGACAAATCTCGCTGGCAACAGGGGAATGT
GTTTAGCTGCAGCGTCATGCACGAAGCTCTGCATAACCACTATA
CCCAGAAAAGCCTGTCTCTCAGCCCCGGGAAAGGCGGAGGAGG
AAGCGGAGGAGGCGGCTCTGGCGGAGGAGGTTCCGGAGGAGGC
GGAAGCGGCGGAGGAGGCTCTTACACCTCCCTGATCCACTCCCT
GATCGAGGAGTCCCAGAATCAGCAGGAGAAGAATGAGCAGGAG
CTGCTGGAGCTGGATAAGTGGGCCTCCCTGTGGAATTGGTTCTA
G
   SEQ ID NO: 14 
MARPLCTLLLLMATLAGALASELTQDPAVSVALKQTVTITCRGDS
LRSHYASWYQKKPGQAPVLLFYGKNNRPSGIPDRFSGSASGNRAS
LTITGAQAEDEADYYCSSRDKSGSRLSVFGGGTKLTVLGGGGSGG
GGSGGGGSEVRLRESGGGLVKPGGSLRLSCSASGFDFDNAWMTW
VRQPPGKGLEWVGRITGPGEGWSVDYAESVKGRFTISRDNTKNTL
YLEMNNVRTEDTGYYFCARTGKYYDFWFGYPPGEEYFQDWGQGT
LVIVSSGGGGSGGGGSGGGGSGGGGSGGGGSDIVMTQSPDSLAVS
LGERVTMNCKSSQSLLYSTNQKNYLAWYQQKPGQSPKLLIYWAST
RESGVPDRFSGSGSGTDFTLTISSVQAEDVAVYYCQQYYSYRTFGG
GTKLEIKRTVAGGGGSGGGGSGGGGSQVQLQQSGPEVVKPGASV
KMSCKASGYTFTSYVIHWVRQKPGQGLDWIGYINPYNDGTDYDE
KFKGKATLTSDTSTSTAYMELSSLRSEDTAVYYCAREKDNYATGA
WFAYWGQGTLVTVSSEPKSCDKTHTCPPCPAPELLGGPSVFLFPPK
PKDTLMISRTPEVTCVVVDVSHEDPEVKFNWYVDGVEVHNAKTK
PREEQYNSTYRVVSVLTVLHQDWLNGKEYKCKVSNKALPAPIEKT
ISKAKGQPREPQVYTLPPSRDELTKNQVSLTCLVKGFYPSDIAVEW
ESNGQPENNYKTTPPVLDSDGSFFLYSKLTVDKSRWQQGNVFSCS
 
VMHEALHNHYTQKSLSLSPGKGGGGSGGGGSGGGGSGGGGSGGG
GS LLEQENKEQQNQSEEILSHILSTYNNIERDWEMW
   SEQ ID NO: 15 
ATGGCGAGACCCCTGTGCACATTACTTCTGTTGATGGCTACCCT
GGCAGGCGCCCTCGCCAGCGAGCTGACACAGGACCCTGCCGTGT
CCGTGGCCCTGAAGCAGACCGTGACAATCACCTGCAGAGGCGA
TTCCCTGAGATCCCACTACGCCTCCTGGTACCAGAAGAAGCCTG
GCCAGGCCCCCGTGCTGCTGTTTTACGGCAAGAATAACCGCCCC
AGCGGCATCCCCGATAGATTTTCCGGCAGCGCCTCCGGCAACAG
AGCCAGCCTGACAATCACCGGCGCCCAGGCCGAGGACGAGGCT
GATTACTACTGCAGCTCCAGAGATAAGAGCGGCAGCAGACTGTC
CGTGTTTGGCGGCGGCACCAAGCTGACCGTGCTCGGAGGAGGA
GGAAGCGGAGGAGGAGGCTCAGGCGGCGGCGGCTCTGAGGTGA
GGCTGAGAGAGTCCGGCGGCGGCCTGGTGAAGCCCGGAGGATC
TCTGAGGCTGTCCTGCTCCGCCTCCGGCTTCGATTTTGACAATGC
CTGGATGACCTGGGTGAGACAGCCCCCTGGCAAGGGCCTGGAG
TGGGTGGGAAGGATCACAGGCCCCGGCGAGGGCTGGTCCGTGG
ATTACGCCGAGTCCGTGAAGGGCAGGTTCACAATCTCCAGGGAT
AACACCAAGAACACCCTGTACCTGGAGATGAACAACGTGAGGA
CAGAGGATACCGGCTACTACTTTTGCGCCAGAACAGGCAAGTAC
TACGACTTTTGGTTCGGCTACCCCCCTGGCGAGGAGTACTTCCA
GGATTGGGGCCAGGGCACCCTGGTCATTGTGTCCAGCGGCGGCG
GCGGCAGTGGCGGCGGCGGAAGCGGCGGCGGCGGCTCTGGCGG
CGGCGGCAGCGGCGGCGGCGGCTCCGACATCGTGATGACCCAG
TCTCCTGATAGCCTGGCCGTGAGCCTGGGCGAGAGAGTGACAAT
GAACTGTAAGTCTAGCCAGAGCCTGCTGTACTCCACCAACCAGA
AGAATTACCTGGCCTGGTATCAGCAGAAGCCTGGCCAGTCCCCA
AAGCTGCTGATCTATTGGGCATCTACAAGGGAGAGCGGAGTGCC
AGACAGATTCAGCGGATCCGGATCTGGAACCGACTTCACCCTGA
CAATCTCCTCTGTGCAGGCCGAGGACGTGGCCGTGTACTATTGC
CAGCAGTACTATAGCTACAGGACATTCGGCGGCGGCACCAAGCT
GGAGATCAAGCGCACCGTGGCCGGAGGAGGAGGATCTGGCGGA
GGAGGGTCCGGCGGCGGCGGCTCCCAGGTGCAGCTGCAGCAGA
GCGGACCAGAGGTGGTGAAGCCTGGAGCCTCCGTGAAGATGTC
TTGTAAGGCCAGCGGCTACACCTTCACATCCTATGTGATCCACT
GGGTGAGGCAGAAGCCAGGACAGGGACTGGACTGGATCGGCTA
CATCAACCCTTATAATGATGGCACCGACTACGATGAGAAGTTTA
AGGGCAAGGCCACCCTGACATCCGATACCAGCACATCCACCGCC
TATATGGAGCTGAGCTCCCTGCGGTCTGAGGACACAGCCGTGTA
CTATTGCGCCAGAGAGAAGGATAACTACGCAACCGGAGCATGG
 
TTCGCATATTGGGGACAGGGTACCCTGGTCACCGTGTCTAGCGA
GCCCAAGAGCTGTGACAAGACACACACCTGCCCTCCATGTCCAG
CACCAGAGCTGCTGGGAGGGCCCTCCGTGTTCCTGTTTCCCCCT
AAGCCAAAGGATACACTGATGATCTCCAGGACACCAGAGGTGA
CCTGCGTGGTGGTGGACGTGTCTCACGAGGATCCTGAGGTGAAG
TTTAACTGGTACGTGGACGGCGTGGAGGTGCACAATGCCAAGAC
CAAGCCTAGGGAGGAGCAGTACAATTCTACATATCGCGTGGTGA
GCGTGCTGACCGTGCTGCACCAGGATTGGCTGAACGGCAAGGA
GTATAAGTGCAAGGTGAGCAATAAGGCCCTGCCTGCCCCAATCG
AGAAGACAATCTCCAAGGCAAAGGGACAGCCAAGGGAGCCTCA
GGTGTACACCCTGCCACCCTCTCGCGACGAGCTGACAAAGAACC
AGGTGAGCCTGACCTGTCTGGTGAAGGGCTTCTATCCTAGCGAT
ATTGCCGTGGAGTGGGAGTCCAATGGCCAGCCAGAGAACAATT
ACAAGACCACACCTCCAGTGCTGGACTCCGATGGCTCTTTCTTT
CTGTATTCCAAGCTGACAGTGGACAAGTCTAGATGGCAGCAGGG
CAACGTGTTTTCTTGTAGCGTGATGCACGAGGCCCTGCACAATC
ACTACACCCAGAAGTCCCTGTCTCTGAGCCCCGGCAAGGGCGGA
GGAGGAAGCGGAGGAGGCGGCTCTGGCGGAGGAGGTTCCGGAG
GAGGCGGAAGCGGCGGAGGAGGCTCTCTGCTGGAGCAGGAGAA
CAAGGAGCAGCAGAATCAGAGCGAGGAGATCCTGTCCCACATC
CTGTCCACATACAACAATATCGAGAGAGATTGGGAGATGTGGTA
G
   SEQ ID NO: 16 
GGCCGCGTTGCTGGCGTTTTTCCATAGGCTCCGCCCCCCTGACGAGCAT
CACAAAAATCGACGCTCAAGTCAGAGGTGGCGAAACCCGACAGGACTA
TAAAGATACCAGGCGTTTCCCCCTGGAAGCTCCCTCGTGCGCTCTCCTG
TTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTA
AGACACGACTTATCGCCACTGGCAGCAGCCACTGGTAACAGGATTAGC
AGAGCGAGGTATGTAGGCGGTGCTACAGAGTTCTTGAAGTGGTGGCCT
AACTACGGCTACACTAGAAGAACAGTATTTGGTATCTGCGCTCTGCTGA
AGCCAGTTACCTTCGGAAAAAGAGTTGGTAGCTCTTGATCCGGCAAAC
AAACCACCGCTGGTAGCGGTGGTTTTTTTGTTTGCAAGCAGCAGATTAC
GCGCAGAAAAAAAGGATCTCAAGAAGATCCTTTGATCTTTTCTACGGG
GTCTGACGCTCAGTGGAACGAAAACTCACGTTAAGGGATTTTGGTCAT
GAAGCGCTTTTGAAGCTCGGATCCGAACAAACGACCCAACACCCGTGC
GTTTTATTCTGTCTTTTTATTGCCGATCCCCTCAGAAGAACTCGTCAAGA
AGGCGATAGAAGGCGATGCGCTGCGAATCGGGAGCGGCGATACCGTA
 
AAGCACGAGGAAGCGGTCAGCCCATTCGCCGCCAAGCTCTTCAGCAAT
ATCACGGGTAGCCAACGCTATGTCCTGATAGCGGTCCGCCACACCCAG
CCGGCCACAGTCGATGAATCCAGAAAAGCGGCCATTTTCCACCATGAT
ATTCGGCAAGCAGGCATCGCCATGGGTCACGACGAGATCCTCGCCGTC
GGGCATGCTCGCCTTGAGCCTGGCGAACAGTTCGGCTGGCGCGAGCCC
CTGATGCTCTTCGTCCAGATCATCCTGATCGACAAGACCGGCTTCCATC
CGAGTACGTGCTCGCTCGATGCGATGTTTCGCTTGGTGGTCGAATGGGC
AGGTAGCCGGATCAAGCGTATGCAGCCGCCGCATTGCATCAGCCATGA
TGGATACTTTCTCGGCAGGAGCAAGGTGAGATGACAGGAGATCCTGCC
CCGGCACTTCGCCCAATAGCAGCCAGTCCCTTCCCGCTTCAGTGACAAC
GTCGAGCACAGCTGCGCAAGGAACGCCCGTCGTGGCCAGCCACGATAG
CCGCGCTGCCTCGTCTTGCAGTTCATTCAGGGCACCGGACAGGTCGGTC
TTGACAAAAAGAACCGGGCGCCCCTGCGCTGACAGCCGGAACACGGCG
GCATCAGAGCAGCCGATTGTCTGTTGTGCCCAGTCATAGCCGAATAGCC
TCTCCACCCAAGCGGCCGGAGAACCTGCGTGCAATCCATCTTGTTCAAT
CATGCGAAACGATCCTCATCCTGTCTCTTGATCAGAGCTTGATCCCCTG
CGCCATCAGATCCTTGGCGGCAAGAAAGCCATCCAGTTTACTTTGCAGG
GCTTCCCAACCTTACCAGAGGCCTGCGCCGCGGCCAGCTGGCTAGCAA
TTCCCGGGTTAACTCTAGAGACATTGATTATTGACTAGTTATTAATAGT
AATCAATTACGGGGTCATTAGTTCATAGCCCATATATGGAGTTCCGCGT
TACATAACTTACGGTAAATGGCCCGCCTGGCTGACCGCCCAACGACCC
CCGCCCATTGACGTCAATAATGACGTATGTTCCCATAGTAACGCCAATA
GGGACTTTCCATTGACGTCAATGGGTGGAGTATTTACGGTAAACTGCCC
ACTTGGCAGTACATCAAGTGTATCATATGCCAAGTACGCCCCCTATTGA
CGTCAATGACGGTAAATGGCCCGCCTGGCATTATGCCCAGTACATGAC
CTTATGGGACTTTCCTACTTGGCAGTACATCTACGTATTAGTCATCGCT
ATTACCATGGTGATGCGGTTTTGGCAGTACATCAATGGGCGTGGATAGC
GGTTTGACTCACGGGGATTTCCAAGTCTCCACCCCATTGACGTCAATGG
GAGTTTGTTTTGGCACCAAAATCAACGGGACTTTCCAAAATGTCGTAAC
AACTCCGCCCCATTGACGCAAATGGGCGGTAGGCGTGTACGGTGGGAG
GTCTATATAAGCAGAGCTCGTTTAGTGAACCGGGGTCTCTCTGGTTAGA
CCAGATCTGAGCCTGGGAGCTCTCTGGCTAACTAGGGAACCCACTGCTT
AAGCCTCAATAAAGCTTGCCTTGAGTGCTTCAAGTAGTGTGTGCCCGTC
TGTTGTGTGACTCTGGTAACTAGAGATCCCTCAGACCCTTTTAGTCAGT
GTGGAAAATCTCTAGCAGTGGCGCCCGAACAGGGACTTGAAAGCGAAA
GGGAAACCAGAGGAGCTCTCTCGACGCAGGACTCGGCTTGCTGAAGCG
CGCACGGCAAGAGGCGAGGGGCGGCGACTGGTGAGTACGCCAAAAAT
TTTGACTAGCGGAGGCTAGAAGGAGAGAGATGGGTGCGAGAGCGTCA
GTATTAAGCGGGGGAGAATTAGATCGCGATGGGAAAAAATTCGGTTAA
GGCCAGGGGGAAAGAAAAAATATAAATTAAAACATATAGTATGGGCA
 
AGCAGGGAGCTAGAACGATTCGCAGTTAATCCTGGCCTGTTAGAAACA
TCAGAAGGCTGTAGACAAATACTGGGACAGCTACAACCATCCCTTCAG
ACAGGATCAGAAGAACTTAGATCATTATATAATACAGTAGCAACCCTC
TATTGTGTGCATCAAAGGATAGAGATAAAAGACACCAAGGAAGCTTTA
GACAAGATAGAGGAAGAGCAAAACAAAAGTAAGACCACCGCACAGCA
AGCGGCCGCTGATCTTCAGACCTGGAGGAGGAGATATGAGGGACAATT
GGAGAAGTGAATTATATAAATATAAAGTAGTAAAAATTGAACCATTAG
GAGTAGCACCCACCAAGGCAAAGAGAAGAGTGGTGCAGAGAGAAAAA
AGAGCAGTGGGAATAGGAGCTTTGTTCCTTGGGTTCTTGGGAGCAGCA
GGAAGCACTATGGGCGCAGCGTCAATGACGCTGACGGTACAGGCCAGA
CAATTATTGTCTGGTATAGTGCAGCAGCAGAACAATTTGCTGAGGGCTA
TTGAGGCGCAACAGCATCTGTTGCAACTCACAGTCTGGGGCATCAAGC
AGCTCCAGGCAAGAATCCTGGCTGTGGAAAGATACCTAAAGGATCAAC
AGCTCCTGGGGATTTGGGGTTGCTCTGGAAAACTCATTTGCACCACTGC
TGTGCCTTGGAATGCTAGTTGGAGTAATAAATCTCTGGAACAGATTTGG
AATCACACGACCTGGATGGAGTGGGACAGAGAAATTAACAATTACACA
AGCTTAATACACTCCTTAATTGAAGAATCGCAAAACCAGCAAGAAAAG
AATGAACAAGAATTATTGGAATTAGATAAATGGGCAAGTTTGTGGAAT
TGGTTTAACATAACAAATTGGCTGTGGTATATAAAATTATTCATAATGA
TAGTAGGAGGCTTGGTAGGTTTAAGAATAGTTTTTGCTGTACTTTCTAT
AGTGAATAGAGTTAGGCAGGGATATTCACCATTATCGTTTCAGACCCAC
CTCCCAACCCCGAGGGGACCCGACAGGCCCGAAGGAATAGAAGAAGA
AGGTGGAGAGAGAGACAGAGACAGATCCATTCGATTAGTGAACGGATC
TCGACGGTATCGGTTAACTTTTAAAAGAAAAGGGGGGATTGGGGGGTA
CAGTGCAGGGGAAAGAATAGTAGACATAATAGCAACAGACATACAAA
CTAAAGAATTACAAAAACAAATTACAAAAATTCAAAATTTTATCGATC
ACGAGACTAGCCTCGAGGCATGCCTGCAGGAATTCGCTCCGGTGCCCG
TCAGTGGGCAGAGCGCACATCGCCCACAGTCCCCGAGAAGTTGGGGGG
AGGGGTCGGCAATTGAACCGGTGCCTAGAGAAGGTGGCGCGGGGTAA
ACTGGGAAAGTGATGTCGTGTACTGGCTCCGCCTTTTTCCCGAGGGTGG
GGGAGAACCGTATATAAGTGCAGTAGTCGCCGTGAACGTTCTTTTTCGC
AACGGGTTTGCCGCCAGAACACAGGTAAGTGCCGTGTGTGGTTCCCGC
GGGCCTGGCCTCTTTACGGGTTATGGCCCTTGCGTGCCTTGAATTACTT
CCACGCCCCTGGCTGCAGTACGTGATTCTTGATCCCGAGCTTCGGGTTG
GAAGTGGGTGGGAGAGTTCGAGGCCTTGCGCTTAAGGAGCCCCTTCGC
CTCGTGCTTGAGTTGAGGCCTGGCTTGGGCGCTGGGGCCGCCGCGTGCG
AATCTGGTGGCACCTTCGCGCCTGTCTCGCTGCTTTCGATAAGTCTCTA
GCCATTTAAAATTTTTGATGACCTGCTGCGACGCTTTTTTTCTGGCAAG
ATAGTCTTGTAAATGCGGGCCAAGATCTGCACACTGGTATTTCGGTTTT
TGGGGCCGCGGGCGGCGACGGGGCCCGTGCGTCCCAGCGCACATGTTC
 
GGCGAGGCGGGGCCTGCGAGCGCGGCCACCGAGAATCGGACGGGGGT
AGTCTCAAGCTGGCCGGCCTGCTCTGGTGCCTGGCCTCGCGCCGCCGTG
TATCGCCCCGCCCTGGGCGGCAAGGCTGGCCCGGTCGGCACCAGTTGC
GTGAGCGGAAAGATGGCCGCTTCCCGGCCCTGCTGCAGGGAGCTCAAA
ATGGAGGACGCGGCGCTCGGGAGAGCGGGCGGGTGAGTCACCCACAC
AAAGGAAAAGGGCCTTTCCGTCCTCAGCCGTCGCTTCATGTGACTCCAC
GGAGTACCGGGCGCCGTCCAGGCACCTCGATTAGTTCTCGAGCTTTTGG
AGTACGTCGTCTTTAGGTTGGGGGGAGGGGTTTTATGCGATGGAGTTTC
CCCACACTGAGTGGGTGGAGACTGAAGTTAGGCCAGCTTGGCACTTGA
TGTAATTCTCCTTGGAATTTGCCCTTTTTGAGTTTGGATCTTGGTTCATT
CTCAAGCCTCAGACAGTGGTTCAAAGTTTTTTTCTTCCATTTCAGGTGTC
GTGAGGATCTATTTCCGGTGAGACCCAAGCTGGCTAGCTAAACTTACGC
GTGCCTCGGATCCTCCAGTGTGGTGTGCAGATATCCAGCACAGTCCCGG
GCCGAGTCTAGACGTTTAAACCCGCTGATCAGGTCGACAATCAACCTCT
GGATTACAAAATTTGTGAAAGATTGACTGGTATTCTTAACTATGTTGCT
CCTTTTACGCTATGTGGATACGCTGCTTTAATGCCTTTGTATCATGCTAT
TGCTTCCCGTATGGCTTTCATTTTCTCCTCCTTGTATAAATCCTGGTTGC
TGTCTCTTTATGAGGAGTTGTGGCCCGTTGTCAGGCAACGTGGCGTGGT
GTGCACTGTGTTTGCTGACGCAACCCCCACTGGTTGGGGCATTGCCACC
ACCTGTCAGCTCCTTTCCGGGACTTTCGCTTTCCCCCTCCCTATTGCCAC
GGCGGAACTCATCGCCGCCTGCCTTGCCCGCTGCTGGACAGGGGCTCG
GCTGTTGGGCACTGACAATTCCGTGGTGTTGTCGGGGAAGCTGACGTCC
TTTCCATGGCTGCTCGCCTGTGTTGCCACCTGGATTCTGCGCGGGACGT
CCTTCTGCTACGTCCCTTCGGCCCTCAATCCAGCGGACCTTCCTTCCCGC
GGCCTGCTGCCGGCTCTGCGGCCTCTTCCGCGTCTTCGCCTTCGCCCTCA
GACGAGTCGGATCTCCCTTTGGGCCGCCTCCCCGCGGTACCTTTAAGAC
CAATGACTTACAAGGCAGCTGTAGATCTTAGCCACTTTTTAAAAGAAA
AGGGGGGACTGGAAGGGCTAATTCACTCCCAACGAAGACAAGATCTGC
TTTTTGCTTGTACTGGGTCTCTCTGGTTAGACCAGATCTGAGCCTGGGA
GCTCTCTGGCTAACTAGGGAACCCACTGCTTAAGCCTCAATAAAGCTTG
CCTTGAGTGCTTCAAGTAGTGTGTGCCCGTCTGTTGTGTGACTCTGGTA
ACTAGAGATCCCTCAGACCCTTTTAGTCAGTGTGGAAAATCTCTAGCAG
TAGTAGTTCATGTCATCTTATTATTCAGTATTTATAACTTGCAAAGAAA
TGAATATCAGAGAGTGAGAGGAACTTGTTTATTGCAGCTTATAATGGTT
ACAAATAAAGCAATAGCATCACAAATTTCACAAATAAAGCATTTTTTTC
ACTGCATTCTAGTTGTGGTTTGTCCAAACTCATCAATGTATCTTATCATG
TCTGGCTCTAGCTATCCCGCCCCTAACTCCGCCCATCCCGCCCCTAACT
CCGCCCAGTTCCGCCCATTCTCCGCCCCATGGCTGACTAATTTTTTTTAT
TTATGCAGAGGCCGAGGCCGCCTCGGCCTCTGAGCTATTCCAGAAGTA
GTGAGGAGGCTTTTTTGGAGGCCGCTAGCGTCGACCATTACTTATTGTT
 
TTAGCTGTCCTCATGAATGTCTTTTCACTACCCATTTGCTTATCCTGCAT
CTCTCAGCCTTGACTCCACTCAGTTCTCTTGCTTAGAGATACCACCTTTC
CCCTGAAGTGTTCCTTCCATGTTTTACGGCGAGATGGTTTCTCCTCGCCT
GGCCACTCAGCCTTAGTTGTCTCTGTTGTCTTATAGAGGTCTACTTGAA
GAAGGAAAAACAGGGGGCATGGTTTGACTGTCCTGTGAGCCCTTCTTC
CCTGCCTCCCCCACTCACAGTGACCCGGAATCCCTCGACATGGCAGTCT
AGCACTAGTGCGGCCGCAGATCTGCTTCCTCGCTCACTGACTCGCTGCG
CTCGGTCGTTCGGCTGCGGCGAGCGGTATCAGCTCACTCAAAGGCGGT
AATACGGTTATCCACAGAATCAGGGGATAACGCAGGAAAGAACATGTG
AGCAAAAGGCCAGCAAAAGGCCAGGAACCGTAAAAA
   SEQ ID NO: 17 
CGTTACATAACTTACGGTAAATGGCCCGCCTGGCTGACCGCCCAACGA
CCCCCGCCCATTGACGTCAATAATGACGTATGTTCCCATAGTAACGCCA
ATAGGGACTTTCCATTGACGTCAATGGGTGGAGTATTTACGGTAAACTG
CCCACTTGGCAGTACATCAAGTGTATCATATGCCAAGTACGCCCCCTAT
TGACGTCAATGACGGTAAATGGCCCGCCTGGCATTATGCCCAGTACAT
GACCTTATGGGACTTTCCTACTTGGCAGTACATCTACGTATTAGTCATC
GCTATTACCATGGTCGAGGTGAGCCCCACGTTCTGCTTCACTCTCCCCA
TCTCCCCCCCCTCCCCACCCCCAATTTTGTATTTATTTATTTTTTAATTAT
TTTGTGCAGCGATGGGGGCGGGGGGGGGGGGGGGGCGCGCGCCAGGC
GGGGCGGGGCGGGGCGAGGGGCGGGGCGGGGCGAGGCGGAGAGGTGC
GGCGGCAGCCAATCAGAGCGGCGCGCTCCGAAAGTTTCCTTTTATGGC
GAGGCGGCGGCGGCGGCGGCCCTATAAAAAGCGAAGCGCGCGGCGGG
CGGGAGTCGCTGCGACGCTGCCTTCGCCCCGTGCCCCGCTCCGCCGCCG
CCTCGCGCCGCCCGCCCCGGCTCTGACTGACCGCGTTACTCCCACAGGT
GAGCGGGCGGGACGGCCCTTCTCCTCCGGGCTGTAATTAGCTGAGCAA
GAGGTAAGGGTTTAAGGGATGGTTGGTTGGTGGGGTATTAATGTTTAAT
TACCTGGAGCACCTGCCTGAAATCACTTTTTTTCAGGT
   SEQ ID NO: 18 
GGCCGCGTTGCTGGCGTTTTTCCATAGGCTCCGCCCCCCTGACGAGCAT
CACAAAAATCGACGCTCAAGTCAGAGGTGGCGAAACCCGACAGGACTA
TAAAGATACCAGGCGTTTCCCCCTGGAAGCTCCCTCGTGCGCTCTCCTG
TTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTA
AGACACGACTTATCGCCACTGGCAGCAGCCACTGGTAACAGGATTAGC
AGAGCGAGGTATGTAGGCGGTGCTACAGAGTTCTTGAAGTGGTGGCCT
AACTACGGCTACACTAGAAGAACAGTATTTGGTATCTGCGCTCTGCTGA
AGCCAGTTACCTTCGGAAAAAGAGTTGGTAGCTCTTGATCCGGCAAAC
 
AAACCACCGCTGGTAGCGGTGGTTTTTTTGTTTGCAAGCAGCAGATTAC
GCGCAGAAAAAAAGGATCTCAAGAAGATCCTTTGATCTTTTCTACGGG
GTCTGACGCTCAGTGGAACGAAAACTCACGTTAAGGGATTTTGGTCAT
GAAGCGCTTTTGAAGCTCGGATCCGAACAAACGACCCAACACCCGTGC
GTTTTATTCTGTCTTTTTATTGCCGATCCCCTCAGAAGAACTCGTCAAGA
AGGCGATAGAAGGCGATGCGCTGCGAATCGGGAGCGGCGATACCGTA
AAGCACGAGGAAGCGGTCAGCCCATTCGCCGCCAAGCTCTTCAGCAAT
ATCACGGGTAGCCAACGCTATGTCCTGATAGCGGTCCGCCACACCCAG
CCGGCCACAGTCGATGAATCCAGAAAAGCGGCCATTTTCCACCATGAT
ATTCGGCAAGCAGGCATCGCCATGGGTCACGACGAGATCCTCGCCGTC
GGGCATGCTCGCCTTGAGCCTGGCGAACAGTTCGGCTGGCGCGAGCCC
CTGATGCTCTTCGTCCAGATCATCCTGATCGACAAGACCGGCTTCCATC
CGAGTACGTGCTCGCTCGATGCGATGTTTCGCTTGGTGGTCGAATGGGC
AGGTAGCCGGATCAAGCGTATGCAGCCGCCGCATTGCATCAGCCATGA
TGGATACTTTCTCGGCAGGAGCAAGGTGAGATGACAGGAGATCCTGCC
CCGGCACTTCGCCCAATAGCAGCCAGTCCCTTCCCGCTTCAGTGACAAC
GTCGAGCACAGCTGCGCAAGGAACGCCCGTCGTGGCCAGCCACGATAG
CCGCGCTGCCTCGTCTTGCAGTTCATTCAGGGCACCGGACAGGTCGGTC
TTGACAAAAAGAACCGGGCGCCCCTGCGCTGACAGCCGGAACACGGCG
GCATCAGAGCAGCCGATTGTCTGTTGTGCCCAGTCATAGCCGAATAGCC
TCTCCACCCAAGCGGCCGGAGAACCTGCGTGCAATCCATCTTGTTCAAT
CATGCGAAACGATCCTCATCCTGTCTCTTGATCAGAGCTTGATCCCCTG
CGCCATCAGATCCTTGGCGGCAAGAAAGCCATCCAGTTTACTTTGCAGG
GCTTCCCAACCTTACCAGAGGCCTGCGCCGCGGCCAGCTGGCTAGCAA
TTCCCGGGTTAACTCTAGAGACATTGATTATTGACTAGTTATTAATAGT
AATCAATTACGGGGTCATTAGTTCATAGCCCATATATGGAGTTCCGCGT
TACATAACTTACGGTAAATGGCCCGCCTGGCTGACCGCCCAACGACCC
CCGCCCATTGACGTCAATAATGACGTATGTTCCCATAGTAACGCCAATA
GGGACTTTCCATTGACGTCAATGGGTGGAGTATTTACGGTAAACTGCCC
ACTTGGCAGTACATCAAGTGTATCATATGCCAAGTACGCCCCCTATTGA
CGTCAATGACGGTAAATGGCCCGCCTGGCATTATGCCCAGTACATGAC
CTTATGGGACTTTCCTACTTGGCAGTACATCTACGTATTAGTCATCGCT
ATTACCATGGTGATGCGGTTTTGGCAGTACATCAATGGGCGTGGATAGC
GGTTTGACTCACGGGGATTTCCAAGTCTCCACCCCATTGACGTCAATGG
GAGTTTGTTTTGGCACCAAAATCAACGGGACTTTCCAAAATGTCGTAAC
AACTCCGCCCCATTGACGCAAATGGGCGGTAGGCGTGTACGGTGGGAG
GTCTATATAAGCAGAGCTCGTTTAGTGAACCGGGGTCTCTCTGGTTAGA
CCAGATCTGAGCCTGGGAGCTCTCTGGCTAACTAGGGAACCCACTGCTT
AAGCCTCAATAAAGCTTGCCTTGAGTGCTTCAAGTAGTGTGTGCCCGTC
TGTTGTGTGACTCTGGTAACTAGAGATCCCTCAGACCCTTTTAGTCAGT
 
GTGGAAAATCTCTAGCAGTGGCGCCCGAACAGGGACTTGAAAGCGAAA
GGGAAACCAGAGGAGCTCTCTCGACGCAGGACTCGGCTTGCTGAAGCG
CGCACGGCAAGAGGCGAGGGGCGGCGACTGGTGAGTACGCCAAAAAT
TTTGACTAGCGGAGGCTAGAAGGAGAGAGATGGGTGCGAGAGCGTCA
GTATTAAGCGGGGGAGAATTAGATCGCGATGGGAAAAAATTCGGTTAA
GGCCAGGGGGAAAGAAAAAATATAAATTAAAACATATAGTATGGGCA
AGCAGGGAGCTAGAACGATTCGCAGTTAATCCTGGCCTGTTAGAAACA
TCAGAAGGCTGTAGACAAATACTGGGACAGCTACAACCATCCCTTCAG
ACAGGATCAGAAGAACTTAGATCATTATATAATACAGTAGCAACCCTC
TATTGTGTGCATCAAAGGATAGAGATAAAAGACACCAAGGAAGCTTTA
GACAAGATAGAGGAAGAGCAAAACAAAAGTAAGACCACCGCACAGCA
AGCGGCCGCTGATCTTCAGACCTGGAGGAGGAGATATGAGGGACAATT
GGAGAAGTGAATTATATAAATATAAAGTAGTAAAAATTGAACCATTAG
GAGTAGCACCCACCAAGGCAAAGAGAAGAGTGGTGCAGAGAGAAAAA
AGAGCAGTGGGAATAGGAGCTTTGTTCCTTGGGTTCTTGGGAGCAGCA
GGAAGCACTATGGGCGCAGCGTCAATGACGCTGACGGTACAGGCCAGA
CAATTATTGTCTGGTATAGTGCAGCAGCAGAACAATTTGCTGAGGGCTA
TTGAGGCGCAACAGCATCTGTTGCAACTCACAGTCTGGGGCATCAAGC
AGCTCCAGGCAAGAATCCTGGCTGTGGAAAGATACCTAAAGGATCAAC
AGCTCCTGGGGATTTGGGGTTGCTCTGGAAAACTCATTTGCACCACTGC
TGTGCCTTGGAATGCTAGTTGGAGTAATAAATCTCTGGAACAGATTTGG
AATCACACGACCTGGATGGAGTGGGACAGAGAAATTAACAATTACACA
AGCTTAATACACTCCTTAATTGAAGAATCGCAAAACCAGCAAGAAAAG
AATGAACAAGAATTATTGGAATTAGATAAATGGGCAAGTTTGTGGAAT
TGGTTTAACATAACAAATTGGCTGTGGTATATAAAATTATTCATAATGA
TAGTAGGAGGCTTGGTAGGTTTAAGAATAGTTTTTGCTGTACTTTCTAT
AGTGAATAGAGTTAGGCAGGGATATTCACCATTATCGTTTCAGACCCAC
CTCCCAACCCCGAGGGGACCCGACAGGCCCGAAGGAATAGAAGAAGA
AGGTGGAGAGAGAGACAGAGACAGATCCATTCGATTAGTGAACGGATC
TCGACGGTATCGGTTAACTTTTAAAAGAAAAGGGGGGATTGGGGGGTA
CAGTGCAGGGGAAAGAATAGTAGACATAATAGCAACAGACATACAAA
CTAAAGAATTACAAAAACAAATTACAAAAATTCAAAATTTTATCGATC
ACGAGACTAGCCTCGAGGCATGCCTGCAGGAATTCCGTTACATAACTT
ACGGTAAATGGCCCGCCTGGCTGACCGCCCAACGACCCCCGCCCATTG
ACGTCAATAATGACGTATGTTCCCATAGTAACGCCAATAGGGACTTTCC
ATTGACGTCAATGGGTGGAGTATTTACGGTAAACTGCCCACTTGGCAGT
ACATCAAGTGTATCATATGCCAAGTACGCCCCCTATTGACGTCAATGAC
GGTAAATGGCCCGCCTGGCATTATGCCCAGTACATGACCTTATGGGACT
TTCCTACTTGGCAGTACATCTACGTATTAGTCATCGCTATTACCATGGTC
GAGGTGAGCCCCACGTTCTGCTTCACTCTCCCCATCTCCCCCCCCTCCCC
 
ACCCCCAATTTTGTATTTATTTATTTTTTAATTATTTTGTGCAGCGATGG
GGGCGGGGGGGGGGGGGGGCGCGCCAGGCGGGGCGGGGCGGGGC
GAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGG
GCGGCCCTATAAAAAGCGAAGCGCGCGGCGGGCGGGAGTCGCTGCGA
CGCTGCCTTCGCCCCGTGCCCCGCTCCGCCGCCGCCTCGCGCCGCCCGC
CCCGGCTCTGACTGACCGCGTTACTCCCACAGGTGAGCGGGCGGGACG
GCCCTTCTCCTCCGGGCTGTAATTAGCTGAGCAAGAGGTAAGGGTTTAA
GGGATGGTTGGTTGGTGGGGTATTAATGTTTAATTACCTGGAGCACCTG
CCTGAAATCACTTTTTTTCAGGTACCGGTCGCCACCATGGCGAGACCCC
TGTGCACATTACTTCTGTTGATGGCTACCCTGGCAGGCGCCCTCGCCAG
CGAGCTGACACAGGACCCTGCCGTGTCCGTGGCCCTGAAGCAGACCGT
GACAATCACCTGCAGAGGCGATTCCCTGAGATCCCACTACGCCTCCTGG
TACCAGAAGAAGCCTGGCCAGGCCCCCGTGCTGCTGTTTTACGGCAAG
AATAACCGCCCCAGCGGCATCCCCGATAGATTTTCCGGCAGCGCCTCCG
GCAACAGAGCCAGCCTGACAATCACCGGCGCCCAGGCCGAGGACGAG
GCTGATTACTACTGCAGCTCCAGAGATAAGAGCGGCAGCAGACTGTCC
GTGTTTGGCGGCGGCACCAAGCTGACCGTGCTCGGAGGAGGAGGAAGC
GGAGGAGGAGGCTCAGGCGGCGGCGGCTCTGAGGTGAGGCTGAGAGA
GTCCGGCGGCGGCCTGGTGAAGCCCGGAGGATCTCTGAGGCTGTCCTG
CTCCGCCTCCGGCTTCGATTTTGACAATGCCTGGATGACCTGGGTGAGA
CAGCCCCCTGGCAAGGGCCTGGAGTGGGTGGGAAGGATCACAGGCCCC
GGCGAGGGCTGGTCCGTGGATTACGCCGAGTCCGTGAAGGGCAGGTTC
ACAATCTCCAGGGATAACACCAAGAACACCCTGTACCTGGAGATGAAC
AACGTGAGGACAGAGGATACCGGCTACTACTTTTGCGCCAGAACAGGC
AAGTACTACGACTTTTGGTTCGGCTACCCCCCTGGCGAGGAGTACTTCC
AGGATTGGGGCCAGGGCACCCTGGTCATTGTGTCCAGCGGCGGCGGCG
GCAGTGGCGGCGGCGGAAGCGGCGGCGGCGGCTCTGGCGGCGGCGGC
AGCGGCGGCGGCGGCTCCGACATCGTGATGACCCAGTCTCCTGATAGC
CTGGCCGTGAGCCTGGGCGAGAGAGTGACAATGAACTGTAAGTCTAGC
CAGAGCCTGCTGTACTCCACCAACCAGAAGAATTACCTGGCCTGGTATC
AGCAGAAGCCTGGCCAGTCCCCAAAGCTGCTGATCTATTGGGCATCTA
CAAGGGAGAGCGGAGTGCCAGACAGATTCAGCGGATCCGGATCTGGA
ACCGACTTCACCCTGACAATCTCCTCTGTGCAGGCCGAGGACGTGGCCG
TGTACTATTGCCAGCAGTACTATAGCTACAGGACATTCGGCGGCGGCA
CCAAGCTGGAGATCAAGCGCACCGTGGCCGGAGGAGGAGGATCTGGC
GGAGGAGGGTCCGGCGGCGGCGGCTCCCAGGTGCAGCTGCAGCAGAG
CGGACCAGAGGTGGTGAAGCCTGGAGCCTCCGTGAAGATGTCTTGTAA
GGCCAGCGGCTACACCTTCACATCCTATGTGATCCACTGGGTGAGGCA
GAAGCCAGGACAGGGACTGGACTGGATCGGCTACATCAACCCTTATAA
 
TGATGGCACCGACTACGATGAGAAGTTTAAGGGCAAGGCCACCCTGAC
ATCCGATACCAGCACATCCACCGCCTATATGGAGCTGAGCTCCCTGCGG
TCTGAGGACACAGCCGTGTACTATTGCGCCAGAGAGAAGGATAACTAC
GCAACCGGAGCATGGTTCGCATATTGGGGACAGGGTACCCTGGTCACC
GTGTCTAGCGAGCCCAAGAGCTGTGACAAGACACACACCTGCCCTCCA
TGTCCAGCACCAGAGCTGCTGGGAGGGCCCTCCGTGTTCCTGTTTCCCC
CTAAGCCAAAGGATACACTGATGATCTCCAGGACACCAGAGGTGACCT
GCGTGGTGGTGGACGTGTCTCACGAGGATCCTGAGGTGAAGTTTAACT
GGTACGTGGACGGCGTGGAGGTGCACAATGCCAAGACCAAGCCTAGGG
AGGAGCAGTACAATTCTACATATCGCGTGGTGAGCGTGCTGACCGTGC
TGCACCAGGATTGGCTGAACGGCAAGGAGTATAAGTGCAAGGTGAGCA
ATAAGGCCCTGCCTGCCCCAATCGAGAAGACAATCTCCAAGGCAAAGG
GACAGCCAAGGGAGCCTCAGGTGTACACCCTGCCACCCTCTCGCGACG
AGCTGACAAAGAACCAGGTGAGCCTGACCTGTCTGGTGAAGGGCTTCT
ATCCTAGCGATATTGCCGTGGAGTGGGAGTCCAATGGCCAGCCAGAGA
ACAATTACAAGACCACACCTCCAGTGCTGGACTCCGATGGCTCTTTCTT
TCTGTATTCCAAGCTGACAGTGGACAAGTCTAGATGGCAGCAGGGCAA
CGTGTTTTCTTGTAGCGTGATGCACGAGGCCCTGCACAATCACTACACC
CAGAAGTCCCTGTCTCTGAGCCCCGGCAAGTAGGATATCCAGCACAGT
CCCGGGCCGAGTCTAGACGTTTAAACCCGCTGATCAGGTCGACAATCA
ACCTCTGGATTACAAAATTTGTGAAAGATTGACTGGTATTCTTAACTAT
GTTGCTCCTTTTACGCTATGTGGATACGCTGCTTTAATGCCTTTGTATCA
TGCTATTGCTTCCCGTATGGCTTTCATTTTCTCCTCCTTGTATAAATCCT
GGTTGCTGTCTCTTTATGAGGAGTTGTGGCCCGTTGTCAGGCAACGTGG
CGTGGTGTGCACTGTGTTTGCTGACGCAACCCCCACTGGTTGGGGCATT
GCCACCACCTGTCAGCTCCTTTCCGGGACTTTCGCTTTCCCCCTCCCTAT
TGCCACGGCGGAACTCATCGCCGCCTGCCTTGCCCGCTGCTGGACAGG
GGCTCGGCTGTTGGGCACTGACAATTCCGTGGTGTTGTCGGGGAAGCTG
ACGTCCTTTCCATGGCTGCTCGCCTGTGTTGCCACCTGGATTCTGCGCG
GGACGTCCTTCTGCTACGTCCCTTCGGCCCTCAATCCAGCGGACCTTCC
TTCCCGCGGCCTGCTGCCGGCTCTGCGGCCTCTTCCGCGTCTTCGCCTTC
GCCCTCAGACGAGTCGGATCTCCCTTTGGGCCGCCTCCCCGCGGTACCT
TTAAGACCAATGACTTACAAGGCAGCTGTAGATCTTAGCCACTTTTTAA
AAGAAAAGGGGGGACTGGAAGGGCTAATTCACTCCCAACGAAGACAA
GATCTGCTTTTTGCTTGTACTGGGTCTCTCTGGTTAGACCAGATCTGAGC
CTGGGAGCTCTCTGGCTAACTAGGGAACCCACTGCTTAAGCCTCAATAA
AGCTTGCCTTGAGTGCTTCAAGTAGTGTGTGCCCGTCTGTTGTGTGACT
CTGGTAACTAGAGATCCCTCAGACCCTTTTAGTCAGTGTGGAAAATCTC
TAGCAGTAGTAGTTCATGTCATCTTATTATTCAGTATTTATAACTTGCAA
AGAAATGAATATCAGAGAGTGAGAGGAACTTGTTTATTGCAGCTTATA
 
ATGGTTACAAATAAAGCAATAGCATCACAAATTTCACAAATAAAGCAT
TTTTTTCACTGCATTCTAGTTGTGGTTTGTCCAAACTCATCAATGTATCT
TATCATGTCTGGCTCTAGCTATCCCGCCCCTAACTCCGCCCATCCCGCC
CCTAACTCCGCCCAGTTCCGCCCATTCTCCGCCCCATGGCTGACTAATT
TTTTTTATTTATGCAGAGGCCGAGGCCGCCTCGGCCTCTGAGCTATTCC
AGAAGTAGTGAGGAGGCTTTTTTGGAGGCCGCTAGCGTCGACCATTAC
TTATTGTTTTAGCTGTCCTCATGAATGTCTTTTCACTACCCATTTGCTTA
TCCTGCATCTCTCAGCCTTGACTCCACTCAGTTCTCTTGCTTAGAGATAC
CACCTTTCCCCTGAAGTGTTCCTTCCATGTTTTACGGCGAGATGGTTTCT
CCTCGCCTGGCCACTCAGCCTTAGTTGTCTCTGTTGTCTTATAGAGGTCT
ACTTGAAGAAGGAAAAACAGGGGGCATGGTTTGACTGTCCTGTGAGCC
CTTCTTCCCTGCCTCCCCCACTCACAGTGACCCGGAATCCCTCGACATG
GCAGTCTAGCACTAGTGCGGCCGCAGATCTGCTTCCTCGCTCACTGACT
CGCTGCGCTCGGTCGTTCGGCTGCGGCGAGCGGTATCAGCTCACTCAAA
GGCGGTAATACGGTTATCCACAGAATCAGGGGATAACGCAGGAAAGA
ACATGTGAGCAAAAGGCCAGCAAAAGGCCAGGAACCGTAAAAA
   SEQ ID NO: 19 
GGCCGCGTTGCTGGCGTTTTTCCATAGGCTCCGCCCCCCTGACGAGCAT
CACAAAAATCGACGCTCAAGTCAGAGGTGGCGAAACCCGACAGGACTA
TAAAGATACCAGGCGTTTCCCCCTGGAAGCTCCCTCGTGCGCTCTCCTG
TTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTA
AGACACGACTTATCGCCACTGGCAGCAGCCACTGGTAACAGGATTAGC
AGAGCGAGGTATGTAGGCGGTGCTACAGAGTTCTTGAAGTGGTGGCCT
AACTACGGCTACACTAGAAGAACAGTATTTGGTATCTGCGCTCTGCTGA
AGCCAGTTACCTTCGGAAAAAGAGTTGGTAGCTCTTGATCCGGCAAAC
AAACCACCGCTGGTAGCGGTGGTTTTTTTGTTTGCAAGCAGCAGATTAC
GCGCAGAAAAAAAGGATCTCAAGAAGATCCTTTGATCTTTTCTACGGG
GTCTGACGCTCAGTGGAACGAAAACTCACGTTAAGGGATTTTGGTCAT
GAAGCGCTTTTGAAGCTCGGATCCGAACAAACGACCCAACACCCGTGC
GTTTTATTCTGTCTTTTTATTGCCGATCCCCTCAGAAGAACTCGTCAAGA
AGGCGATAGAAGGCGATGCGCTGCGAATCGGGAGCGGCGATACCGTA
AAGCACGAGGAAGCGGTCAGCCCATTCGCCGCCAAGCTCTTCAGCAAT
ATCACGGGTAGCCAACGCTATGTCCTGATAGCGGTCCGCCACACCCAG
CCGGCCACAGTCGATGAATCCAGAAAAGCGGCCATTTTCCACCATGAT
ATTCGGCAAGCAGGCATCGCCATGGGTCACGACGAGATCCTCGCCGTC
GGGCATGCTCGCCTTGAGCCTGGCGAACAGTTCGGCTGGCGCGAGCCC
CTGATGCTCTTCGTCCAGATCATCCTGATCGACAAGACCGGCTTCCATC
 
CGAGTACGTGCTCGCTCGATGCGATGTTTCGCTTGGTGGTCGAATGGGC
AGGTAGCCGGATCAAGCGTATGCAGCCGCCGCATTGCATCAGCCATGA
TGGATACTTTCTCGGCAGGAGCAAGGTGAGATGACAGGAGATCCTGCC
CCGGCACTTCGCCCAATAGCAGCCAGTCCCTTCCCGCTTCAGTGACAAC
GTCGAGCACAGCTGCGCAAGGAACGCCCGTCGTGGCCAGCCACGATAG
CCGCGCTGCCTCGTCTTGCAGTTCATTCAGGGCACCGGACAGGTCGGTC
TTGACAAAAAGAACCGGGCGCCCCTGCGCTGACAGCCGGAACACGGCG
GCATCAGAGCAGCCGATTGTCTGTTGTGCCCAGTCATAGCCGAATAGCC
TCTCCACCCAAGCGGCCGGAGAACCTGCGTGCAATCCATCTTGTTCAAT
CATGCGAAACGATCCTCATCCTGTCTCTTGATCAGAGCTTGATCCCCTG
CGCCATCAGATCCTTGGCGGCAAGAAAGCCATCCAGTTTACTTTGCAGG
GCTTCCCAACCTTACCAGAGGCCTGCGCCGCGGCCAGCTGGCTAGCAA
TTCCCGGGTTAACTCTAGAGACATTGATTATTGACTAGTTATTAATAGT
AATCAATTACGGGGTCATTAGTTCATAGCCCATATATGGAGTTCCGCGT
TACATAACTTACGGTAAATGGCCCGCCTGGCTGACCGCCCAACGACCC
CCGCCCATTGACGTCAATAATGACGTATGTTCCCATAGTAACGCCAATA
GGGACTTTCCATTGACGTCAATGGGTGGAGTATTTACGGTAAACTGCCC
ACTTGGCAGTACATCAAGTGTATCATATGCCAAGTACGCCCCCTATTGA
CGTCAATGACGGTAAATGGCCCGCCTGGCATTATGCCCAGTACATGAC
CTTATGGGACTTTCCTACTTGGCAGTACATCTACGTATTAGTCATCGCT
ATTACCATGGTGATGCGGTTTTGGCAGTACATCAATGGGCGTGGATAGC
GGTTTGACTCACGGGGATTTCCAAGTCTCCACCCCATTGACGTCAATGG
GAGTTTGTTTTGGCACCAAAATCAACGGGACTTTCCAAAATGTCGTAAC
AACTCCGCCCCATTGACGCAAATGGGCGGTAGGCGTGTACGGTGGGAG
GTCTATATAAGCAGAGCTCGTTTAGTGAACCGGGGTCTCTCTGGTTAGA
CCAGATCTGAGCCTGGGAGCTCTCTGGCTAACTAGGGAACCCACTGCTT
AAGCCTCAATAAAGCTTGCCTTGAGTGCTTCAAGTAGTGTGTGCCCGTC
TGTTGTGTGACTCTGGTAACTAGAGATCCCTCAGACCCTTTTAGTCAGT
GTGGAAAATCTCTAGCAGTGGCGCCCGAACAGGGACTTGAAAGCGAAA
GGGAAACCAGAGGAGCTCTCTCGACGCAGGACTCGGCTTGCTGAAGCG
CGCACGGCAAGAGGCGAGGGGCGGCGACTGGTGAGTACGCCAAAAAT
TTTGACTAGCGGAGGCTAGAAGGAGAGAGATGGGTGCGAGAGCGTCA
GTATTAAGCGGGGGAGAATTAGATCGCGATGGGAAAAAATTCGGTTAA
GGCCAGGGGGAAAGAAAAAATATAAATTAAAACATATAGTATGGGCA
AGCAGGGAGCTAGAACGATTCGCAGTTAATCCTGGCCTGTTAGAAACA
TCAGAAGGCTGTAGACAAATACTGGGACAGCTACAACCATCCCTTCAG
ACAGGATCAGAAGAACTTAGATCATTATATAATACAGTAGCAACCCTC
TATTGTGTGCATCAAAGGATAGAGATAAAAGACACCAAGGAAGCTTTA
GACAAGATAGAGGAAGAGCAAAACAAAAGTAAGACCACCGCACAGCA
AGCGGCCGCTGATCTTCAGACCTGGAGGAGGAGATATGAGGGACAATT
 
GGAGAAGTGAATTATATAAATATAAAGTAGTAAAAATTGAACCATTAG
GAGTAGCACCCACCAAGGCAAAGAGAAGAGTGGTGCAGAGAGAAAAA
AGAGCAGTGGGAATAGGAGCTTTGTTCCTTGGGTTCTTGGGAGCAGCA
GGAAGCACTATGGGCGCAGCGTCAATGACGCTGACGGTACAGGCCAGA
CAATTATTGTCTGGTATAGTGCAGCAGCAGAACAATTTGCTGAGGGCTA
TTGAGGCGCAACAGCATCTGTTGCAACTCACAGTCTGGGGCATCAAGC
AGCTCCAGGCAAGAATCCTGGCTGTGGAAAGATACCTAAAGGATCAAC
AGCTCCTGGGGATTTGGGGTTGCTCTGGAAAACTCATTTGCACCACTGC
TGTGCCTTGGAATGCTAGTTGGAGTAATAAATCTCTGGAACAGATTTGG
AATCACACGACCTGGATGGAGTGGGACAGAGAAATTAACAATTACACA
AGCTTAATACACTCCTTAATTGAAGAATCGCAAAACCAGCAAGAAAAG
AATGAACAAGAATTATTGGAATTAGATAAATGGGCAAGTTTGTGGAAT
TGGTTTAACATAACAAATTGGCTGTGGTATATAAAATTATTCATAATGA
TAGTAGGAGGCTTGGTAGGTTTAAGAATAGTTTTTGCTGTACTTTCTAT
AGTGAATAGAGTTAGGCAGGGATATTCACCATTATCGTTTCAGACCCAC
CTCCCAACCCCGAGGGGACCCGACAGGCCCGAAGGAATAGAAGAAGA
AGGTGGAGAGAGAGACAGAGACAGATCCATTCGATTAGTGAACGGATC
TCGACGGTATCGGTTAACTTTTAAAAGAAAAGGGGGGATTGGGGGGTA
CAGTGCAGGGGAAAGAATAGTAGACATAATAGCAACAGACATACAAA
CTAAAGAATTACAAAAACAAATTACAAAAATTCAAAATTTTATCGATC
ACGAGACTAGCCTCGAGGCATGCCTGCAGGAATTCCGTTACATAACTT
ACGGTAAATGGCCCGCCTGGCTGACCGCCCAACGACCCCCGCCCATTG
ACGTCAATAATGACGTATGTTCCCATAGTAACGCCAATAGGGACTTTCC
ATTGACGTCAATGGGTGGAGTATTTACGGTAAACTGCCCACTTGGCAGT
ACATCAAGTGTATCATATGCCAAGTACGCCCCCTATTGACGTCAATGAC
GGTAAATGGCCCGCCTGGCATTATGCCCAGTACATGACCTTATGGGACT
TTCCTACTTGGCAGTACATCTACGTATTAGTCATCGCTATTACCATGGTC
GAGGTGAGCCCCACGTTCTGCTTCACTCTCCCCATCTCCCCCCCCTCCCC
ACCCCCAATTTTGTATTTATTTATTTTTTAATTATTTTGTGCAGCGATGG
GGGCGGGGGGGGGGGGGGGGCGCGCGCCAGGCGGGGCGGGGCGGGGC
GAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCG
GCGGCCCTATAAAAAGCGAAGCGCGCGGCGGGCGGGAGTCGCTGCGA
CGCTGCCTTCGCCCCGTGCCCCGCTCCGCCGCCGCCTCGCGCCGCCCGC
CCCGGCTCTGACTGACCGCGTTACTCCCACAGGTGAGCGGGCGGGACG
GCCCTTCTCCTCCGGGCTGTAATTAGCTGAGCAAGAGGTAAGGGTTTAA
GGGATGGTTGGTTGGTGGGGTATTAATGTTTAATTACCTGGAGCACCTG
CCTGAAATCACTTTTTTTCAGGTACCGGTCGCCACCATGGCGAGACCCC
TGTGCACATTACTTCTGTTGATGGCTACCCTGGCAGGCGCCCTCGCCAG
CGAGCTGACACAGGACCCTGCCGTGTCCGTGGCCCTGAAGCAGACCGT
 
GACAATCACCTGCAGAGGCGATTCCCTGAGATCCCACTACGCCTCCTGG
TACCAGAAGAAGCCTGGCCAGGCCCCCGTGCTGCTGTTTTACGGCAAG
AATAACCGCCCCAGCGGCATCCCCGATAGATTTTCCGGCAGCGCCTCCG
GCAACAGAGCCAGCCTGACAATCACCGGCGCCCAGGCCGAGGACGAG
GCTGATTACTACTGCAGCTCCAGAGATAAGAGCGGCAGCAGACTGTCC
GTGTTTGGCGGCGGCACCAAGCTGACCGTGCTCGGAGGAGGAGGAAGC
GGAGGAGGAGGCTCAGGCGGCGGCGGCTCTGAGGTGAGGCTGAGAGA
GTCCGGCGGCGGCCTGGTGAAGCCCGGAGGATCTCTGAGGCTGTCCTG
CTCCGCCTCCGGCTTCGATTTTGACAATGCCTGGATGACCTGGGTGAGA
CAGCCCCCTGGCAAGGGCCTGGAGTGGGTGGGAAGGATCACAGGCCCC
GGCGAGGGCTGGTCCGTGGATTACGCCGAGTCCGTGAAGGGCAGGTTC
ACAATCTCCAGGGATAACACCAAGAACACCCTGTACCTGGAGATGAAC
AACGTGAGGACAGAGGATACCGGCTACTACTTTTGCGCCAGAACAGGC
AAGTACTACGACTTTTGGTTCGGCTACCCCCCTGGCGAGGAGTACTTCC
AGGATTGGGGCCAGGGCACCCTGGTCATTGTGTCCAGCGGCGGCGGCG
GCAGTGGCGGCGGCGGAAGCGGCGGCGGCGGCTCTGGCGGCGGCGGC
AGCGGCGGCGGCGGCTCCGACATCGTGATGACCCAGTCTCCTGATAGC
CTGGCCGTGAGCCTGGGCGAGAGAGTGACAATGAACTGTAAGTCTAGC
CAGAGCCTGCTGTACTCCACCAACCAGAAGAATTACCTGGCCTGGTATC
AGCAGAAGCCTGGCCAGTCCCCAAAGCTGCTGATCTATTGGGCATCTA
CAAGGGAGAGCGGAGTGCCAGACAGATTCAGCGGATCCGGATCTGGA
ACCGACTTCACCCTGACAATCTCCTCTGTGCAGGCCGAGGACGTGGCCG
TGTACTATTGCCAGCAGTACTATAGCTACAGGACATTCGGCGGCGGCA
CCAAGCTGGAGATCAAGCGCACCGTGGCCGGAGGAGGAGGATCTGGC
GGAGGAGGGTCCGGCGGCGGCGGCTCCCAGGTGCAGCTGCAGCAGAG
CGGACCAGAGGTGGTGAAGCCTGGAGCCTCCGTGAAGATGTCTTGTAA
GGCCAGCGGCTACACCTTCACATCCTATGTGATCCACTGGGTGAGGCA
GAAGCCAGGACAGGGACTGGACTGGATCGGCTACATCAACCCTTATAA
TGATGGCACCGACTACGATGAGAAGTTTAAGGGCAAGGCCACCCTGAC
ATCCGATACCAGCACATCCACCGCCTATATGGAGCTGAGCTCCCTGCGG
TCTGAGGACACAGCCGTGTACTATTGCGCCAGAGAGAAGGATAACTAC
GCAACCGGAGCATGGTTCGCATATTGGGGACAGGGTACCCTGGTCACC
GTGTCTAGCGAGCCCAAGAGCTGTGACAAGACACACACCTGCCCTCCA
TGTCCAGCACCAGAGCTGCTGGGAGGGCCCTCCGTGTTCCTGTTTCCCC
CTAAGCCAAAGGATACACTGATGATCTCCAGGACACCAGAGGTGACCT
GCGTGGTGGTGGACGTGTCTCACGAGGATCCTGAGGTGAAGTTTAACT
GGTACGTGGACGGCGTGGAGGTGCACAATGCCAAGACCAAGCCTAGGG
AGGAGCAGTACAATTCTACATATCGCGTGGTGAGCGTGCTGACCGTGC
TGCACCAGGATTGGCTGAACGGCAAGGAGTATAAGTGCAAGGTGAGCA
ATAAGGCCCTGCCTGCCCCAATCGAGAAGACAATCTCCAAGGCAAAGG
 
GACAGCCAAGGGAGCCTCAGGTGTACACCCTGCCACCCTCTCGCGACG
AGCTGACAAAGAACCAGGTGAGCCTGACCTGTCTGGTGAAGGGCTTCT
ATCCTAGCGATATTGCCGTGGAGTGGGAGTCCAATGGCCAGCCAGAGA
ACAATTACAAGACCACACCTCCAGTGCTGGACTCCGATGGCTCTTTCTT
TCTGTATTCCAAGCTGACAGTGGACAAGTCTAGATGGCAGCAGGGCAA
CGTGTTTTCTTGTAGCGTGATGCACGAGGCCCTGCACAATCACTACACC
CAGAAGTCCCTGTCTCTGAGCCCCGGCAAGGGCGGAGGAGGAAGCGGA
GGAGGCGGCTCTGGCGGAGGAGGTTCCGGAGGAGGCGGAAGCGGCGG
AGGAGGCTCTTGGGAGCAGAAGATCGAGGAGCTGCTGAAGAAGGCCG
AGGAGCAGCAGAAGAAGAATGAGGAGGAGCTGAAGAAGCTGGAGAAG
TAGGTCGACAATCAACCTCTGGATTACAAAATTTGTGAAAGATTGACTG
GTATTCTTAACTATGTTGCTCCTTTTACGCTATGTGGATACGCTGCTTTA
ATGCCTTTGTATCATGCTATTGCTTCCCGTATGGCTTTCATTTTCTCCTC
CTTGTATAAATCCTGGTTGCTGTCTCTTTATGAGGAGTTGTGGCCCGTTG
TCAGGCAACGTGGCGTGGTGTGCACTGTGTTTGCTGACGCAACCCCCAC
TGGTTGGGGCATTGCCACCACCTGTCAGCTCCTTTCCGGGACTTTCGCT
TTCCCCCTCCCTATTGCCACGGCGGAACTCATCGCCGCCTGCCTTGCCC
GCTGCTGGACAGGGGCTCGGCTGTTGGGCACTGACAATTCCGTGGTGTT
GTCGGGGAAGCTGACGTCCTTTCCATGGCTGCTCGCCTGTGTTGCCACC
TGGATTCTGCGCGGGACGTCCTTCTGCTACGTCCCTTCGGCCCTCAATC
CAGCGGACCTTCCTTCCCGCGGCCTGCTGCCGGCTCTGCGGCCTCTTCC
GCGTCTTCGCCTTCGCCCTCAGACGAGTCGGATCTCCCTTTGGGCCGCC
TCCCCGCGGTACCTTTAAGACCAATGACTTACAAGGCAGCTGTAGATCT
TAGCCACTTTTTAAAAGAAAAGGGGGGACTGGAAGGGCTAATTCACTC
CCAACGAAGACAAGATCTGCTTTTTGCTTGTACTGGGTCTCTCTGGTTA
GACCAGATCTGAGCCTGGGAGCTCTCTGGCTAACTAGGGAACCCACTG
CTTAAGCCTCAATAAAGCTTGCCTTGAGTGCTTCAAGTAGTGTGTGCCC
GTCTGTTGTGTGACTCTGGTAACTAGAGATCCCTCAGACCCTTTTAGTC
AGTGTGGAAAATCTCTAGCAGTAGTAGTTCATGTCATCTTATTATTCAG
TATTTATAACTTGCAAAGAAATGAATATCAGAGAGTGAGAGGAACTTG
TTTATTGCAGCTTATAATGGTTACAAATAAAGCAATAGCATCACAAATT
TCACAAATAAAGCATTTTTTTCACTGCATTCTAGTTGTGGTTTGTCCAAA
CTCATCAATGTATCTTATCATGTCTGGCTCTAGCTATCCCGCCCCTAACT
CCGCCCATCCCGCCCCTAACTCCGCCCAGTTCCGCCCATTCTCCGCCCC
ATGGCTGACTAATTTTTTTTATTTATGCAGAGGCCGAGGCCGCCTCGGC
CTCTGAGCTATTCCAGAAGTAGTGAGGAGGCTTTTTTGGAGGCCGCTAG
CGTCGACCATTACTTATTGTTTTAGCTGTCCTCATGAATGTCTTTTCACT
ACCCATTTGCTTATCCTGCATCTCTCAGCCTTGACTCCACTCAGTTCTCT
TGCTTAGAGATACCACCTTTCCCCTGAAGTGTTCCTTCCATGTTTTACGG
CGAGATGGTTTCTCCTCGCCTGGCCACTCAGCCTTAGTTGTCTCTGTTGT
 
CTTATAGAGGTCTACTTGAAGAAGGAAAAACAGGGGGCATGGTTTGAC
TGTCCTGTGAGCCCTTCTTCCCTGCCTCCCCCACTCACAGTGACCCGGA
ATCCCTCGACATGGCAGTCTAGCACTAGTGCGGCCGCAGATCTGCTTCC
TCGCTCACTGACTCGCTGCGCTCGGTCGTTCGGCTGCGGCGAGCGGTAT
CAGCTCACTCAAAGGCGGTAATACGGTTATCCACAGAATCAGGGGATA
ACGCAGGAAAGAACATGTGAGCAAAAGGCCAGCAAAAGGCCAGGAAC
CGTAAAAA
   SEQ ID NO: 20 
ACATGTGAGCAAAAGGCCAGCAAAAGGCCAGGAACCGTAAAAAGGCC
GCGTTGCTGGCGTTTTTCCATAGGCTCCGCCCCCCTGACGAGCATCACA
AAAATCGACGCTCAAGTCAGAGGTGGCGAAACCCGACAGGACTATAAA
GATACCAGGCGTTTCCCCCTGGAAGCTCCCTCGTGCGCTCTCCTGTTCC
GACCCTGCCGCTTACCGGATACCTGTCCGCCTTTCTCCCTTCGGGAAGC
GTGGCGCTTTCTCATAGCTCACGCTGTAGGTATCTCAGTTCGGTGTAGG
TCGTTCGCTCCAAGCTGGGCTGTGTGCACGAACCCCCCGTTCAGCCCGA
CCGCTGCGCCTTATCCGGTAACTATCGTCTTGAGTCCAACCCGGTAAGA
CACGACTTATCGCCACTGGCAGCAGCCACTGGTAACAGGATTAGCAGA
GCGAGGTATGTAGGCGGTGCTACAGAGTTCTTGAAGTGGTGGCCTAAC
TACGGCTACACTAGAAGGACAGTATTTGGTATCTGCGCTCTGCTGAAGC
CAGTTACCTTCGGAAAAAGAGTTGGTAGCTCTTGATCCGGCAAACAAA
CCACCGCTGGTAGCGGTGGTTTTTTTGTTTGCAAGCAGCAGATTACGCG
CAGAAAAAAAGGATCTCAAGAAGATCCTTTGATCTTTTCTACGGGGTCT
GACGCTCAGTGGAACGAAAACTCACGTTAAGGGATTTTGGTCATGAGA
TTATCAAAAAGGATCTTCACCTAGATCCTTTTAAATTAAAAATGAAGTT
TTAAATCAATCTAAAGTATATATGAGTAAACTTGGTCTGACAGTTACCA
ATGCTTAATCAGTGAGGCACCTATCTCAGCGATCTGTCTATTTCGTTCA
TCCATAGTTGCCTGACTCCCCGTCGTGTAGATAACTACGATACGGGAGG
GCTTACCATCTGGCCCCAGTGCTGCAATGATACCGCGAGACCCACGCTC
ACCGGCTCCAGATTTATCAGCAATAAACCAGCCAGCCGGAAGGGCCGA
GCGCAGAAGTGGTCCTGCAACTTTATCCGCCTCCATCCAGTCTATTAAT
TGTTGCCGGGAAGCTAGAGTAAGTAGTTCGCCAGTTAATAGTTTGCGCA
ACGTTGTTGCCATTGCTACAGGCATCGTGGTGTCACGCTCGTCGTTTGG
TATGGCTTCATTCAGCTCCGGTTCCCAACGATCAAGGCGAGTTACATGA
TCCCCCATGTTGTGCAAAAAAGCGGTTAGCTCCTTCGGTCCTCCGATCG
TTGTCAGAAGTAAGTTGGCCGCAGTGTTATCACTCATGGTTATGGCAGC
ACTGCATAATTCTCTTACTGTCATGCCATCCGTAAGATGCTTTTCTGTGA
CTGGTGAGTACTCAACCAAGTCATTCTGAGAATAGTGTATGCGGCGAC
CGAGTTGCTCTTGCCCGGCGTCAATACGGGATAATACCGCGCCACATA
GCAGAACTTTAAAAGTGCTCATCATTGGAAAACGTTCTTCGGGGCGAA
AACTCTCAAGGATCTTACCGCTGTTGAGATCCAGTTCGATGTAACCCAC
 
TCGTGCACCCAACTGATCTTCAGCATCTTTTACTTTCACCAGCGTTTCTG
GGTGAGCAAAAACAGGAAGGCAAAATGCCGCAAAAAAGGGAATAAGG
GCGACACGGAAATGTTGAATACTCATACTCTTCCTTTTTCAATATTATT
GAAGCATTTATCAGGGTTATTGTCTCATGAGCGGATACATATTTGAATG
TATTTAGAAAAATAAACAAATAGGGGTTCCGCGCACATTTCCCCGAAA
AGTGCCACCTGACGTCTAAGAAACCATTATTATCATGACATTAACCTAT
AAAAATAGGCGTATCACGAGGCCCTTTCGTCTCGCGCGTTTCGGTGATG
ACGGTGAAAACCTCTGACACATGCAGCTCCCGGAGACGGTCACAGCTT
GTCTGTAAGCGGATGCCGGGAGCAGACAAGCCCGTCAGGGCGCGTCAG
CGGGTGTTGGCGGGTGTCGGGGCTGGCTTAACTATGCGGCATCAGAGC
AGATTGTACTGAGAGTGCACCATAAAATTGTAAACGTTAATATTTTGTT
AAAATTCGCGTTAAATTTTTGTTAAATCAGCTCATTTTTTAACCAATAG
GCCGAAATCGGCAAAATCCCTTATAAATCAAAAGAATAGCCCGAGATA
GGGTTGAGTGTTGTTCCAGTTTGGAACAAGAGTCCACTATTAAAGAAC
GTGGACTCCAACGTCAAAGGGCGAAAAACCGTCTATCAGGGCGATGGC
CCACTACGTGAACCATCACCCAAATCAAGTTTTTTGGGGTCGAGGTGCC
GTAAAGCACTAAATCGGAACCCTAAAGGGAGCCCCCGATTTAGAGCTT
GACGGGGAAAGCCGGCGAACGTGGCGAGAAAGGAAGGGAAGAAAGC
GAAAGGAGCGGGCGCTAGGGCGCTGGCAAGTGTAGCGGTCACGCTGCG
CGTAACCACCACACCCGCCGCGCTTAATGCGCCGCTACAGGGCGCGTA
CTATGGTTGCTTTGACGTATGCGGTGTGAAATACCGCACAGATGCGTAA
GGAGAAAATACCGCATCAGGCGCCCCTGCAGGCAGCTGCGCGCTCGCT
CGCTCACTGAGGCCGCCCGGGCAAAGCCCGGGCGTCGGGCGACCTTTG
GTCGCCCGGCCTCAGTGAGCGAGCGAGCGCGCAGAGAGGGAGTGGCC
AACTCCATCACTAGGGGTTCCTACGCGTCGTTACATAACTTACGGTAAA
TGGCCCGCCTGGCTGACCGCCCAACGACCCCCGCCCATTGACGTCAATA
ATGACGTATGTTCCCATAGTAACGCCAATAGGGACTTTCCATTGACGTC
AATGGGTGGAGTATTTACGGTAAACTGCCCACTTGGCAGTACATCAAG
TGTATCATATGCCAAGTACGCCCCCTATTGACGTCAATGACGGTAAATG
GCCCGCCTGGCATTATGCCCAGTACATGACCTTATGGGACTTTCCTACT
TGGCAGTACATCTACGTATTAGTCATCGCTATTACCATGGTCGAGGTGA
GCCCCACGTTCTGCTTCACTCTCCCCATCTCCCCCCCCTCCCCACCCCCA
ATTTTGTATTTATTTATTTTTTAATTATTTTGTGCAGCGATGGGGGCGGG
GGGGGGGGGGGGGCGCGCGCCAGGCGGGGCGGGGCGGGGCGAGGGGC
GGGGCGGGGCGAGGCGGAGAGGTGCGGCGGCAGCCAATCAGAGCGGC
GCGCTCCGAAAGTTTCCTTTTATGGCGAGGCGGCGGCGGCGGCGGCCC
TATAAAAAGCGAAGCGCGCGGCGGGCGGGAGTCGCTGCGACGCTGCCT
TCGCCCCGTGCCCCGCTCCGCCGCCGCCTCGCGCCGCCCGCCCCGGCTC
TGACTGACCGCGTTACTCCCACAGGTGAGCGGGCGGGACGGCCCTTCT
CCTCCGGGCTGTAATTAGCTGAGCAAGAGGTAAGGGTTTAAGGGATGG
 
TTGGTTGGTGGGGTATTAATGTTTAATTACCTGGAGCACCTGCCTGAAA
TCACTTTTTTTCAGGTACCGGTCGCCACCATGGCGAGACCCCTGTGCAC
ATTACTTCTGTTGATGGCTACCCTGGCAGGCGCCCTCGCCAGCGAGCTG
ACACAGGACCCTGCCGTGTCCGTGGCCCTGAAGCAGACCGTGACAATC
ACCTGCAGAGGCGATTCCCTGAGATCCCACTACGCCTCCTGGTACCAGA
AGAAGCCTGGCCAGGCCCCCGTGCTGCTGTTTTACGGCAAGAATAACC
GCCCCAGCGGCATCCCCGATAGATTTTCCGGCAGCGCCTCCGGCAACA
GAGCCAGCCTGACAATCACCGGCGCCCAGGCCGAGGACGAGGCTGATT
ACTACTGCAGCTCCAGAGATAAGAGCGGCAGCAGACTGTCCGTGTTTG
GCGGCGGCACCAAGCTGACCGTGCTCGGAGGAGGAGGAAGCGGAGGA
GGAGGCTCAGGCGGCGGCGGCTCTGAGGTGAGGCTGAGAGAGTCCGGC
GGCGGCCTGGTGAAGCCCGGAGGATCTCTGAGGCTGTCCTGCTCCGCCT
CCGGCTTCGATTTTGACAATGCCTGGATGACCTGGGTGAGACAGCCCCC
TGGCAAGGGCCTGGAGTGGGTGGGAAGGATCACAGGCCCCGGCGAGG
GCTGGTCCGTGGATTACGCCGAGTCCGTGAAGGGCAGGTTCACAATCT
CCAGGGATAACACCAAGAACACCCTGTACCTGGAGATGAACAACGTGA
GGACAGAGGATACCGGCTACTACTTTTGCGCCAGAACAGGCAAGTACT
ACGACTTTTGGTTCGGCTACCCCCCTGGCGAGGAGTACTTCCAGGATTG
GGGCCAGGGCACCCTGGTCATTGTGTCCAGCGGCGGCGGCAGTGG
CGGCGGCGGAAGCGGCGGCGGCTCTGGCGGCGGCAGCGGCG
GCGGCGGCTCCGACATCGTGATGACCCAGTCTCCTGATAGCCTGGCCGT
GAGCCTGGGCGAGAGAGTGACAATGAACTGTAAGTCTAGCCAGAGCCT
GCTGTACTCCACCAACCAGAAGAATTACCTGGCCTGGTATCAGCAGAA
GCCTGGCCAGTCCCCAAAGCTGCTGATCTATTGGGCATCTACAAGGGA
GAGCGGAGTGCCAGACAGATTCAGCGGATCCGGATCTGGAACCGACTT
CACCCTGACAATCTCCTCTGTGCAGGCCGAGGACGTGGCCGTGTACTAT
TGCCAGCAGTACTATAGCTACAGGACATTCGGCGGCGGCACCAAGCTG
GAGATCAAGCGCACCGTGGCCGGAGGAGGAGGATCTGGCGGAGGAGG
GTCCGGCGGCGGCGGCTCCCAGGTGCAGCTGCAGCAGAGCGGACCAGA
GGTGGTGAAGCCTGGAGCCTCCGTGAAGATGTCTTGTAAGGCCAGCGG
CTACACCTTCACATCCTATGTGATCCACTGGGTGAGGCAGAAGCCAGG
ACAGGGACTGGACTGGATCGGCTACATCAACCCTTATAATGATGGCAC
CGACTACGATGAGAAGTTTAAGGGCAAGGCCACCCTGACATCCGATAC
CAGCACATCCACCGCCTATATGGAGCTGAGCTCCCTGCGGTCTGAGGA
CACAGCCGTGTACTATTGCGCCAGAGAGAAGGATAACTACGCAACCGG
AGCATGGTTCGCATATTGGGGACAGGGTACCCTGGTCACCGTGTCTAGC
GAGCCCAAGAGCTGTGACAAGACACACACCTGCCCTCCATGTCCAGCA
CCAGAGCTGCTGGGAGGGCCCTCCGTGTTCCTGTTTCCCCCTAAGCCAA
AGGATACACTGATGATCTCCAGGACACCAGAGGTGACCTGCGTGGTGG
TGGACGTGTCTCACGAGGATCCTGAGGTGAAGTTTAACTGGTACGTGG
 
ACGGCGTGGAGGTGCACAATGCCAAGACCAAGCCTAGGGAGGAGCAG
TACAATTCTACATATCGCGTGGTGAGCGTGCTGACCGTGCTGCACCAGG
ATTGGCTGAACGGCAAGGAGTATAAGTGCAAGGTGAGCAATAAGGCCC
TGCCTGCCCCAATCGAGAAGACAATCTCCAAGGCAAAGGGACAGCCAA
GGGAGCCTCAGGTGTACACCCTGCCACCCTCTCGCGACGAGCTGACAA
AGAACCAGGTGAGCCTGACCTGTCTGGTGAAGGGCTTCTATCCTAGCG
ATATTGCCGTGGAGTGGGAGTCCAATGGCCAGCCAGAGAACAATTACA
AGACCACACCTCCAGTGCTGGACTCCGATGGCTCTTTCTTTCTGTATTCC
AAGCTGACAGTGGACAAGTCTAGATGGCAGCAGGGCAACGTGTTTTCT
TGTAGCGTGATGCACGAGGCCCTGCACAATCACTACACCCAGAAGTCC
CTGTCTCTGAGCCCCGGCAAGGGCGGAGGAGGAAGCGGAGGAGGCGG
CTCTGGCGGAGGAGGTTCCGGAGGAGGCGGAAGCGGCGGAGGAGGCT
CTCTGCTGGAGCAGGAGAACAAGGAGCAGCAGAATCAGAGCGAGGAG
ATCCTGTCCCACATCCTGTCCACATACAACAATATCGAGAGAGATTGGG
AGATGTGGTAGGTCGACAATCAACCTCTGGATTACAAAATTTGTGAAA
GATTGACTGGTATTCTTAACTATGTTGCTCCTTTTACGCTATGTGGATAC
GCTGCTTTAATGCCTTTGTATCATGCTATTGCTTCCCGTATGGCTTTCAT
TTTCTCCTCCTTGTATAAATCCTGGTTGCTGTCTCTTTATGAGGAGTTGT
GGCCCGTTGTCAGGCAACGTGGCGTGGTGTGCACTGTGTTTGCTGACGC
AACCCCCACTGGTTGGGGCATTGCCACCACCTGTCAGCTCCTTTCCGGG
ACTTTCGCTTTCCCCCTCCCTATTGCCACGGCGGAACTCATCGCCGCCT
GCCTTGCCCGCTGCTGGACAGGGGCTCGGCTGTTGGGCACTGACAATTC
CGTGGTGTTGTCGGGGAAGCTGACGTCCTTTCCATGGCTGCTCGCCTGT
GTTGCCACCTGGATTCTGCGCGGGACGTCCTTCTGCTACGTCCCTTCGG
CCCTCAATCCAGCGGACCTTCCTTCCCGCGGCCTGCTGCCGGCTCTGCG
GCCTCTTCCGCGTCTTCGCCTTCGCCCTCAGACGAGTCGGATCTCCCTTT
GGGCCGCCTCCCCGCGAATTCGTTTATTTGTGAAATTTGTGATGCTATT
GCTTTATTTGTAACCATCTAGCTTTATTTGTGAAATTTGTGATGCTATTG
CTTTATTTGTAACCATTATAAGCTGCAATAAACAAGTTAACAACAACAA
TTGCATTCATTTTATGTTTCAGGTTCAGGGGGAGATGTGGGAGGTTTTT
TAAAGTTTAAACAGGAACCCCTAGTGATGGAGTTGGCCACTCCCTCTCT
GCGCGCTCGCTCGCTCACTGAGGCCGGGCGACCAAAGGTCGCCCGACG
CCCGGGCGGCCTCAGTGAGCGAGCGAGCGCGCAGCTGCCTGCAGG
   SEQ ID NO: 21 
ACATGTGAGCAAAAGGCCAGCAAAAGGCCAGGAACCGTAAAAAGGCC
GCGTTGCTGGCGTTTTTCCATAGGCTCCGCCCCCCTGACGAGCATCACA
AAAATCGACGCTCAAGTCAGAGGTGGCGAAACCCGACAGGACTATAAA
GATACCAGGCGTTTCCCCCTGGAAGCTCCCTCGTGCGCTCTCCTGTTCC
GACCCTGCCGCTTACCGGATACCTGTCCGCCTTTCTCCCTTCGGGAAGC
GTGGCGCTTTCTCATAGCTCACGCTGTAGGTATCTCAGTTCGGTGTAGG
 
TCGTTCGCTCCAAGCTGGGCTGTGTGCACGAACCCCCCGTTCAGCCCGA
CCGCTGCGCCTTATCCGGTAACTATCGTCTTGAGTCCAACCCGGTAAGA
CACGACTTATCGCCACTGGCAGCAGCCACTGGTAACAGGATTAGCAGA
GCGAGGTATGTAGGCGGTGCTACAGAGTTCTTGAAGTGGTGGCCTAAC
TACGGCTACACTAGAAGGACAGTATTTGGTATCTGCGCTCTGCTGAAGC
CAGTTACCTTCGGAAAAAGAGTTGGTAGCTCTTGATCCGGCAAACAAA
CCACCGCTGGTAGCGGTGGTTTTTTTGTTTGCAAGCAGCAGATTACGCG
CAGAAAAAAAGGATCTCAAGAAGATCCTTTGATCTTTTCTACGGGGTCT
GACGCTCAGTGGAACGAAAACTCACGTTAAGGGATTTTGGTCATGAGA
TTATCAAAAAGGATCTTCACCTAGATCCTTTTAAATTAAAAATGAAGTT
TTAAATCAATCTAAAGTATATATGAGTAAACTTGGTCTGACAGTTACCA
ATGCTTAATCAGTGAGGCACCTATCTCAGCGATCTGTCTATTTCGTTCA
TCCATAGTTGCCTGACTCCCCGTCGTGTAGATAACTACGATACGGGAGG
GCTTACCATCTGGCCCCAGTGCTGCAATGATACCGCGAGACCCACGCTC
ACCGGCTCCAGATTTATCAGCAATAAACCAGCCAGCCGGAAGGGCCGA
GCGCAGAAGTGGTCCTGCAACTTTATCCGCCTCCATCCAGTCTATTAAT
TGTTGCCGGGAAGCTAGAGTAAGTAGTTCGCCAGTTAATAGTTTGCGCA
ACGTTGTTGCCATTGCTACAGGCATCGTGGTGTCACGCTCGTCGTTTGG
TATGGCTTCATTCAGCTCCGGTTCCCAACGATCAAGGCGAGTTACATGA
TCCCCCATGTTGTGCAAAAAAGCGGTTAGCTCCTTCGGTCCTCCGATCG
TTGTCAGAAGTAAGTTGGCCGCAGTGTTATCACTCATGGTTATGGCAGC
ACTGCATAATTCTCTTACTGTCATGCCATCCGTAAGATGCTTTTCTGTGA
CTGGTGAGTACTCAACCAAGTCATTCTGAGAATAGTGTATGCGGCGAC
CGAGTTGCTCTTGCCCGGCGTCAATACGGGATAATACCGCGCCACATA
GCAGAACTTTAAAAGTGCTCATCATTGGAAAACGTTCTTCGGGGCGAA
AACTCTCAAGGATCTTACCGCTGTTGAGATCCAGTTCGATGTAACCCAC
TCGTGCACCCAACTGATCTTCAGCATCTTTTACTTTCACCAGCGTTTCTG
GGTGAGCAAAAACAGGAAGGCAAAATGCCGCAAAAAAGGGAATAAGG
GCGACACGGAAATGTTGAATACTCATACTCTTCCTTTTTCAATATTATT
GAAGCATTTATCAGGGTTATTGTCTCATGAGCGGATACATATTTGAATG
TATTTAGAAAAATAAACAAATAGGGGTTCCGCGCACATTTCCCCGAAA
AGTGCCACCTGACGTCTAAGAAACCATTATTATCATGACATTAACCTAT
AAAAATAGGCGTATCACGAGGCCCTTTCGTCTCGCGCGTTTCGGTGATG
ACGGTGAAAACCTCTGACACATGCAGCTCCCGGAGACGGTCACAGCTT
GTCTGTAAGCGGATGCCGGGAGCAGACAAGCCCGTCAGGGCGCGTCAG
CGGGTGTTGGCGGGTGTCGGGGCTGGCTTAACTATGCGGCATCAGAGC
AGATTGTACTGAGAGTGCACCATAAAATTGTAAACGTTAATATTTTGTT
AAAATTCGCGTTAAATTTTTGTTAAATCAGCTCATTTTTTAACCAATAG
GCCGAAATCGGCAAAATCCCTTATAAATCAAAAGAATAGCCCGAGATA
GGGTTGAGTGTTGTTCCAGTTTGGAACAAGAGTCCACTATTAAAGAAC
 
GTGGACTCCAACGTCAAAGGGCGAAAAACCGTCTATCAGGGCGATGGC
CCACTACGTGAACCATCACCCAAATCAAGTTTTTTGGGGTCGAGGTGCC
GTAAAGCACTAAATCGGAACCCTAAAGGGAGCCCCCGATTTAGAGCTT
GACGGGGAAAGCCGGCGAACGTGGCGAGAAAGGAAGGGAAGAAAGC
GAAAGGAGCGGGCGCTAGGGCGCTGGCAAGTGTAGCGGTCACGCTGCG
CGTAACCACCACACCCGCCGCGCTTAATGCGCCGCTACAGGGCGCGTA
CTATGGTTGCTTTGACGTATGCGGTGTGAAATACCGCACAGATGCGTAA
GGAGAAAATACCGCATCAGGCGCCCCTGCAGGCAGCTGCGCGCTCGCT
CGCTCACTGAGGCCGCCCGGGCAAAGCCCGGGCGTCGGGCGACCTTTG
GTCGCCCGGCCTCAGTGAGCGAGCGAGCGCGCAGAGAGGGAGTGGCC
AACTCCATCACTAGGGGTTCCTACGCGTCGTTACATAACTTACGGTAAA
TGGCCCGCCTGGCTGACCGCCCAACGACCCCCGCCCATTGACGTCAATA
ATGACGTATGTTCCCATAGTAACGCCAATAGGGACTTTCCATTGACGTC
AATGGGTGGAGTATTTACGGTAAACTGCCCACTTGGCAGTACATCAAG
TGTATCATATGCCAAGTACGCCCCCTATTGACGTCAATGACGGTAAATG
GCCCGCCTGGCATTATGCCCAGTACATGACCTTATGGGACTTTCCTACT
TGGCAGTACATCTACGTATTAGTCATCGCTATTACCATGGTCGAGGTGA
GCCCCACGTTCTGCTTCACTCTCCCCATCTCCCCCCCCTCCCCACCCCCA
ATTTTGTATTTATTTATTTTTTAATTATTTTGTGCAGCGATGGGGGCGGGG
GGGGGGGGGGGGCGCGCGCCAGGCGGGGCGGGGCGGGGCGAGGGGC
GGGGCGGGGCGAGGCGGAGAGGTGCGGCGGCAGCCAATCAGAGCGGC
GCGCTCCGAAAGTTTCCTTTTATGGCGAGGCGGCGGCGGCGGCGGCCC
TATAAAAAGCGAAGCGCGCGGCGGGCGGGAGTCGCTGCGACGCTGCCT
TCGCCCCGTGCCCCGCTCCGCCGCCGCCTCGCGCCGCCCGCCCCGGCTC
TGACTGACCGCGTTACTCCCACAGGTGAGCGGGCGGGACGGCCCTTCT
CCTCCGGGCTGTAATTAGCTGAGCAAGAGGTAAGGGTTTAAGGGATGG
TTGGTTGGTGGGGTATTAATGTTTAATTACCTGGAGCACCTGCCTGAAA
TCACTTTTTTTCAGGTACCGGTCGCCACCATGGCGAGACCCCTGTGCAC
ATTACTTCTGTTGATGGCTACCCTGGCAGGCGCCCTCGCCAGCGAGCTG
ACACAGGACCCTGCCGTGTCCGTGGCCCTGAAGCAGACCGTGACAATC
ACCTGCAGAGGCGATTCCCTGAGATCCCACTACGCCTCCTGGTACCAGA
AGAAGCCTGGCCAGGCCCCCGTGCTGCTGTTTTACGGCAAGAATAACC
GCCCCAGCGGCATCCCCGATAGATTTTCCGGCAGCGCCTCCGGCAACA
GAGCCAGCCTGACAATCACCGGCGCCCAGGCCGAGGACGAGGCTGATT
ACTACTGCAGCTCCAGAGATAAGAGCGGCAGCAGACTGTCCGTGTTTG
GCGGCGGCACCAAGCTGACCGTGCTCGGAGGAGGAGGAAGCGGAGGA
GGAGGCTCAGGCGGCGGCGGCTCTGAGGTGAGGCTGAGAGAGTCCGGC
GGCGGCCTGGTGAAGCCCGGAGGATCTCTGAGGCTGTCCTGCTCCGCCT
CCGGCTTCGATTTTGACAATGCCTGGATGACCTGGGTGAGACAGCCCCC
TGGCAAGGGCCTGGAGTGGGTGGGAAGGATCACAGGCCCCGGCGAGG
 
GCTGGTCCGTGGATTACGCCGAGTCCGTGAAGGGCAGGTTCACAATCT
CCAGGGATAACACCAAGAACACCCTGTACCTGGAGATGAACAACGTGA
GGACAGAGGATACCGGCTACTACTTTTGCGCCAGAACAGGCAAGTACT
ACGACTTTTGGTTCGGCTACCCCCCTGGCGAGGAGTACTTCCAGGATTG
GGGCCAGGGCACCCTGGTCATTGTGTCCAGCGGCGGCGGCAGTGG
CGGCGGCGGAAGCGGCGGCGGCTCTGGCGGCGGCGGCAGCGGCG
GCGGCGGCTCCGACATCGTGATGACCCAGTCTCCTGATAGCCTGGCCGT
GAGCCTGGGCGAGAGAGTGACAATGAACTGTAAGTCTAGCCAGAGCCT
GCTGTACTCCACCAACCAGAAGAATTACCTGGCCTGGTATCAGCAGAA
GCCTGGCCAGTCCCCAAAGCTGCTGATCTATTGGGCATCTACAAGGGA
GAGCGGAGTGCCAGACAGATTCAGCGGATCCGGATCTGGAACCGACTT
CACCCTGACAATCTCCTCTGTGCAGGCCGAGGACGTGGCCGTGTACTAT
TGCCAGCAGTACTATAGCTACAGGACATTCGGCGGCGGCACCAAGCTG
GAGATCAAGCGCACCGTGGCCGGAGGAGGAGGATCTGGCGGAGGAGG
GTCCGGCGGCGGCGGCTCCCAGGTGCAGCTGCAGCAGAGCGGACCAGA
GGTGGTGAAGCCTGGAGCCTCCGTGAAGATGTCTTGTAAGGCCAGCGG
CTACACCTTCACATCCTATGTGATCCACTGGGTGAGGCAGAAGCCAGG
ACAGGGACTGGACTGGATCGGCTACATCAACCCTTATAATGATGGCAC
CGACTACGATGAGAAGTTTAAGGGCAAGGCCACCCTGACATCCGATAC
CAGCACATCCACCGCCTATATGGAGCTGAGCTCCCTGCGGTCTGAGGA
CACAGCCGTGTACTATTGCGCCAGAGAGAAGGATAACTACGCAACCGG
AGCATGGTTCGCATATTGGGGACAGGGTACCCTGGTCACCGTGTCTAGC
GAGCCCAAGAGCTGTGACAAGACACACACCTGCCCTCCATGTCCAGCA
CCAGAGCTGCTGGGAGGGCCCTCCGTGTTCCTGTTCCCCCCTAAGCCAA
AGGATACACTGATGATCTCCAGGACACCAGAGGTGACCTGCGTGGTGG
TGGACGTGTCTCACGAGGATCCTGAGGTGAAGTTTAACTGGTACGTGG
ACGGCGTGGAGGTGCACAATGCCAAGACCAAGCCTAGGGAGGAGCAG
TACAATTCTACATATCGCGTGGTGAGCGTGCTGACCGTGCTGCACCAGG
ATTGGCTGAACGGCAAGGAGTATAAGTGCAAGGTGAGCAATAAGGCCC
TGCCTGCCCCAATCGAGAAGACAATCTCCAAGGCAAAGGGACAGCCAA
GGGAGCCTCAGGTGTACACCCTGCCACCCTCTCGCGACGAGCTGACAA
AGAACCAGGTGAGCCTGACCTGTCTGGTGAAGGGCTTCTATCCTAGCG
ATATTGCCGTGGAGTGGGAGTCCAATGGCCAGCCAGAGAACAATTACA
AGACCACACCTCCAGTGCTGGACTCCGATGGCTCTTTCTTTCTGTATTCC
AAGCTGACAGTGGACAAGTCTAGATGGCAGCAGGGCAACGTGTTTTCT
TGTAGCGTGATGCACGAGGCCCTGCACAATCACTACACCCAGAAGTCC
CTGTCTCTGAGCCCCGGCAAGTAGGTCGACAATCAACCTCTGGATTACA
AAATTTGTGAAAGATTGACTGGTATTCTTAACTATGTTGCTCCTTTTACG
CTATGTGGATACGCTGCTTTAATGCCTTTGTATCATGCTATTGCTTCCCG
TATGGCTTTCATTTTCTCCTCCTTGTATAAATCCTGGTTGCTGTCTCTTTA
 
TGAGGAGTTGTGGCCCGTTGTCAGGCAACGTGGCGTGGTGTGCACTGT
GTTTGCTGACGCAACCCCCACTGGTTGGGGCATTGCCACCACCTGTCAG
CTCCTTTCCGGGACTTTCGCTTTCCCCCTCCCTATTGCCACGGCGGAACT
CATCGCCGCCTGCCTTGCCCGCTGCTGGACAGGGGCTCGGCTGTTGGGC
ACTGACAATTCCGTGGTGTTGTCGGGGAAGCTGACGTCCTTTCCATGGC
TGCTCGCCTGTGTTGCCACCTGGATTCTGCGCGGGACGTCCTTCTGCTA
CGTCCCTTCGGCCCTCAATCCAGCGGACCTTCCTTCCCGCGGCCTGCTG
CCGGCTCTGCGGCCTCTTCCGCGTCTTCGCCTTCGCCCTCAGACGAGTC
GGATCTCCCTTTGGGCCGCCTCCCCGCGAATTCGTTTATTTGTGAAATTT
GTGATGCTATTGCTTTATTTGTAACCATCTAGCTTTATTTGTGAAATTTG
TGATGCTATTGCTTTATTTGTAACCATTATAAGCTGCAATAAACAAGTT
AACAACAACAATTGCATTCATTTTATGTTTCAGGTTCAGGGGGAGATGT
GGGAGGTTTTTTAAAGTTTAAACAGGAACCCCTAGTGATGGAGTTGGC
CACTCCCTCTCTGCGCGCTCGCTCGCTCACTGAGGCCGGGCGACCAAAG
GTCGCCCGACGCCCGGGCGGCCTCAGTGAGCGAGCGAGCGCGCAGCTG
CCTGCAGG
   SEQ ID NO: 22 
ACATGTGAGCAAAAGGCCAGCAAAAGGCCAGGAACCGTAAAAA
GGCCGCGTTGCTGGCGTTTTTCCATAGGCTCCGCCCCCCTGACG
AGCATCACAAAAATCGACGCTCAAGTCAGAGGTGGCGAAACCC
GACAGGACTATAAAGATACCAGGCGTTTCCCCCTGGAAGCTCCC
TCGTGCGCTCTCCTGTTCCGACCCTGCCGCTTACCGGATACCTGT
CCGCCTTTCTCCCTTCGGGAAGCGTGGCGCTTTCTCATAGCTCAC
GCTGTAGGTATCTCAGTTCGGTGTAGGTCGTTCGCTCCAAGCTG
GGCTGTGTGCACGAACCCCCCGTTCAGCCCGACCGCTGCGCCTT
ATCCGGTAACTATCGTCTTGAGTCCAACCCGGTAAGACACGACT
TATCGCCACTGGCAGCAGCCACTGGTAACAGGATTAGCAGAGC
GAGGTATGTAGGCGGTGCTACAGAGTTCTTGAAGTGGTGGCCTA
ACTACGGCTACACTAGAAGGACAGTATTTGGTATCTGCGCTCTG
CTGAAGCCAGTTACCTTCGGAAAAAGAGTTGGTAGCTCTTGATC
CGGCAAACAAACCACCGCTGGTAGCGGTGGTTTTTTTGTTTGCA
AGCAGCAGATTACGCGCAGAAAAAAAGGATCTCAAGAAGATCC
TTTGATCTTTTCTACGGGGTCTGACGCTCAGTGGAACGAAAACT
CACGTTAAGGGATTTTGGTCATGAGATTATCAAAAAGGATCTTC
ACCTAGATCCTTTTAAATTAAAAATGAAGTTTTAAATCAATCTA
AAGTATATATGAGTAAACTTGGTCTGACAGTTACCAATGCTTAA
TCAGTGAGGCACCTATCTCAGCGATCTGTCTATTTCGTTCATCCA
TAGTTGCCTGACTCCCCGTCGTGTAGATAACTACGATACGGGAG
GGCTTACCATCTGGCCCCAGTGCTGCAATGATACCGCGAGACCC
ACGCTCACCGGCTCCAGATTTATCAGCAATAAACCAGCCAGCCG
 
GAAGGGCCGAGCGCAGAAGTGGTCCTGCAACTTTATCCGCCTCC
ATCCAGTCTATTAATTGTTGCCGGGAAGCTAGAGTAAGTAGTTC
GCCAGTTAATAGTTTGCGCAACGTTGTTGCCATTGCTACAGGCA
TCGTGGTGTCACGCTCGTCGTTTGGTATGGCTTCATTCAGCTCCG
GTTCCCAACGATCAAGGCGAGTTACATGATCCCCCATGTTGTGC
AAAAAAGCGGTTAGCTCCTTCGGTCCTCCGATCGTTGTCAGAAG
TAAGTTGGCCGCAGTGTTATCACTCATGGTTATGGCAGCACTGC
ATAATTCTCTTACTGTCATGCCATCCGTAAGATGCTTTTCTGTGA
CTGGTGAGTACTCAACCAAGTCATTCTGAGAATAGTGTATGCGG
CGACCGAGTTGCTCTTGCCCGGCGTCAATACGGGATAATACCGC
GCCACATAGCAGAACTTTAAAAGTGCTCATCATTGGAAAACGTT
CTTCGGGGCGAAAACTCTCAAGGATCTTACCGCTGTTGAGATCC
AGTTCGATGTAACCCACTCGTGCACCCAACTGATCTTCAGCATC
TTTTACTTTCACCAGCGTTTCTGGGTGAGCAAAAACAGGAAGGC
AAAATGCCGCAAAAAAGGGAATAAGGGCGACACGGAAATGTTG
AATACTCATACTCTTCCTTTTTCAATATTATTGAAGCATTTATCA
GGGTTATTGTCTCATGAGCGGATACATATTTGAATGTATTTAGA
AAAATAAACAAATAGGGGTTCCGCGCACATTTCCCCGAAAAGT
GCCACCTGACGTCTAAGAAACCATTATTATCATGACATTAACCT
ATAAAAATAGGCGTATCACGAGGCCCTTTCGTCTCGCGCGTTTC
GGTGATGACGGTGAAAACCTCTGACACATGCAGCTCCCGGAGA
CGGTCACAGCTTGTCTGTAAGCGGATGCCGGGAGCAGACAAGC
CCGTCAGGGCGCGTCAGCGGGTGTTGGCGGGTGTCGGGGCTGGC
TTAACTATGCGGCATCAGAGCAGATTGTACTGAGAGTGCACCAT
AAAATTGTAAACGTTAATATTTTGTTAAAATTCGCGTTAAATTTT
TGTTAAATCAGCTCATTTTTTAACCAATAGGCCGAAATCGGCAA
AATCCCTTATAAATCAAAAGAATAGCCCGAGATAGGGTTGAGTG
TTGTTCCAGTTTGGAACAAGAGTCCACTATTAAAGAACGTGGAC
TCCAACGTCAAAGGGCGAAAAACCGTCTATCAGGGCGATGGCC
CACTACGTGAACCATCACCCAAATCAAGTTTTTTGGGGTCGAGG
TGCCGTAAAGCACTAAATCGGAACCCTAAAGGGAGCCCCCGATT
TAGAGCTTGACGGGGAAAGCCGGCGAACGTGGCGAGAAAGGAA
GGGAAGAAAGCGAAAGGAGCGGGCGCTAGGGCGCTGGCAAGTG
TAGCGGTCACGCTGCGCGTAACCACCACACCCGCCGCGCTTAAT
GCGCCGCTACAGGGCGCGTACTATGGTTGCTTTGACGTATGCGG
TGTGAAATACCGCACAGATGCGTAAGGAGAAAATACCGCATCA
GGCGCCCCTGCAGGCAGCTGCGCGCTCGCTCGCTCACTGAGGCC
GCCCGGGCAAAGCCCGGGCGTCGGGCGACCTTTGGTCGCCCGGC
CTCAGTGAGCGAGCGAGCGCGCAGAGAGGGAGTGGCCAACTCC
ATCACTAGGGGTTCCTACGCGTCGTTACATAACTTACGGTAAAT
 
GGCCCGCCTGGCTGACCGCCCAACGACCCCCGCCCATTGACGTC
AATAATGACGTATGTTCCCATAGTAACGCCAATAGGGACTTTCC
ATTGACGTCAATGGGTGGAGTATTTACGGTAAACTGCCCACTTG
GCAGTACATCAAGTGTATCATATGCCAAGTACGCCCCCTATTGA
CGTCAATGACGGTAAATGGCCCGCCTGGCATTATGCCCAGTACA
TGACCTTATGGGACTTTCCTACTTGGCAGTACATCTACGTATTAG
TCATCGCTATTACCATGGTCGAGGTGAGCCCCACGTTCTGCTTC
ACTCTCCCCATCTCCCCCCCCTCCCCACCCCCAATTTTGTATTTA
TTTATTTTTTAATTATTTTGTGCAGCGATGGGGGCGGGGGGGGG
GGGGGGGCGCGCGCCAGGCGGGGCGGGGCGGGGCGAGGGGCG
GGGCGGGGCGAGGCGGAGAGGTGCGGCGGCAGCCAATCAGAGC
GGCGCGCTCCGAAAGTTTCCTTTTATGGCGAGGCGGCGGCGGCG
GCGGCCCTATAAAAAGCGAAGCGCGCGGCGGGCGGGAGTCGCT
GCGACGCTGCCTTCGCCCCGTGCCCCGCTCCGCCGCCGCCTCGC
GCCGCCCGCCCCGGCTCTGACTGACCGCGTTACTCCCACAGGTG
AGCGGGCGGGACGGCCCTTCTCCTCCGGGCTGTAATTAGCTGAG
CAAGAGGTAAGGGTTTAAGGGATGGTTGGTTGGTGGGGTATTAA
TGTTTAATTACCTGGAGCACCTGCCTGAAATCACTTTTTTTCAGG
TACCGGTCGCCACCATGGCGAGACCCCTGTGCACATTACTTCTG
TTGATGGCTACCCTGGCAGGCGCCCTCGCCAGCGAGCTGACACA
GGACCCTGCCGTGTCCGTGGCCCTGAAGCAGACCGTGACAATCA
CCTGCAGAGGCGATTCCCTGAGATCCCACTACGCCTCCTGGTAC
CAGAAGAAGCCTGGCCAGGCCCCCGTGCTGCTGTTTTACGGCAA
GAATAACCGCCCCAGCGGCATCCCCGATAGATTTTCCGGCAGCG
CCTCCGGCAACAGAGCCAGCCTGACAATCACCGGCGCCCAGGC
CGAGGACGAGGCTGATTACTACTGCAGCTCCAGAGATAAGAGC
GGCAGCAGACTGTCCGTGTTTGGCGGCGGCACCAAGCTGACCGT
GCTCGGAGGAGGAGGAAGCGGAGGAGGAGGCTCAGGCGGCGG
CGGCTCTGAGGTGAGGCTGAGAGAGTCCGGCGGCGGCCTGGTG
AAGCCCGGAGGATCTCTGAGGCTGTCCTGCTCCGCCTCCGGCTT
CGATTTTGACAATGCCTGGATGACCTGGGTGAGACAGCCCCCTG
GCAAGGGCCTGGAGTGGGTGGGAAGGATCACAGGCCCCGGCGA
GGGCTGGTCCGTGGATTACGCCGAGTCCGTGAAGGGCAGGTTCA
CAATCTCCAGGGATAACACCAAGAACACCCTGTACCTGGAGATG
AACAACGTGAGGACAGAGGATACCGGCTACTACTTTTGCGCCAG
AACAGGCAAGTACTACGACTTTTGGTTCGGCTACCCCCCTGGCG
AGGAGTACTTCCAGGATTGGGGCCAGGGCACCCTGGTCATTGTG
TCCAGCGGCGGCGGCGGCAGTGGCGGCGGCGGAAGCGGCGGCG
GCGGCTCTGGCGGCGGCGGCAGCGGCGGCGGCGGCTCCGACAT
CGTGATGACCCAGTCTCCTGATAGCCTGGCCGTGAGCCTGGGCG
 
AGAGAGTGACAATGAACTGTAAGTCTAGCCAGAGCCTGCTGTAC
TCCACCAACCAGAAGAATTACCTGGCCTGGTATCAGCAGAAGCC
TGGCCAGTCCCCAAAGCTGCTGATCTATTGGGCATCTACAAGGG
AGAGCGGAGTGCCAGACAGATTCAGCGGATCCGGATCTGGAAC
CGACTTCACCCTGACAATCTCCTCTGTGCAGGCCGAGGACGTGG
CCGTGTACTATTGCCAGCAGTACTATAGCTACAGGACATTCGGC
GGCGGCACCAAGCTGGAGATCAAGCGCACCGTGGCCGGAGGAG
GAGGATCTGGCGGAGGAGGGTCCGGCGGCGGCGGCTCCCAGGT
GCAGCTGCAGCAGAGCGGACCAGAGGTGGTGAAGCCTGGAGCC
TCCGTGAAGATGTCTTGTAAGGCCAGCGGCTACACCTTCACATC
CTATGTGATCCACTGGGTGAGGCAGAAGCCAGGACAGGGACTG
GACTGGATCGGCTACATCAACCCTTATAATGATGGCACCGACTA
CGATGAGAAGTTTAAGGGCAAGGCCACCCTGACATCCGATACC
AGCACATCCACCGCCTATATGGAGCTGAGCTCCCTGCGGTCTGA
GGACACAGCCGTGTACTATTGCGCCAGAGAGAAGGATAACTAC
GCAACCGGAGCATGGTTCGCATATTGGGGACAGGGTACCCTGGT
CACCGTGTCTAGCGAGCCCAAGAGCTGTGACAAGACACACACCT
GCCCTCCATGTCCAGCACCAGAGCTGCTGGGAGGGCCCTCCGTG
TTCCTGTTTCCCCCTAAGCCAAAGGATACACTGATGATCTCCAG
GACACCAGAGGTGACCTGCGTGGTGGTGGACGTGTCTCACGAG
GATCCTGAGGTGAAGTTTAACTGGTACGTGGACGGCGTGGAGGT
GCACAATGCCAAGACCAAGCCTAGGGAGGAGCAGTACAATTCT
ACATATCGCGTGGTGAGCGTGCTGACCGTGCTGCACCAGGATTG
GCTGAACGGCAAGGAGTATAAGTGCAAGGTGAGCAATAAGGCC
CTGCCTGCCCCAATCGAGAAGACAATCTCCAAGGCAAAGGGAC
AGCCAAGGGAGCCTCAGGTGTACACCCTGCCACCCTCTCGCGAC
GAGCTGACAAAGAACCAGGTGAGCCTGACCTGTCTGGTGAAGG
GCTTCTATCCTAGCGATATTGCCGTGGAGTGGGAGTCCAATGGC
CAGCCAGAGAACAATTACAAGACCACACCTCCAGTGCTGGACTC
CGATGGCTCTTTCTTTCTGTATTCCAAGCTGACAGTGGACAAGT
CTAGATGGCAGCAGGGCAACGTGTTTTCTTGTAGCGTGATGCAC
GAGGCCCTGCACAATCACTACACCCAGAAGTCCCTGTCTCTGAG
CCCCGGCAAGGGCGGAGGAGGAAGCGGAGGAGGCGGCTCTGGC
GGAGGAGGTTCCGGAGGAGGCGGAAGCGGCGGAGGAGGCTCTT
GGGAGCAGAAGATCGAGGAGCTGCTGAAGAAGGCCGAGGAGCA
GCAGAAGAAGAATGAGGAGGAGCTGAAGAAGCTGGAGAAGTA
GGTCGACAATCAACCTCTGGATTACAAAATTTGTGAAAGATTGA
CTGGTATTCTTAACTATGTTGCTCCTTTTACGCTATGTGGATACG
CTGCTTTAATGCCTTTGTATCATGCTATTGCTTCCCGTATGGCTT
TCATTTTCTCCTCCTTGTATAAATCCTGGTTGCTGTCTCTTTATG
 
AGGAGTTGTGGCCCGTTGTCAGGCAACGTGGCGTGGTGTGCACT
GTGTTTGCTGACGCAACCCCCACTGGTTGGGGCATTGCCACCAC
CTGTCAGCTCCTTTCCGGGACTTTCGCTTTCCCCCTCCCTATTGC
CACGGCGGAACTCATCGCCGCCTGCCTTGCCCGCTGCTGGACAG
GGGCTCGGCTGTTGGGCACTGACAATTCCGTGGTGTTGTCGGGG
AAGCTGACGTCCTTTCCATGGCTGCTCGCCTGTGTTGCCACCTG
GATTCTGCGCGGGACGTCCTTCTGCTACGTCCCTTCGGCCCTCA
ATCCAGCGGACCTTCCTTCCCGCGGCCTGCTGCCGGCTCTGCGG
CCTCTTCCGCGTCTTCGCCTTCGCCCTCAGACGAGTCGGATCTCC
CTTTGGGCCGCCTCCCCGCGAATTCGTTTATTTGTGAAATTTGTG
ATGCTATTGCTTTATTTGTAACCATTATAAGCTGCAATAAACAA
GTTAACAACAACAATTGCATTCATTTTATGTTTCAGGTTCAGGG
GGAGATGTGGGAGGTTTTTTAAAGTTTAAACAGGAACCCCTAGT
GATGGAGTTGGCCACTCCCTCTCTGCGCGCTCGCTCGCTCACTG
AGGCCGGGCGACCAAAGGTCGCCCGACGCCCGGGCGGCCTCAG
TGAGCGAGCGAGCGCGCAGCTGCCTGCAGG
   SEQ ID NO: 23 
ACATGTGAGCAAAAGGCCAGCAAAAGGCCAGGAACCGTAAAAA
GGCCGCGTTGCTGGCGTTTTTCCATAGGCTCCGCCCCCCTGACG
AGCATCACAAAAATCGACGCTCAAGTCAGAGGTGGCGAAACCC
GACAGGACTATAAAGATACCAGGCGTTTCCCCCTGGAAGCTCCC
TCGTGCGCTCTCCTGTTCCGACCCTGCCGCTTACCGGATACCTGT
CCGCCTTTCTCCCTTCGGGAAGCGTGGCGCTTTCTCATAGCTCAC
GCTGTAGGTATCTCAGTTCGGTGTAGGTCGTTCGCTCCAAGCTG
GGCTGTGTGCACGAACCCCCCGTTCAGCCCGACCGCTGCGCCTT
ATCCGGTAACTATCGTCTTGAGTCCAACCCGGTAAGACACGACT
TATCGCCACTGGCAGCAGCCACTGGTAACAGGATTAGCAGAGC
GAGGTATGTAGGCGGTGCTACAGAGTTCTTGAAGTGGTGGCCTA
ACTACGGCTACACTAGAAGGACAGTATTTGGTATCTGCGCTCTG
CTGAAGCCAGTTACCTTCGGAAAAAGAGTTGGTAGCTCTTGATC
CGGCAAACAAACCACCGCTGGTAGCGGTGGTTTTTTTGTTTGCA
AGCAGCAGATTACGCGCAGAAAAAAAGGATCTCAAGAAGATCC
TTTGATCTTTTCTACGGGGTCTGACGCTCAGTGGAACGAAAACT
CACGTTAAGGGATTTTGGTCATGAGATTATCAAAAAGGATCTTC
ACCTAGATCCTTTTAAATTAAAAATGAAGTTTTAAATCAATCTA
AAGTATATATGAGTAAACTTGGTCTGACAGTTACCAATGCTTAA
TCAGTGAGGCACCTATCTCAGCGATCTGTCTATTTCGTTCATCCA
TAGTTGCCTGACTCCCCGTCGTGTAGATAACTACGATACGGGAG
GGCTTACCATCTGGCCCCAGTGCTGCAATGATACCGCGAGACCC
ACGCTCACCGGCTCCAGATTTATCAGCAATAAACCAGCCAGCCG
 
GAAGGGCCGAGCGCAGAAGTGGTCCTGCAACTTTATCCGCCTCC
ATCCAGTCTATTAATTGTTGCCGGGAAGCTAGAGTAAGTAGTTC
GCCAGTTAATAGTTTGCGCAACGTTGTTGCCATTGCTACAGGCA
TCGTGGTGTCACGCTCGTCGTTTGGTATGGCTTCATTCAGCTCCG
GTTCCCAACGATCAAGGCGAGTTACATGATCCCCCATGTTGTGC
AAAAAAGCGGTTAGCTCCTTCGGTCCTCCGATCGTTGTCAGAAG
TAAGTTGGCCGCAGTGTTATCACTCATGGTTATGGCAGCACTGC
ATAATTCTCTTACTGTCATGCCATCCGTAAGATGCTTTTCTGTGA
CTGGTGAGTACTCAACCAAGTCATTCTGAGAATAGTGTATGCGG
CGACCGAGTTGCTCTTGCCCGGCGTCAATACGGGATAATACCGC
GCCACATAGCAGAACTTTAAAAGTGCTCATCATTGGAAAACGTT
CTTCGGGGCGAAAACTCTCAAGGATCTTACCGCTGTTGAGATCC
AGTTCGATGTAACCCACTCGTGCACCCAACTGATCTTCAGCATC
TTTTACTTTCACCAGCGTTTCTGGGTGAGCAAAAACAGGAAGGC
AAAATGCCGCAAAAAAGGGAATAAGGGCGACACGGAAATGTTG
AATACTCATACTCTTCCTTTTTCAATATTATTGAAGCATTTATCA
GGGTTATTGTCTCATGAGCGGATACATATTTGAATGTATTTAGA
AAAATAAACAAATAGGGGTTCCGCGCACATTTCCCCGAAAAGT
GCCACCTGACGTCTAAGAAACCATTATTATCATGACATTAACCT
ATAAAAATAGGCGTATCACGAGGCCCTTTCGTCTCGCGCGTTTC
GGTGATGACGGTGAAAACCTCTGACACATGCAGCTCCCGGAGA
CGGTCACAGCTTGTCTGTAAGCGGATGCCGGGAGCAGACAAGC
CCGTCAGGGCGCGTCAGCGGGTGTTGGCGGGTGTCGGGGCTGGC
TTAACTATGCGGCATCAGAGCAGATTGTACTGAGAGTGCACCAT
AAAATTGTAAACGTTAATATTTTGTTAAAATTCGCGTTAAATTTT
TGTTAAATCAGCTCATTTTTTAACCAATAGGCCGAAATCGGCAA
AATCCCTTATAAATCAAAAGAATAGCCCGAGATAGGGTTGAGTG
TTGTTCCAGTTTGGAACAAGAGTCCACTATTAAAGAACGTGGAC
TCCAACGTCAAAGGGCGAAAAACCGTCTATCAGGGCGATGGCC
CACTACGTGAACCATCACCCAAATCAAGTTTTTTGGGGTCGAGG
TGCCGTAAAGCACTAAATCGGAACCCTAAAGGGAGCCCCCGATT
TAGAGCTTGACGGGGAAAGCCGGCGAACGTGGCGAGAAAGGAA
GGGAAGAAAGCGAAAGGAGCGGGCGCTAGGGCGCTGGCAAGTG
TAGCGGTCACGCTGCGCGTAACCACCACACCCGCCGCGCTTAAT
GCGCCGCTACAGGGCGCGTACTATGGTTGCTTTGACGTATGCGG
TGTGAAATACCGCACAGATGCGTAAGGAGAAAATACCGCATCA
GGCGCCCCTGCAGGCAGCTGCGCGCTCGCTCGCTCACTGAGGCC
GCCCGGGCAAAGCCCGGGCGTCGGGCGACCTTTGGTCGCCCGGC
CTCAGTGAGCGAGCGAGCGCGCAGAGAGGGAGTGGCCAACTCC
ATCACTAGGGGTTCCTACGCGTCGTTACATAACTTACGGTAAAT
 
GGCCCGCCTGGCTGACCGCCCAACGACCCCCGCCCATTGACGTC
AATAATGACGTATGTTCCCATAGTAACGCCAATAGGGACTTTCC
ATTGACGTCAATGGGTGGAGTATTTACGGTAAACTGCCCACTTG
GCAGTACATCAAGTGTATCATATGCCAAGTACGCCCCCTATTGA
CGTCAATGACGGTAAATGGCCCGCCTGGCATTATGCCCAGTACA
TGACCTTATGGGACTTTCCTACTTGGCAGTACATCTACGTATTAG
TCATCGCTATTACCATGGTCGAGGTGAGCCCCACGTTCTGCTTC
ACTCTCCCCATCTCCCCCCCCTCCCCACCCCCAATTTTGTATTTA
TTTATTTTTTAATTATTTTGTGCAGCGATGGGGGCGGGGGGGGG
GGGGGGGCGCGCGCCAGGCGGGGCGGGGCGGGGCGAGGGGCG
GGGCGGGGCGAGGCGGAGAGGTGCGGCGGCAGCCAATCAGAGC
GGCGCGCTCCGAAAGTTTCCTTTTATGGCGAGGCGGCGGCGGCG
GCGGCCCTATAAAAAGCGAAGCGCGCGGCGGGCGGGAGTCGCT
GCGACGCTGCCTTCGCCCCGTGCCCCGCTCCGCCGCCGCCTCGC
GCCGCCCGCCCCGGCTCTGACTGACCGCGTTACTCCCACAGGTG
AGCGGGCGGGACGGCCCTTCTCCTCCGGGCTGTAATTAGCTGAG
CAAGAGGTAAGGGTTTAAGGGATGGTTGGTTGGTGGGGTATTAA
TGTTTAATTACCTGGAGCACCTGCCTGAAATCACTTTTTTTCAGG
TACCGGTCGCCACCATGGCGAGACCCCTGTGCACATTACTTCTG
TTGATGGCTACCCTGGCAGGCGCCCTCGCCAGCGAGCTGACACA
GGACCCTGCCGTGTCCGTGGCCCTGAAGCAGACCGTGACAATCA
CCTGCAGAGGCGATTCCCTGAGATCCCACTACGCCTCCTGGTAC
CAGAAGAAGCCTGGCCAGGCCCCCGTGCTGCTGTTTTACGGCAA
GAATAACCGCCCCAGCGGCATCCCCGATAGATTTTCCGGCAGCG
CCTCCGGCAACAGAGCCAGCCTGACAATCACCGGCGCCCAGGC
CGAGGACGAGGCTGATTACTACTGCAGCTCCAGAGATAAGAGC
GGCAGCAGACTGTCCGTGTTTGGCGGCGGCACCAAGCTGACCGT
GCTCGGAGGAGGAGGAAGCGGAGGAGGAGGCTCAGGCGGCGG
CGGCTCTGAGGTGAGGCTGAGAGAGTCCGGCGGCGGCCTGGTG
AAGCCCGGAGGATCTCTGAGGCTGTCCTGCTCCGCCTCCGGCTT
CGATTTTGACAATGCCTGGATGACCTGGGTGAGACAGCCCCCTG
GCAAGGGCCTGGAGTGGGTGGGAAGGATCACAGGCCCCGGCGA
GGGCTGGTCCGTGGATTACGCCGAGTCCGTGAAGGGCAGGTTCA
CAATCTCCAGGGATAACACCAAGAACACCCTGTACCTGGAGATG
AACAACGTGAGGACAGAGGATACCGGCTACTACTTTTGCGCCAG
AACAGGCAAGTACTACGACTTTTGGTTCGGCTACCCCCCTGGCG
AGGAGTACTTCCAGGATTGGGGCCAGGGCACCCTGGTCATTGTG
TCCAGCGGCGGCGGCGGCAGTGGCGGCGGCGGAAGCGGCGGCG
GCGGCTCTGGCGGCGGCGGCAGCGGCGGCGGCGGCTCCGACAT
CGTGATGACCCAGTCTCCTGATAGCCTGGCCGTGAGCCTGGGCG
 
AGAGAGTGACAATGAACTGTAAGTCTAGCCAGAGCCTGCTGTAC
TCCACCAACCAGAAGAATTACCTGGCCTGGTATCAGCAGAAGCC
TGGCCAGTCCCCAAAGCTGCTGATCTATTGGGCATCTACAAGGG
AGAGCGGAGTGCCAGACAGATTCAGCGGATCCGGATCTGGAAC
CGACTTCACCCTGACAATCTCCTCTGTGCAGGCCGAGGACGTGG
CCGTGTACTATTGCCAGCAGTACTATAGCTACAGGACATTCGGC
GGCGGCACCAAGCTGGAGATCAAGCGCACCGTGGCCGGAGGAG
GAGGATCTGGCGGAGGAGGGTCCGGCGGCGGCGGCTCCCAGGT
GCAGCTGCAGCAGAGCGGACCAGAGGTGGTGAAGCCTGGAGCC
TCCGTGAAGATGTCTTGTAAGGCCAGCGGCTACACCTTCACATC
CTATGTGATCCACTGGGTGAGGCAGAAGCCAGGACAGGGACTG
GACTGGATCGGCTACATCAACCCTTATAATGATGGCACCGACTA
CGATGAGAAGTTTAAGGGCAAGGCCACCCTGACATCCGATACC
AGCACATCCACCGCCTATATGGAGCTGAGCTCCCTGCGGTCTGA
GGACACAGCCGTGTACTATTGCGCCAGAGAGAAGGATAACTAC
GCAACCGGAGCATGGTTCGCATATTGGGGACAGGGTACCCTGGT
CACCGTGTCTAGCGAGCCCAAGAGCTGTGACAAGACACACACCT
GCCCTCCATGTCCAGCACCAGAGCTGCTGGGAGGGCCCTCCGTG
TTCCTGTTTCCCCCTAAGCCAAAGGATACACTGATGATCTCCAG
GACACCAGAGGTGACCTGCGTGGTGGTGGACGTGTCTCACGAG
GATCCTGAGGTGAAGTTTAACTGGTACGTGGACGGCGTGGAGGT
GCACAATGCCAAGACCAAGCCTAGGGAGGAGCAGTACAATTCT
ACATATCGCGTGGTGAGCGTGCTGACCGTGCTGCACCAGGATTG
GCTGAACGGCAAGGAGTATAAGTGCAAGGTGAGCAATAAGGCC
CTGCCTGCCCCAATCGAGAAGACAATCTCCAAGGCAAAGGGAC
AGCCAAGGGAGCCTCAGGTGTACACCCTGCCACCCTCTCGCGAC
GAGCTGACAAAGAACCAGGTGAGCCTGACCTGTCTGGTGAAGG
GCTTCTATCCTAGCGATATTGCCGTGGAGTGGGAGTCCAATGGC
CAGCCAGAGAACAATTACAAGACCACACCTCCAGTGCTGGACTC
CGATGGCTCTTTCTTTCTGTATTCCAAGCTGACAGTGGACAAGT
CTAGATGGCAGCAGGGCAACGTGTTTTCTTGTAGCGTGATGCAC
GAGGCCCTGCACAATCACTACACCCAGAAGTCCCTGTCTCTGAG
CCCCGGCAAGGGCGGAGGAGGAAGCGGAGGAGGCGGCTCTGGC
GGAGGAGGTTCCGGAGGAGGCGGAAGCGGCGGAGGAGGCTCTC
TGCTGGAGCAGGAGAACAAGGAGCAGCAGAATCAGAGCGAGGA
GATCCTGTCCCACATCCTGTCCACATACAACAATATCGAGAGAG
ATTGGGAGATGTGGTAGGTCGACAATCAACCTCTGGATTACAAA
ATTTGTGAAAGATTGACTGGTATTCTTAACTATGTTGCTCCTTTT
ACGCTATGTGGATACGCTGCTTTAATGCCTTTGTATCATGCTATT
GCTTCCCGTATGGCTTTCATTTTCTCCTCCTTGTATAAATCCTGG
 
TTGCTGTCTCTTTATGAGGAGTTGTGGCCCGTTGTCAGGCAACG
TGGCGTGGTGTGCACTGTGTTTGCTGACGCAACCCCCACTGGTT
GGGGCATTGCCACCACCTGTCAGCTCCTTTCCGGGACTTTCGCTT
TCCCCCTCCCTATTGCCACGGCGGAACTCATCGCCGCCTGCCTT
GCCCGCTGCTGGACAGGGGCTCGGCTGTTGGGCACTGACAATTC
CGTGGTGTTGTCGGGGAAGCTGACGTCCTTTCCATGGCTGCTCG
CCTGTGTTGCCACCTGGATTCTGCGCGGGACGTCCTTCTGCTAC
GTCCCTTCGGCCCTCAATCCAGCGGACCTTCCTTCCCGCGGCCT
GCTGCCGGCTCTGCGGCCTCTTCCGCGTCTTCGCCTTCGCCCTCA
GACGAGTCGGATCTCCCTTTGGGCCGCCTCCCCGCGAATTCGTT
TATTTGTGAAATTTGTGATGCTATTGCTTTATTTGTAACCATCTA
GCTTTATTTGTGAAATTTGTGATGCTATTGCTTTATTTGTAACCA
TTATAAGCTGCAATAAACAAGTTAACAACAACAATTGCATTCAT
TTTATGTTTCAGGTTCAGGGGGAGATGTGGGAGGTTTTTTAAAG
TTTAAACAGGAACCCCTAGTGATGGAGTTGGCCACTCCCTCTCT
GCGCGCTCGCTCGCTCACTGAGGCCGGGCGACCAAAGGTCGCCC
GACGCCCGGGCGGCCTCAGTGAGCGAGCGAGCGCGCAGCTGCC
TGCAGG
   SEQ ID NO: 24 
ACATGTGAGCAAAAGGCCAGCAAAAGGCCAGGAACCGTAAAAA
GGCCGCGTTGCTGGCGTTTTTCCATAGGCTCCGCCCCCCTGACG
AGCATCACAAAAATCGACGCTCAAGTCAGAGGTGGCGAAACCC
GACAGGACTATAAAGATACCAGGCGTTTCCCCCTGGAAGCTCCC
TCGTGCGCTCTCCTGTTCCGACCCTGCCGCTTACCGGATACCTGT
CCGCCTTTCTCCCTTCGGGAAGCGTGGCGCTTTCTCATAGCTCAC
GCTGTAGGTATCTCAGTTCGGTGTAGGTCGTTCGCTCCAAGCTG
GGCTGTGTGCACGAACCCCCCGTTCAGCCCGACCGCTGCGCCTT
ATCCGGTAACTATCGTCTTGAGTCCAACCCGGTAAGACACGACT
TATCGCCACTGGCAGCAGCCACTGGTAACAGGATTAGCAGAGC
GAGGTATGTAGGCGGTGCTACAGAGTTCTTGAAGTGGTGGCCTA
ACTACGGCTACACTAGAAGGACAGTATTTGGTATCTGCGCTCTG
CTGAAGCCAGTTACCTTCGGAAAAAGAGTTGGTAGCTCTTGATC
CGGCAAACAAACCACCGCTGGTAGCGGTGGTTTTTTTGTTTGCA
AGCAGCAGATTACGCGCAGAAAAAAAGGATCTCAAGAAGATCC
TTTGATCTTTTCTACGGGGTCTGACGCTCAGTGGAACGAAAACT
CACGTTAAGGGATTTTGGTCATGAGATTATCAAAAAGGATCTTC
ACCTAGATCCTTTTAAATTAAAAATGAAGTTTTAAATCAATCTA
AAGTATATATGAGTAAACTTGGTCTGACAGTTACCAATGCTTAA
TCAGTGAGGCACCTATCTCAGCGATCTGTCTATTTCGTTCATCCA
TAGTTGCCTGACTCCCCGTCGTGTAGATAACTACGATACGGGAG
 
GGCTTACCATCTGGCCCCAGTGCTGCAATGATACCGCGAGACCC
ACGCTCACCGGCTCCAGATTTATCAGCAATAAACCAGCCAGCCG
GAAGGGCCGAGCGCAGAAGTGGTCCTGCAACTTTATCCGCCTCC
ATCCAGTCTATTAATTGTTGCCGGGAAGCTAGAGTAAGTAGTTC
GCCAGTTAATAGTTTGCGCAACGTTGTTGCCATTGCTACAGGCA
TCGTGGTGTCACGCTCGTCGTTTGGTATGGCTTCATTCAGCTCCG
GTTCCCAACGATCAAGGCGAGTTACATGATCCCCCATGTTGTGC
AAAAAAGCGGTTAGCTCCTTCGGTCCTCCGATCGTTGTCAGAAG
TAAGTTGGCCGCAGTGTTATCACTCATGGTTATGGCAGCACTGC
ATAATTCTCTTACTGTCATGCCATCCGTAAGATGCTTTTCTGTGA
CTGGTGAGTACTCAACCAAGTCATTCTGAGAATAGTGTATGCGG
CGACCGAGTTGCTCTTGCCCGGCGTCAATACGGGATAATACCGC
GCCACATAGCAGAACTTTAAAAGTGCTCATCATTGGAAAACGTT
CTTCGGGGCGAAAACTCTCAAGGATCTTACCGCTGTTGAGATCC
AGTTCGATGTAACCCACTCGTGCACCCAACTGATCTTCAGCATC
TTTTACTTTCACCAGCGTTTCTGGGTGAGCAAAAACAGGAAGGC
AAAATGCCGCAAAAAAGGGAATAAGGGCGACACGGAAATGTTG
AATACTCATACTCTTCCTTTTTCAATATTATTGAAGCATTTATCA
GGGTTATTGTCTCATGAGCGGATACATATTTGAATGTATTTAGA
AAAATAAACAAATAGGGGTTCCGCGCACATTTCCCCGAAAAGT
GCCACCTGACGTCTAAGAAACCATTATTATCATGACATTAACCT
ATAAAAATAGGCGTATCACGAGGCCCTTTCGTCTCGCGCGTTTC
GGTGATGACGGTGAAAACCTCTGACACATGCAGCTCCCGGAGA
CGGTCACAGCTTGTCTGTAAGCGGATGCCGGGAGCAGACAAGC
CCGTCAGGGCGCGTCAGCGGGTGTTGGCGGGTGTCGGGGCTGGC
TTAACTATGCGGCATCAGAGCAGATTGTACTGAGAGTGCACCAT
AAAATTGTAAACGTTAATATTTTGTTAAAATTCGCGTTAAATTTT
TGTTAAATCAGCTCATTTTTTAACCAATAGGCCGAAATCGGCAA
AATCCCTTATAAATCAAAAGAATAGCCCGAGATAGGGTTGAGTG
TTGTTCCAGTTTGGAACAAGAGTCCACTATTAAAGAACGTGGAC
TCCAACGTCAAAGGGCGAAAAACCGTCTATCAGGGCGATGGCC
CACTACGTGAACCATCACCCAAATCAAGTTTTTTGGGGTCGAGG
TGCCGTAAAGCACTAAATCGGAACCCTAAAGGGAGCCCCCGATT
TAGAGCTTGACGGGGAAAGCCGGCGAACGTGGCGAGAAAGGAA
GGGAAGAAAGCGAAAGGAGCGGGCGCTAGGGCGCTGGCAAGTG
TAGCGGTCACGCTGCGCGTAACCACCACACCCGCCGCGCTTAAT
GCGCCGCTACAGGGCGCGTACTATGGTTGCTTTGACGTATGCGG
TGTGAAATACCGCACAGATGCGTAAGGAGAAAATACCGCATCA
GGCGCCCCTGCAGGCAGCTGCGCGCTCGCTCGCTCACTGAGGCC
GCCCGGGCAAAGCCCGGGCGTCGGGCGACCTTTGGTCGCCCGGC
 
CTCAGTGAGCGAGCGAGCGCGCAGAGAGGGAGTGGCCAACTCC
ATCACTAGGGGTTCCTACGCGTCGTTACATAACTTACGGTAAAT
GGCCCGCCTGGCTGACCGCCCAACGACCCCCGCCCATTGACGTC
AATAATGACGTATGTTCCCATAGTAACGCCAATAGGGACTTTCC
ATTGACGTCAATGGGTGGAGTATTTACGGTAAACTGCCCACTTG
GCAGTACATCAAGTGTATCATATGCCAAGTACGCCCCCTATTGA
CGTCAATGACGGTAAATGGCCCGCCTGGCATTATGCCCAGTACA
TGACCTTATGGGACTTTCCTACTTGGCAGTACATCTACGTATTAG
TCATCGCTATTACCATGGTCGAGGTGAGCCCCACGTTCTGCTTC
ACTCTCCCCATCTCCCCCCCCTCCCCACCCCCAATTTTGTATTTA
TTTATTTTTTAATTATTTTGTGCAGCGATGGGGGCGGGGGGGG
GGGGGGGGCGCGCGCCAGGCGGGGCGGGGCGGGGCGAGGGGCG
GGGCGGGGCGAGGCGGAGAGGTGCGGCGGCAGCCAATCAGAGC
GGCGCGCTCCGAAAGTTTCCTTTTATGGCGAGGCGGCGGCGG
GCGGCCCTATAAAAAGCGAAGCGCGCGGCGGGCGGGAGTCGCT
GCGACGCTGCCTTCGCCCCGTGCCCCGCTCCGCCGCCGCCTCGC
GCCGCCCGCCCCGGCTCTGACTGACCGCGTTACTCCCACAGGTG
AGCGGGCGGGACGGCCCTTCTCCTCCGGGCTGTAATTAGCTGAG
CAAGAGGTAAGGGTTTAAGGGATGGTTGGTTGGTGGGGTATTAA
TGTTTAATTACCTGGAGCACCTGCCTGAAATCACTTTTTTTCAGG
TACCGGTCGCCACCATGGCGAGACCCCTGTGCACATTACTTCTG
TTGATGGCTACCCTGGCAGGCGCCCTCGCCAGCGAGCTGACACA
GGACCCTGCCGTGTCCGTGGCCCTGAAGCAGACCGTGACAATCA
CCTGCAGAGGCGATTCCCTGAGATCCCACTACGCCTCCTGGTAC
CAGAAGAAGCCTGGCCAGGCCCCCGTGCTGCTGTTTTACGGCAA
GAATAACCGCCCCAGCGGCATCCCCGATAGATTTTCCGGCAGCG
CCTCCGGCAACAGAGCCAGCCTGACAATCACCGGCGCCCAGGC
CGAGGACGAGGCTGATTACTACTGCAGCTCCAGAGATAAGAGC
GGCAGCAGACTGTCCGTGTTTGGCGGCGGCACCAAGCTGACCGT
GCTCGGAGGAGGAGGAAGCGGAGGAGGAGGCTCAGGCGGCGG
CGGCTCTGAGGTGAGGCTGAGAGAGTCCGGCGGCGGCCTGGTG
AAGCCCGGAGGATCTCTGAGGCTGTCCTGCTCCGCCTCCGGCTT
CGATTTTGACAATGCCTGGATGACCTGGGTGAGACAGCCCCCTG
GCAAGGGCCTGGAGTGGGTGGGAAGGATCACAGGCCCCGGCGA
GGGCTGGTCCGTGGATTACGCCGAGTCCGTGAAGGGCAGGTTCA
CAATCTCCAGGGATAACACCAAGAACACCCTGTACCTGGAGATG
AACAACGTGAGGACAGAGGATACCGGCTACTACTTTTGCGCCAG
AACAGGCAAGTACTACGACTTTTGGTTCGGCTACCCCCCTGGCG
AGGAGTACTTCCAGGATTGGGGCCAGGGCACCCTGGTCATTGTG
TCCAGCGGCGGCGGCGGCAGTGGCGGCGGCGGAAGCGGCGGCG
 
GCGGCTCTGGCGGCGGCGGCAGCGGCGGCGGCGGCTCCGACAT
CGTGATGACCCAGTCTCCTGATAGCCTGGCCGTGAGCCTGGGCG
AGAGAGTGACAATGAACTGTAAGTCTAGCCAGAGCCTGCTGTAC
TCCACCAACCAGAAGAATTACCTGGCCTGGTATCAGCAGAAGCC
TGGCCAGTCCCCAAAGCTGCTGATCTATTGGGCATCTACAAGGG
AGAGCGGAGTGCCAGACAGATTCAGCGGATCCGGATCTGGAAC
CGACTTCACCCTGACAATCTCCTCTGTGCAGGCCGAGGACGTGG
CCGTGTACTATTGCCAGCAGTACTATAGCTACAGGACATTCGGC
GGCGGCACCAAGCTGGAGATCAAGCGCACCGTGGCCGGAGGAG
GAGGATCTGGCGGAGGAGGGTCCGGCGGCGGCGGCTCCCAGGT
GCAGCTGCAGCAGAGCGGACCAGAGGTGGTGAAGCCTGGAGCC
TCCGTGAAGATGTCTTGTAAGGCCAGCGGCTACACCTTCACATC
CTATGTGATCCACTGGGTGAGGCAGAAGCCAGGACAGGGACTG
GACTGGATCGGCTACATCAACCCTTATAATGATGGCACCGACTA
CGATGAGAAGTTTAAGGGCAAGGCCACCCTGACATCCGATACC
AGCACATCCACCGCCTATATGGAGCTGAGCTCCCTGCGGTCTGA
GGACACAGCCGTGTACTATTGCGCCAGAGAGAAGGATAACTAC
GCAACCGGAGCATGGTTCGCATATTGGGGACAGGGTACCCTGGT
CACCGTGTCTAGCGAGCCCAAGAGCTGTGACAAGACACACACCT
GCCCTCCATGTCCAGCACCAGAGCTGCTGGGAGGGCCCTCCGTG
TTCCTGTTTCCCCCTAAGCCAAAGGATACACTGATGATCTCCAG
GACACCAGAGGTGACCTGCGTGGTGGTGGACGTGTCTCACGAG
GATCCTGAGGTGAAGTTTAACTGGTACGTGGACGGCGTGGAGGT
GCACAATGCCAAGACCAAGCCTAGGGAGGAGCAGTACAATTCT
ACATATCGCGTGGTGAGCGTGCTGACCGTGCTGCACCAGGATTG
GCTGAACGGCAAGGAGTATAAGTGCAAGGTGAGCAATAAGGCC
CTGCCTGCCCCAATCGAGAAGACAATCTCCAAGGCAAAGGGAC
AGCCAAGGGAGCCTCAGGTGTACACCCTGCCACCCTCTCGCGAC
GAGCTGACAAAGAACCAGGTGAGCCTGACCTGTCTGGTGAAGG
GCTTCTATCCTAGCGATATTGCCGTGGAGTGGGAGTCCAATGGC
CAGCCAGAGAACAATTACAAGACCACACCTCCAGTGCTGGACTC
CGATGGCTCTTTCTTTCTGTATTCCAAGCTGACAGTGGACAAGT
CTAGATGGCAGCAGGGCAACGTGTTTTCTTGTAGCGTGATGCAC
GAGGCCCTGCACAATCACTACACCCAGAAGTCCCTGTCTCTGAG
CCCCGGCAAGGGCGGAGGAGGAAGCGGAGGAGGCGGCTCTGGC
GGAGGAGGTTCCGGAGGAGGCGGAAGCGGCGGAGGAGGCTCTT
ACACCTCCCTGATCCACTCCCTGATCGAGGAGTCCCAGAATCAG
CAGGAGAAGAATGAGCAGGAGCTGCTGGAGCTGGATAAGTGGG
CCTCCCTGTGGAATTGGTTCTAGGTCGACAATCAACCTCTGGAT
TACAAAATTTGTGAAAGATTGACTGGTATTCTTAACTATGTTGC
 
TCCTTTTACGCTATGTGGATACGCTGCTTTAATGCCTTTGTATCA
TGCTATTGCTTCCCGTATGGCTTTCATTTTCTCCTCCTTGTATAA
ATCCTGGTTGCTGTCTCTTTATGAGGAGTTGTGGCCCGTTGTCAG
GCAACGTGGCGTGGTGTGCACTGTGTTTGCTGACGCAACCCCCA
CTGGTTGGGGCATTGCCACCACCTGTCAGCTCCTTTCCGGGACT
TTCGCTTTCCCCCTCCCTATTGCCACGGCGGAACTCATCGCCGCC
TGCCTTGCCCGCTGCTGGACAGGGGCTCGGCTGTTGGGCACTGA
CAATTCCGTGGTGTTGTCGGGGAAGCTGACGTCCTTTCCATGGC
TGCTCGCCTGTGTTGCCACCTGGATTCTGCGCGGGACGTCCTTCT
GCTACGTCCCTTCGGCCCTCAATCCAGCGGACCTTCCTTCCCGC
GGCCTGCTGCCGGCTCTGCGGCCTCTTCCGCGTCTTCGCCTTCGC
CCTCAGACGAGTCGGATCTCCCTTTGGGCCGCCTCCCCGCAAT
TCGTTTATTTGTGAAATTTGTGATGCTATTGCTTTATTTGTAACC
ATCTAGCTTTATTTGTGAAATTTGTGATGCTATTGCTTTATTTGT
AACCATTATAAGCTGCAATAAACAAGTTAACAACAACAATTGCA
TTCATTTTATGTTTCAGGTTCAGGGGGAGATGTGGGAGGTTTTTT
AAAGTTTAAACAGGAACCCCTAGTGATGGAGTTGGCCACTCCCT
CTCTGCGCGCTCGCTCGCTCACTGAGGCCGGGCGACCAAAGGTC
GCCCGACGCCCGGGCGGCCTCAGTGAGCGAGCGAGCGCGCAGC
TGCCTGCAGG
   SEQ ID NO: 25 
GGCCGCGTTGCTGGCGTTTTTCCATAGGCTCCGCCCCCCTGACGAGCAT
CACAAAAATCGACGCTCAAGTCAGAGGTGGCGAAACCCGACAGGACTA
TAAAGATACCAGGCGTTTCCCCCTGGAAGCTCCCTCGTGCGCTCTCCTG
TTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTA
AGACACGACTTATCGCCACTGGCAGCAGCCACTGGTAACAGGATTAGC
AGAGCGAGGTATGTAGGCGGTGCTACAGAGTTCTTGAAGTGGTGGCCT
AACTACGGCTACACTAGAAGAACAGTATTTGGTATCTGCGCTCTGCTGA
AGCCAGTTACCTTCGGAAAAAGAGTTGGTAGCTCTTGATCCGGCAAAC
AAACCACCGCTGGTAGCGGTGGTTTTTTTGTTTGCAAGCAGCAGATTAC
GCGCAGAAAAAAAGGATCTCAAGAAGATCCTTTGATCTTTTCTACGGG
GTCTGACGCTCAGTGGAACGAAAACTCACGTTAAGGGATTTTGGTCAT
GAAGCGCTTTTGAAGCTCGGATCCGAACAAACGACCCAACACCCGTGC
GTTTTATTCTGTCTTTTTATTGCCGATCCCCTCAGAAGAACTCGTCAAGA
AGGCGATAGAAGGCGATGCGCTGCGAATCGGGAGCGGCGATACCGTA
AAGCACGAGGAAGCGGTCAGCCCATTCGCCGCCAAGCTCTTCAGCAAT
ATCACGGGTAGCCAACGCTATGTCCTGATAGCGGTCCGCCACACCCAG
 
CCGGCCACAGTCGATGAATCCAGAAAAGCGGCCATTTTCCACCATGAT
ATTCGGCAAGCAGGCATCGCCATGGGTCACGACGAGATCCTCGCCGTC
GGGCATGCTCGCCTTGAGCCTGGCGAACAGTTCGGCTGGCGCGAGCCC
CTGATGCTCTTCGTCCAGATCATCCTGATCGACAAGACCGGCTTCCATC
CGAGTACGTGCTCGCTCGATGCGATGTTTCGCTTGGTGGTCGAATGGGC
AGGTAGCCGGATCAAGCGTATGCAGCCGCCGCATTGCATCAGCCATGA
TGGATACTTTCTCGGCAGGAGCAAGGTGAGATGACAGGAGATCCTGCC
CCGGCACTTCGCCCAATAGCAGCCAGTCCCTTCCCGCTTCAGTGACAAC
GTCGAGCACAGCTGCGCAAGGAACGCCCGTCGTGGCCAGCCACGATAG
CCGCGCTGCCTCGTCTTGCAGTTCATTCAGGGCACCGGACAGGTCGGTC
TTGACAAAAAGAACCGGGCGCCCCTGCGCTGACAGCCGGAACACGGCG
GCATCAGAGCAGCCGATTGTCTGTTGTGCCCAGTCATAGCCGAATAGCC
TCTCCACCCAAGCGGCCGGAGAACCTGCGTGCAATCCATCTTGTTCAAT
CATGCGAAACGATCCTCATCCTGTCTCTTGATCAGAGCTTGATCCCCTG
CGCCATCAGATCCTTGGCGGCAAGAAAGCCATCCAGTTTACTTTGCAGG
GCTTCCCAACCTTACCAGAGGCCTGCGCCGCGGCCAGCTGGCTAGCAA
TTCCCGGGTTAACTCTAGAGACATTGATTATTGACTAGTTATTAATAGT
AATCAATTACGGGGTCATTAGTTCATAGCCCATATATGGAGTTCCGCGT
TACATAACTTACGGTAAATGGCCCGCCTGGCTGACCGCCCAACGACCC
CCGCCCATTGACGTCAATAATGACGTATGTTCCCATAGTAACGCCAATA
GGGACTTTCCATTGACGTCAATGGGTGGAGTATTTACGGTAAACTGCCC
ACTTGGCAGTACATCAAGTGTATCATATGCCAAGTACGCCCCCTATTGA
CGTCAATGACGGTAAATGGCCCGCCTGGCATTATGCCCAGTACATGAC
CTTATGGGACTTTCCTACTTGGCAGTACATCTACGTATTAGTCATCGCT
ATTACCATGGTGATGCGGTTTTGGCAGTACATCAATGGGCGTGGATAGC
GGTTTGACTCACGGGGATTTCCAAGTCTCCACCCCATTGACGTCAATGG
GAGTTTGTTTTGGCACCAAAATCAACGGGACTTTCCAAAATGTCGTAAC
AACTCCGCCCCATTGACGCAAATGGGCGGTAGGCGTGTACGGTGGGAG
GTCTATATAAGCAGAGCTCGTTTAGTGAACCGTCAGATCGCCTGGAGA
CGCCATCCACGCTGTTTTGACCTCCATAGAAGACACCGGGACCGATCCA
GCCTCCCCTCGAAGCTTACATGTGGTACCGAGCTCGGATCCTGAGAACT
TCAGGGTGAGTCTATGGGACCCTTGATGTTTTCTTTCCCCTTCTTTTCTA
TGGTTAAGTTCATGTCATAGGAAGGGGAGAAGTAACAGGGTACACATA
TTGACCAAATCAGGGTAATTTTGCATTTGTAATTTTAAAAAATGCTTTC
TTCTTTTAATATACTTTTTTGTTTATCTTATTTCTAATACTTTCCCTAATC
TCTTTCTTTCAGGGCAATAATGATACAATGTATCATGCCTCTTTGCACC
ATTCTAAAGAATAACAGTGATAATTTCTGGGTTAAGGCAATAGCAATA
TTTCTGCATATAAATATTTCTGCATATAAATTGTAACTGATGTAAGAGG
TTTCATATTGCTAATAGCAGCTACAATCCAGCTACCATTCTGCTTTTATT
TTATGGTTGGGATAAGGCTGGATTATTCTGAGTCCAAGCTAGGCCCTTT
 
TGCTAATCATGTTCATACCTCTTATCTTCCTCCCACAGCTCCTGGGCAAC
GTGCTGGTCTGTGTGCTGGCCCATCACTTTGGCAAAGCACGTGAGATCT
GAATTCTGACACTATGAAGTGCCTTTTGTACTTAGCCTTTTTATTCATTG
GGGTGAATTGCAAGTTCACCATAGTTTTTCCACACAACCAAAAAGGAA
ACTGGAAAAATGTTCCTTCTAATTACCATTATTGCCCGTCAAGCTCAGA
TTTAAATTGGCATAATGACTTAATAGGCACAGCCTTACAAGTCAAAATG
CCCAAGAGTCACAAGGCTATTCAAGCAGACGGTTGGATGTGTCATGCT
TCCAAATGGGTCACTACTTGTGATTTCCGCTGGTATGGACCGAAGTATA
TAACACATTCCATCCGATCCTTCACTCCATCTGTAGAACAATGCAAGGA
AAGCATTGAACAAACGAAACAAGGAACTTGGCTGAATCCAGGCTTCCC
TCCTCAAAGTTGTGGATATGCAACTGTGACGGATGCCGAAGCAGTGAT
TGTCCAGGTGACTCCTCACCATGTGCTGGTTGATGAATACACAGGAGA
ATGGGTTGATTCACAGTTCATCAACGGAAAATGCAGCAATTACATATG
CCCCACTGTCCATAACTCTACAACCTGGCATTCTGACTATAAGGTCAAA
GGGCTATGTGATTCTAACCTCATTTCCATGGACATCACCTTCTTCTCAG
AGGACGGAGAGCTATCATCCCTGGGAAAGGAGGGCACAGGGTTCAGA
AGTAACTACTTTGCTTATGAAACTGGAGGCAAGGCCTGCAAAATGCAA
TACTGCAAGCATTGGGGAGTCAGACTCCCATCAGGTGTCTGGTTCGAG
ATGGCTGATAAGGATCTCTTTGCTGCAGCCAGATTCCCTGAATGCCCAG
AAGGGTCAAGTATCTCTGCTCCATCTCAGACCTCAGTGGATGTAAGTCT
AATTCAGGACGTTGAGAGGATCTTGGATTATTCCCTCTGCCAAGAAACC
TGGAGCAAAATCAGAGCGGGTCTTCCAATCTCTCCAGTGGATCTCAGCT
ATCTTGCTCCTAAAAACCCAGGAACCGGTCCTGCTTTCACCATAATCAA
TGGTACCCTAAAATACTTTGAGACCAGATACATCAGAGTCGATATTGCT
GCTCCAATCCTCTCAAGAATGGTCGGAATGATCAGTGGAACTACCACA
GAAAGGGAACTGTGGGATGACTGGGCACCATATGAAGACGTGGAAATT
GGACCCAATGGAGTTCTGAGGACCAGTTCAGGATATAAGTTTCCTTTAT
ACATGATTGGACATGGTATGTTGGACTCCGATCTTCATCTTAGCTCAAA
GGCTCAGGTGTTCGAACATCCTCACATTCAAGACGCTGCTTCGCAACTT
CCTGATGATGAGAGTTTATTTTTTGGTGATACTGGGCTATCCAAAAATC
CAATCGAGCTTGTAGAAGGTTGGTTCAGTAGTTGGAAAAGCTCTATTGC
CTCTTTTTTCTTTATCATAGGGTTAATCATTGGACTATTCTTGGTTCTCC
GAGTTGGTATCCATCTTTGCATTAAATTAAAGCACACCAAGAAAAGAC
AGATTTATACAGACATAGAGATGAACCGACTTGGAAAGTAACTCAAAT
CCTGCACAACAGATTCTTCATGTTTGGACCAAATCAACTTGTGATACCA
TGCTCAAAGAGGCCTCAATTATATTTGAGTTTTTAATTTTTATGAAAAA
AAAAAAAAAAAACGGAATTCACCCCACCAGTGCAGGCTGCCTATCAGA
AAGTGGTGGCTGGTGTGGCTAATGCCCTGGCCCACAAGTATCACTAAG
CTCGCTTTCTTGCTGTCCAATTTCTATTAAAGGTTCCTTTGTTCCCTAAG
TCCAACTACTAAACTGGGGGATATTATGAAGGGCCTTGAGCATCTGGA
 
TTCTGCCTAATAAAAAACATTTATTTTCATTGCAATGATGTATTTAAATT
ATTTCTGAATATTTTACTAAAAAGGGAATGTGGGAGGTCAGTGCATTTA
AAACATAAAGAAATGAAGAGCTAGTTCAAACCTTGGGAAAATACACTA
TATCTTAAACTCCATGAAAGAAGGTGAGGCTGCAAACAGCTAATGCAC
ATTGGCAACAGCCCCTGATGCCTATGCCTTATTCATCCCTCAGAAAAGG
ATTCAAGTAGAGGCTTGATTTGGAGGTTAAAGTTTTGCTATGCTGTATT
TTAGTCGACCATTACTTATTGTTTTAGCTGTCCTCATGAATGTCTTTTCA
CTACCCATTTGCTTATCCTGCATCTCTCAGCCTTGACTCCACTCAGTTCT
CTTGCTTAGAGATACCACCTTTCCCCTGAAGTGTTCCTTCCATGTTTTAC
GGCGAGATGGTTTCTCCTCGCCTGGCCACTCAGCCTTAGTTGTCTCTGTT
GTCTTATAGAGGTCTACTTGAAGAAGGAAAAACAGGGGGCATGGTTTG
ACTGTCCTGTGAGCCCTTCTTCCCTGCCTCCCCCACTCACAGTGACCCG
GAATCCCTCGACATGGCAGTCTAGCACTAGTGCGGCCGCAGATCTGCTT
CCTCGCTCACTGACTCGCTGCGCTCGGTCGTTCGGCTGCGGCGAGCGGT
ATCAGCTCACTCAAAGGCGGTAATACGGTTATCCACAGAATCAGGGGA
TAACGCAGGAAAGAACATGTGAGCAAAAGGCCAGCAAAAGGCCAGGA
ACCGTAAAAA
   SEQ ID NO: 26 
GGCCGCGTTGCTGGCGTTTTTCCATAGGCTCCGCCCCCCTGACGAGCAT
CACAAAAATCGACGCTCAAGTCAGAGGTGGCGAAACCCGACAGGACTA
TAAAGATACCAGGCGTTTCCCCCTGGAAGCTCCCTCGTGCGCTCTCCTG
TTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTA
AGACACGACTTATCGCCACTGGCAGCAGCCACTGGTAACAGGATTAGC
AGAGCGAGGTATGTAGGCGGTGCTACAGAGTTCTTGAAGTGGTGGCCT
AACTACGGCTACACTAGAAGAACAGTATTTGGTATCTGCGCTCTGCTGA
AGCCAGTTACCTTCGGAAAAAGAGTTGGTAGCTCTTGATCCGGCAAAC
AAACCACCGCTGGTAGCGGTGGTTTTTTTGTTTGCAAGCAGCAGATTAC
GCGCAGAAAAAAAGGATCTCAAGAAGATCCTTTGATCTTTTCTACGGG
GTCTGACGCTCAGTGGAACGAAAACTCACGTTAAGGGATTTTGGTCAT
GAAGCGCTTTTGAAGCTCGGATCCGAACAAACGACCCAACACCCGTGC
GTTTTATTCTGTCTTTTTATTGCCGATCCCCTCAGAAGAACTCGTCAAGA
AGGCGATAGAAGGCGATGCGCTGCGAATCGGGAGCGGCGATACCGTA
AAGCACGAGGAAGCGGTCAGCCCATTCGCCGCCAAGCTCTTCAGCAAT
ATCACGGGTAGCCAACGCTATGTCCTGATAGCGGTCCGCCACACCCAG
CCGGCCACAGTCGATGAATCCAGAAAAGCGGCCATTTTCCACCATGAT
ATTCGGCAAGCAGGCATCGCCATGGGTCACGACGAGATCCTCGCCGTC
GGGCATGCTCGCCTTGAGCCTGGCGAACAGTTCGGCTGGCGCGAGCCC
 
CTGATGCTCTTCGTCCAGATCATCCTGATCGACAAGACCGGCTTCCATC
CGAGTACGTGCTCGCTCGATGCGATGTTTCGCTTGGTGGTCGAATGGGC
AGGTAGCCGGATCAAGCGTATGCAGCCGCCGCATTGCATCAGCCATGA
TGGATACTTTCTCGGCAGGAGCAAGGTGAGATGACAGGAGATCCTGCC
CCGGCACTTCGCCCAATAGCAGCCAGTCCCTTCCCGCTTCAGTGACAAC
GTCGAGCACAGCTGCGCAAGGAACGCCCGTCGTGGCCAGCCACGATAG
CCGCGCTGCCTCGTCTTGCAGTTCATTCAGGGCACCGGACAGGTCGGTC
TTGACAAAAAGAACCGGGCGCCCCTGCGCTGACAGCCGGAACACGGCG
GCATCAGAGCAGCCGATTGTCTGTTGTGCCCAGTCATAGCCGAATAGCC
TCTCCACCCAAGCGGCCGGAGAACCTGCGTGCAATCCATCTTGTTCAAT
CATGCGAAACGATCCTCATCCTGTCTCTTGATCAGAGCTTGATCCCCTG
CGCCATCAGATCCTTGGCGGCAAGAAAGCCATCCAGTTTACTTTGCAGG
GCTTCCCAACCTTACCAGAGGCCTGCGCCGCGGCCAGCTGGCTAGCAA
TTCCCGGGTTAACTCTAGAGAATGTAGTCTTATGCAATACTCTTGTAGT
CTTGCAACATGGTAACGATGAGTTAGCAACATGCCTTACAAGGAGAGA
AAAAGCACCGTGCATGCCGATTGGTGGAAGTAAGGTGGTACGATCGTG
CCTTATTAGGAAGGCAACAGACGGGTCTGACATGGATTGGACGAACCA
CTGAATTCCGCATTGCAGAGATATTGTATTTAAGTGCCTAGCTCGATAC
AATAAACGCCATTTGACCATTCACCACATTGGTGTGCACCTCCAAGCTC
GAGCTCGTTTAGTGAACCGTCAGATCGCCTGGAGACGCCATCCACGCT
GTTTTGACCTCCATAGAAGACACCGGGACCGATCCAGCCTCCCCTCGAA
GCTAGTCGATTAGGCATCTCCTATGGCAGGAAGAAGCGGAGACAGCGA
CGAAGACCTCCTCAAGGCAGTCAGACTCATCAAGTTTCTCTATCAAAGC
AACCCACCTCCCAATCCCGAGGGGACCCGACAGGCCCGAAGGAATAGA
AGAAGAAGGTGGAGAGAGAGACAGAGACAGATCCATTCGATTAGTGA
ACGGATCCTTAGCACTTATCTGGGACGATCTGCGGAGCCTGTGCCTCTT
CAGCTACCACCGCTTGAGAGACTTACTCTTGATTGTAACGAGGATTGTG
GAACTTCTGGGACGCAGGGGGTGGGAAGCCCTCAAATATTGGTGGAAT
CTCCTACAATATTGGAGTCAGGAGCTAAAGAATAGTGCTGTTAGCTTGC
TCAATGCCACAGCTATAGCAGTAGCTGAGGGGACAGATAGGGTTATAG
AAGTAGTACAAGAAGCTTGGCACTGGCCGTCGTTTTACAACGTCGTGAT
CTGAGCCTGGGAGATCTCTGGCTAACTAGGGAACCCACTGCTTAAGCCT
CAATAAAGCTTGCCTTGAGTGCTTCAAGTAGTGTGTGCCCGTCTGTTGT
GTGACTCTGGTAACTAGAGATCAGGAAAACCCTGGCGTTACCCAACTT
AATCGCCTTGCAGCACATCCCCCTTTCGCCAGCTGGCGTAATAGCGAAG
AGGCCCGCACCGATCGCCCTTCCCAACAGTTGCGCAGCCTGAATGGCG
AATGGCGCCTGATGCGGTATTTTCTCCTTACGCATCTGTGCGGTATTTC
ACACCGCATACGTCAAAGCAACCATAGTGTCGACCATTACTTATTGTTT
TAGCTGTCCTCATGAATGTCTTTTCACTACCCATTTGCTTATCCTGCATC
TCTCAGCCTTGACTCCACTCAGTTCTCTTGCTTAGAGATACCACCTTTCC
 
CCTGAAGTGTTCCTTCCATGTTTTACGGCGAGATGGTTTCTCCTCGCCTG
GCCACTCAGCCTTAGTTGTCTCTGTTGTCTTATAGAGGTCTACTTGAAG
AAGGAAAAACAGGGGGCATGGTTTGACTGTCCTGTGAGCCCTTCTTCCC
TGCCTCCCCCACTCACAGTGACCCGGAATCCCTCGACATGGCAGTCTAG
CACTAGTGCGGCCGCAGATCTGCTTCCTCGCTCACTGACTCGCTGCGCT
CGGTCGTTCGGCTGCGGCGAGCGGTATCAGCTCACTCAAAGGCGGTAA
TACGGTTATCCACAGAATCAGGGGATAACGCAGGAAAGAACATGTGAG
CAAAAGGCCAGCAAAAGGCCAGGAACCGTAAAAA
   SEQ ID NO: 27 
GGCCGCGTTGCTGGCGTTTTTCCATAGGCTCCGCCCCCCTGACGAGCAT
CACAAAAATCGACGCTCAAGTCAGAGGTGGCGAAACCCGACAGGACTA
TAAAGATACCAGGCGTTTCCCCCTGGAAGCTCCCTCGTGCGCTCTCCTG
TTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTA
AGACACGACTTATCGCCACTGGCAGCAGCCACTGGTAACAGGATTAGC
AGAGCGAGGTATGTAGGCGGTGCTACAGAGTTCTTGAAGTGGTGGCCT
AACTACGGCTACACTAGAAGAACAGTATTTGGTATCTGCGCTCTGCTGA
AGCCAGTTACCTTCGGAAAAAGAGTTGGTAGCTCTTGATCCGGCAAAC
AAACCACCGCTGGTAGCGGTGGTTTTTTTGTTTGCAAGCAGCAGATTAC
GCGCAGAAAAAAAGGATCTCAAGAAGATCCTTTGATCTTTTCTACGGG
GTCTGACGCTCAGTGGAACGAAAACTCACGTTAAGGGATTTTGGTCAT
GAAGCGCTTTTGAAGCTCGGATCCGAACAAACGACCCAACACCCGTGC
GTTTTATTCTGTCTTTTTATTGCCGATCCCCTCAGAAGAACTCGTCAAGA
AGGCGATAGAAGGCGATGCGCTGCGAATCGGGAGCGGCGATACCGTA
AAGCACGAGGAAGCGGTCAGCCCATTCGCCGCCAAGCTCTTCAGCAAT
ATCACGGGTAGCCAACGCTATGTCCTGATAGCGGTCCGCCACACCCAG
CCGGCCACAGTCGATGAATCCAGAAAAGCGGCCATTTTCCACCATGAT
ATTCGGCAAGCAGGCATCGCCATGGGTCACGACGAGATCCTCGCCGTC
GGGCATGCTCGCCTTGAGCCTGGCGAACAGTTCGGCTGGCGCGAGCCC
CTGATGCTCTTCGTCCAGATCATCCTGATCGACAAGACCGGCTTCCATC
CGAGTACGTGCTCGCTCGATGCGATGTTTCGCTTGGTGGTCGAATGGGC
AGGTAGCCGGATCAAGCGTATGCAGCCGCCGCATTGCATCAGCCATGA
TGGATACTTTCTCGGCAGGAGCAAGGTGAGATGACAGGAGATCCTGCC
CCGGCACTTCGCCCAATAGCAGCCAGTCCCTTCCCGCTTCAGTGACAAC
GTCGAGCACAGCTGCGCAAGGAACGCCCGTCGTGGCCAGCCACGATAG
CCGCGCTGCCTCGTCTTGCAGTTCATTCAGGGCACCGGACAGGTCGGTC
TTGACAAAAAGAACCGGGCGCCCCTGCGCTGACAGCCGGAACACGGCG
GCATCAGAGCAGCCGATTGTCTGTTGTGCCCAGTCATAGCCGAATAGCC
 
TCTCCACCCAAGCGGCCGGAGAACCTGCGTGCAATCCATCTTGTTCAAT
CATGCGAAACGATCCTCATCCTGTCTCTTGATCAGAGCTTGATCCCCTG
CGCCATCAGATCCTTGGCGGCAAGAAAGCCATCCAGTTTACTTTGCAGG
GCTTCCCAACCTTACCAGAGGCCTGCGCCGCGGCCAGCTGGCTAGCAA
TTCCCGGGTTAACTCTAGAGACATTGATTATTGACTAGTTATTAATAGT
AATCAATTACGGGGTCATTAGTTCATAGCCCATATATGGAGTTCCGCGT
TACATAACTTACGGTAAATGGCCCGCCTGGCTGACCGCCCAACGACCC
CCGCCCATTGACGTCAATAATGACGTATGTTCCCATAGTAACGCCAATA
GGGACTTTCCATTGACGTCAATGGGTGGAGTATTTACGGTAAACTGCCC
ACTTGGCAGTACATCAAGTGTATCATATGCCAAGTACGCCCCCTATTGA
CGTCAATGACGGTAAATGGCCCGCCTGGCATTATGCCCAGTACATGAC
CTTATGGGACTTTCCTACTTGGCAGTACATCTACGTATTAGTCATCGCT
ATTACCATGGTGATGCGGTTTTGGCAGTACATCAATGGGCGTGGATAGC
GGTTTGACTCACGGGGATTTCCAAGTCTCCACCCCATTGACGTCAATGG
GAGTTTGTTTTGGCACCAAAATCAACGGGACTTTCCAAAATGTCGTAAC
AACTCCGCCCCATTGACGCAAATGGGCGGTAGGCGTGTACGGTGGGAG
GTCTATATAAGCAGAGCTCGTTTAGTGAACCGTCAGATCGCCTGGAGA
CGCCATCCACGCTGTTTTGACCTCCATAGAAGACACCGGGACCGATCCA
GCCTCCCCTCGAAGCTTACATGTGGTACCGAGCTCGGATCCTGAGAACT
TCAGGGTGAGTCTATGGGACCCTTGATGTTTTCTTTCCCCTTCTTTTCTA
TGGTTAAGTTCATGTCATAGGAAGGGGAGAAGTAACAGGGTACACATA
TTGACCAAATCAGGGTAATTTTGCATTTGTAATTTTAAAAAATGCTTTC
TTCTTTTAATATACTTTTTTGTTTATCTTATTTCTAATACTTTCCCTAATC
TCTTTCTTTCAGGGCAATAATGATACAATGTATCATGCCTCTTTGCACC
ATTCTAAAGAATAACAGTGATAATTTCTGGGTTAAGGCAATAGCAATA
TTTCTGCATATAAATATTTCTGCATATAAATTGTAACTGATGTAAGAGG
TTTCATATTGCTAATAGCAGCTACAATCCAGCTACCATTCTGCTTTTATT
TTATGGTTGGGATAAGGCTGGATTATTCTGAGTCCAAGCTAGGCCCTTT
TGCTAATCATGTTCATACCTCTTATCTTCCTCCCACAGCTCCTGGGCAAC
GTGCTGGTCTGTGTGCTGGCCCATCACTTTGGCAAAGCACGTGAGATCT
GAATTCGAGATCTGCCGCCGCCATGGGTGCGAGAGCGTCAGTATTAAG
CGGGGGAGAATTAGATCGATGGGAAAAAATTCGGTTAAGGCCAGGGG
GAAAGAAAAAATATAAATTAAAACATATAGTATGGGCAAGCAGGGAG
CTAGAACGATTCGCAGTTAATCCTGGCCTGTTAGAAACATCAGAAGGC
TGTAGACAAATACTGGGACAGCTACAACCATCCCTTCAGACAGGATCA
GAAGAACTTAGATCATTATATAATACAGTAGCAACCCTCTATTGTGTGC
ATCAAAGGATAGAGATAAAAGACACCAAGGAAGCTTTAGACAAGATA
GAGGAAGAGCAAAACAAAAGTAAGAAAAAAGCACAGCAAGCAGCAGC
TGACACAGGACACAGCAATCAGGTCAGCCAAAATTACCCTATAGTGCA
GAACATCCAGGGGCAAATGGTACATCAGGCCATATCACCTAGAACTTT
 
AAATGCATGGGTAAAAGTAGTAGAAGAGAAGGCTTTCAGCCCAGAAGT
GATACCCATGTTTTCAGCATTATCAGAAGGAGCCACCCCACAAGATTTA
AACACCATGCTAAACACAGTGGGGGGACATCAAGCAGCCATGCAAATG
TTAAAAGAGACCATCAATGAGGAAGCTGCAGAATGGGATAGAGTGCAT
CCAGTGCATGCAGGGCCTATTGCACCAGGCCAGATGAGAGAACCAAGG
GGATCAGACATCGCTGGAACTACTAGTACCCTTCAGGAACAAATAGGA
TGGATGACACATAATCCACCTATCCCAGTAGGAGAAATCTATAAAAGA
TGGATAATCCTGGGATTAAATAAAATAGTAAGAATGTATAGCCCTACC
AGCATTCTGGACATAAGACAAGGACCAAAGGAACCCTTTAGAGACTAT
GTAGACCGATTCTATAAAACTCTAAGAGCCGAGCAAGCTTCACAAGAG
GTAAAAAATTGGATGACAGAAACCTTGTTGGTCCAAAATGCGAACCCA
GATTGTAAGACTATTTTAAAAGCATTGGGACCAGGAGCGACACTAGAA
GAAATGATGACAGCATGTCAGGGAGTGGGGGGACCCGGCCATAAAGC
AAGAGTTTTGGCTGAAGCAATGAGCCAAGTAACAAATCCAGCTACCAT
AATGATACAGAAAGGCAATTTTAGGAACCAAAGAAAGACTGTTAAGTG
TTTCAATTGTGGCAAAGAAGGGCACATAGCCAAAAATTGCAGGGCCCC
TAGGAAAAAGGGCTGTTGGAAATGTGGAAAGGAAGGACACCAAATGA
AAGATTGTACTGAGAGACAGGCTAATTTTTTAGGGAAGATCTGGCCTTC
CCACAAGGGAAGGCCAGGGAATTTTCTTCAGAGCAGACCAGAGCCAAC
AGCCCCACCAGAAGAGAGCTTCAGGTTTGGGGAAGAGACAACAACTCC
CTCTCAGAAGCAGGAGCCGATAGACAAGGAACTGTATCCTTTAGCTTC
CCTCAGATCACTCTTTGGCAGCGACCCCTCGTCACAATAAAGATAGGG
GGGCAATTAAAGGAAGCTCTATTAGATACTGGTGCTGACGACACAGTA
TTAGAAGAAATGAATTTGCCAGGAAGATGGAAACCAAAAATGATAGG
GGGAATTGGAGGTTTTATCAAAGTAAGACAGTATGATCAGATACTCAT
AGAAATCTGCGGACATAAAGCTATAGGTACAGTATTAGTAGGACCTAC
ACCTGTCAACATAATTGGAAGAAATCTGTTGACTCAGATTGGCTGCACT
TTAAATTTTCCCATTAGTCCTATTGAGACTGTACCAGTAAAATTAAAGC
CAGGAATGGATGGCCCAAAAGTTAAACAATGGCCATTGACAGAAGAA
AAAATAAAAGCATTAGTAGAAATTTGTACAGAAATGGAAAAGGAAGG
AAAAATTTCAAAAATTGGGCCTGAAAATCCATACAATACTCCAGTATTT
GCCATAAAGAAAAAAGACAGTACTAAATGGAGAAAATTAGTAGATTTC
AGAGAACTTAATAAGAGAACTCAAGATTTCTGGGAAGTTCAATTAGGA
ATACCACATCCTGCAGGGTTAAAACAGAAAAAATCAGTAACAGTACTG
GATGTGGGCGATGCATATTTTTCAGTTCCCTTAGATAAAGACTTCAGGA
AGTATACTGCATTTACCATACCTAGTATAAACAATGAGACACCAGGGA
TTAGATATCAGTACAATGTGCTTCCACAGGGATGGAAAGGATCACCAG
CAATATTCCAGTGTAGCATGACAAAAATCTTAGAGCCTTTTAGAAAAC
AAAATCCAGACATAGTCATCTATCAATACATGGATGATTTGTATGTAGG
ATCTGACTTAGAAATAGGGCAGCATAGAACAAAAATAGAGGAACTGA
 
GACAACATCTGTTGAGGTGGGGATTTACCACACCAGACAAAAAACATC
AGAAAGAACCTCCATTCCTTTGGATGGGTTATGAACTCCATCCTGATAA
ATGGACAGTACAGCCTATAGTGCTGCCAGAAAAGGACAGCTGGACTGT
CAATGACATACAGAAATTAGTGGGAAAATTGAATTGGGCAAGTCAGAT
TTATGCAGGGATTAAAGTAAGGCAATTATGTAAACTTCTTAGGGGAAC
CAAAGCACTAACAGAAGTAGTACCACTAACAGAAGAAGCAGAGCTAG
AACTGGCAGAAAACAGGGAGATTCTAAAAGAACCGGTACATGGAGTGT
ATTATGACCCATCAAAAGACTTAATAGCAGAAATACAGAAGCAGGGGC
AAGGCCAATGGACATATCAAATTTATCAAGAGCCATTTAAAAATCTGA
AAACAGGAAAGTATGCAAGAATGAAGGGTGCCCACACTAATGATGTGA
AACAATTAACAGAGGCAGTACAAAAAATAGCCACAGAAAGCATAGTA
ATATGGGGAAAGACTCCTAAATTTAAATTACCCATACAAAAGGAAACA
TGGGAAGCATGGTGGACAGAGTATTGGCAAGCCACCTGGATTCCTGAG
TGGGAGTTTGTCAATACCCCTCCCTTAGTGAAGTTATGGTACCAGTTAG
AGAAAGAACCCATAATAGGAGCAGAAACTTTCTATGTAGATGGGGCAG
CCAATAGGGAAACTAAATTAGGAAAAGCAGGATATGTAACTGACAGA
GGAAGACAAAAAGTTGTCCCCCTAACGGACACAACAAATCAGAAGACT
GAGTTACAAGCAATTCATCTAGCTTTGCAGGATTCGGGATTAGAAGTA
AACATAGTGACAGACTCACAATATGCATTGGGAATCATTCAAGCACAA
CCAGATAAGAGTGAATCAGAGTTAGTCAGTCAAATAATAGAGCAGTTA
ATAAAAAAGGAAAAAGTCTACCTGGCATGGGTACCAGCACACAAAGG
AATTGGAGGAAATGAACAAGTAGATAAATTGGTCAGTGCTGGAATCAG
GAAAGTACTATTTTTAGATGGAATAGATAAGGCCCAAGAAGAACATGA
GAAATATCACAGTAATTGGAGAGCAATGGCTAGTGATTTTAACCTACC
ACCTGTAGTAGCAAAAGAAATAGTAGCCAGCTGTGATAAATGTCAGCT
AAAAGGGGAAGCCATGCATGGACAAGTAGACTGTAGCCCAGGAATAT
GGCAGCTAGATTGTACACATTTAGAAGGAAAAGTTATCTTGGTAGCAG
TTCATGTAGCCAGTGGATATATAGAAGCAGAAGTAATTCCAGCAGAGA
CAGGGCAAGAAACAGCATACTTCCTCTTAAAATTAGCAGGAAGATGGC
CAGTAAAAACAGTACATACAGACAATGGCAGCAATTTCACCAGTACTA
CAGTTAAGGCCGCCTGTTGGTGGGCGGGGATCAAGCAGGAATTTGGCA
TTCCCTACAATCCGCAGTCACAAGGAGTAATAGAATCTATGAATAAAG
AATTAAAGAAAATTATAGGACAGGTAAGAGATCAGGCTGAACATCTTA
AAACAGCAGTACAAATGGCAGTATTCATCCACAATTTTAAAAGAAAAG
GGGGGATTGGGGGGTACAGTGCAGGGGAAAGAATAGTAGACATAATA
GCAACAGACATACAAACTAAAGAATTACAAAAACAAATTACAAAAATT
CAAAATTTTCGGGTTTATTACAGGGACAGCAGAGATCCAGTTTGGAAA
GGACCAGCAAAGCTCCTCTGGAAAGGTGAAGGGGCAGTAGTAATACAA
GATAATAGTGACATAAAAGTAGTGCCAAGAAGAAAAGCAAAGATCAT
CAGGGATTATGGAAAACAGATGGCAGGTGATGATTGTGTGGCAAGTAG
 
ACAGGATGAGGATTAACACATGGAATTCCGGAGCGGCCGCAGGAGCTT
TGTTCCTTGGGTTCTTGGGAGCAGCAGGAAGCACTATGGGCGCAGCGT
CAATGACGCTGACGGTACAGGCCAGACAATTATTGTCTGGTATAGTGC
AGCAGCAGAACAATTTGCTGAGGGCTATTGAGGCGCAACAGCATCTGT
TGCAACTCACAGTCTGGGGCATCAAGCAGCTCCAGGCAAGAATCCTGG
CTGTGGAAAGATACCTAAAGGATCAACAGCTCCTGGGGATTTGGGGTT
GCTCTGGAAAACTCATTTGCACCACTGCTGTGCCTTGGAATGCTAGTTG
GAGTAATAAATCTCTGGAACAGATTTGGAATCACACGACCTGGATGGA
GTGGGACAGAGAAATTAACAATTACACAAGCTTCCGCGGAATTCACCC
CACCAGTGCAGGCTGCCTATCAGAAAGTGGTGGCTGGTGTGGCTAATG
CCCTGGCCCACAAGTATCACTAAGCTCGCTTTCTTGCTGTCCAATTTCTA
TTAAAGGTTCCTTTGTTCCCTAAGTCCAACTACTAAACTGGGGGATATT
ATGAAGGGCCTTGAGCATCTGGATTCTGCCTAATAAAAAACATTTATTT
TCATTGCAATGATGTATTTAAATTATTTCTGAATATTTTACTAAAAAGG
GAATGTGGGAGGTCAGTGCATTTAAAACATAAAGAAATGAAGAGCTAG
TTCAAACCTTGGGAAAATACACTATATCTTAAACTCCATGAAAGAAGG
TGAGGCTGCAAACAGCTAATGCACATTGGCAACAGCCCCTGATGCCTA
TGCCTTATTCATCCCTCAGAAAAGGATTCAAGTAGAGGCTTGATTTGGA
GGTTAAAGTTTTGCTATGCTGTATTTTACATTACTTATTGTTTTAGCTGT
CCTCATGAATGTCTTTTCACTACCCATTTGCTTATCCTGCATCTCTCAGC
CTTGACTCCACTCAGTTCTCTTGCTTAGAGATACCACCTTTCCCCTGAAG
TGTTCCTTCCATGTTTTACGGCGAGATGGTTTCTCCTCGCCTGGCCACTC
AGCCTTAGTTGTCTCTGTTGTCTTATAGAGGTCTACTTGAAGAAGGAAA
AACAGGGGGCATGGTTTGACTGTCCTGTGAGCCCTTCTTCCCTGCCTCC
CCCACTCACAGTGACCCGGAATCCCTCGACATGGCAGTCTAGCACTAGT
GCGGCCGCAGATCTGCTTCCTCGCTCACTGACTCGCTGCGCTCGGTCGT
TCGGCTGCGGCGAGCGGTATCAGCTCACTCAAAGGCGGTAATACGGTT
ATCCACAGAATCAGGGGATAACGCAGGAAAGAACATGTGAGCAAAAG
GCCAGCAAAAGGCCAGGAACCGTAAAAAGTCGACCATTACTTATTGTT
TTAGCTGTCCTCATGAATGTCTTTTCACTACCCATTTGCTTATCCTGCAT
CTCTCAGCCTTGACTCCACTCAGTTCTCTTGCTTAGAGATACCACCTTTC
CCCTGAAGTGTTCCTTCCATGTTTTACGGCGAGATGGTTTCTCCTCGCCT
GGCCACTCAGCCTTAGTTGTCTCTGTTGTCTTATAGAGGTCTACTTGAA
GAAGGAAAAACAGGGGGCATGGTTTGACTGTCCTGTGAGCCCTTCTTC
CCTGCCTCCCCCACTCACAGTGACCCGGAATCCCTCGACATGGCAGTCT
AGCACTAGTGCGGCCGCAGATCTGCTTCCTCGCTCACTGACTCGCTGCG
CTCGGTCGTTCGGCTGCGGCGAGCGGTATCAGCTCACTCAAAGGCGGT
AATACGGTTATCCACAGAATCAGGGGATAACGCAGGAAAGAACATGTG
AGCAAAAGGCCAGCAAAAGGCCAGGAACCGTAAAAA
   SEQ ID NO: 28 
ATGCCGGGGTTTTACGAGATTGTGATTAAGGTCCCCAGCGACCTTGACG
AGCATCTGCCCGGCATTTCTGACAGCTTTGTGAACTGGGTGGCCGAGAA
GGAATGGGAGTTGCCGCCAGATTCTGACATGGATCTGAATCTGATTGA
GCAGGCACCCCTGACCGTGGCCGAGAAGCTGCAGCGCGACTTTCTGAC
GGAATGGCGCCGTGTGAGTAAGGCCCCGGAGGCTCTTTTCTTTGTGCAA
TTTGAGAAGGGAGAGAGCTACTTCCACATGCACGTGCTCGTGGAAACC
ACCGGGGTGAAATCCATGGTTTTGGGACGTTTCCTGAGTCAGATTCGCG
AAAAACTGATTCAGAGAATTTACCGCGGGATCGAGCCGACTTTGCCAA
ACTGGTTCGCGGTCACAAAGACCAGAAATGGCGCCGGAGGCGGGAAC
AAGGTGGTGGATGAGTGCTACATCCCCAATTACTTGCTCCCCAAAACCC
AGCCTGAGCTCCAGTGGGCGTGGACTAATATGGAACAGTATTTAAGCG
CCTGTTTGAATCTCACGGAGCGTAAACGGTTGGTGGCGCAGCATCTGAC
GCACGTGTCGCAGACGCAGGAGCAGAACAAAGAGAATCAGAATCCCA
ATTCTGATGCGCCGGTGATCAGATCAAAAACTTCAGCCAGGTACATGG
AGCTGGTCGGGTGGCTCGTGGACAAGGGGATTACCTCGGAGAAGCAGT
GGATCCAGGAGGACCAGGCCTCATACATCTCCTTCAATGCGGCCTCCA
ACTCGCGGTCCCAAATCAAGGCTGCCTTGGACAATGCGGGAAAGATTA
TGAGCCTGACTAAAACCGCCCCCGACTACCTGGTGGGCCAGCAGCCCG
TGGAGGACATTTCCAGCAATCGGATTTATAAAATTTTGGAACTAAACG
GGTACGATCCCCAATATGCGGCTTCCGTCTTTCTGGGATGGGCCACGAA
AAAGTTCGGCAAGAGGAACACCATCTGGCTGTTTGGGCCTGCAACTAC
CGGGAAGACCAACATCGCGGAGGCCATAGCCCACACTGTGCCCTTCTA
CGGGTGCGTAAACTGGACCAATGAGAACTTTCCCTTCAACGACTGTGTC
GACAAGATGGTGATCTGGTGGGAGGAGGGGAAGATGACCGCCAAGGT
CGTGGAGTCGGCCAAAGCCATTCTCGGAGGAAGCAAGGTGCGCGTGGA
CCAGAAATGCAAGTCCTCGGCCCAGATAGACCCGACTCCCGTGATCGT
CACCTCCAACACCAACATGTGCGCCGTGATTGACGGGAACTCAACGAC
CTTCGAACACCAGCAGCCGTTGCAAGACCGGATGTTCAAATTTGAACTC
ACCCGCCGTCTGGATCATGACTTTGGGAAGGTCACCAAGCAGGAAGTC
AAAGACTTTTTCCGGTGGGCAAAGGATCACGTGGTTGAGGTGGAGCAT
GAATTCTACGTCAAAAAGGGTGGAGCCAAGAAAAGACCCGCCCCCAGT
GACGCAGATATAAGTGAGCCCAAACGGGTGCGCGAGTCAGTTGCGCAG
CCATCGACGTCAGACGCGGAAGCTTCGATCAACTACGCAGACAGGTAC
CAAAACAAATGTTCTCGTCACGTGGGCATGAATCTGATGCTGTTTCCCT
GCAGACAATGCGAGAGAATGAATCAGAATTCAAATATCTGCTTCACTC
ACGGACAGAAAGACTGTTTAGAGTGCTTTCCCGTGTCAGAATCTCAACC
CGTTTCTGTCGTCAAAAAGGCGTATCAGAAACTGTGCTACATTCATCAT
ATCATGGGAAAGGTGCCAGACGCTTGCACTGCCTGCGATCTGGTCAAT
GTGGATTTGGATGACTGCATCTTTGAACAATAAATGATTTAAATCAGGT
ATGGCTGCCGATGGTTATCTTCCAGATTGGCTCGAGGACAACCTCTCTG
 
AGGGCATTCGCGAGTGGTGGGCGCTGAAACCTGGAGCCCCGAAGCCCA
AAGCCAACCAGCAAAAGCAGGACGACGGCCGGGGTCTGGTGCTTCCTG
GCTACAAGTACCTCGGACCCTTCAACGGACTCGACAAGGGGGAGCCCG
TCAACGCGGCGGACGCAGCGGCCCTCGAGCACGACAAGGCCTACGACC
AGCAGCTGCAGGCGGGTGACAATCCGTACCTGCGGTATAACCACGCCG
ACGCCGAGTTTCAGGAGCGTCTGCAAGAAGATACGTCTTTTGGGGGCA
ACCTCGGGCGAGCAGTCTTCCAGGCCAAGAAGCGGGTTCTCGAACCTC
TCGGTCTGGTTGAGGAAGGCGCTAAGACGGCTCCTGGAAAGAAGAGAC
CGGTAGAGCCATCACCCCAGCGTTCTCCAGACTCCTCTACGGGCATCGG
CAAGAAAGGCCAACAGCCCGCCAGAAAAAGACTCAATTTTGGTCAGAC
TGGCGACTCAGAGTCAGTTCCAGACCCTCAACCTCTCGGAGAACCTCCA
GCAGCGCCCTCTGGTGTGGGACCTAATACAATGGCTGCAGGCGGTGGC
GCACCAATGGCAGACAATAACGAAGGCGCCGACGGAGTGGGTAGTTCC
TCGGGAAATTGGCATTGCGATTCCACATGGCTGGGCGACAGAGTCATC
ACCACCAGCACCCGAACCTGGGCCCTGCCCACCTACAACAACCACCTC
TACAAGCAAATCTCCAACGGGACATCGGGAGGAGCCACCAACGACAAC
ACCTACTTCGGCTACAGCACCCCCTGGGGGTATTTTGACTTTAACAGAT
TCCACTGCCACTTTTCACCACGTGACTGGCAGCGACTCATCAACAACAA
CTGGGGATTCCGGCCCAAGAGACTCAGCTTCAAGCTCTTCAACATCCAG
GTCAAGGAGGTCACGCAGAATGAAGGCACCAAGACCATCGCCAATAAC
CTCACCAGCACCATCCAGGTGTTTACGGACTCGGAGTACCAGCTGCCGT
ACGTTCTCGGCTCTGCCCACCAGGGCTGCCTGCCTCCGTTCCCGGCGGA
CGTGTTCATGATTCCCCAGTACGGCTACCTAACACTCAACAACGGTAGT
CAGGCCGTGGGACGCTCCTCCTTCTACTGCCTGGAATACTTTCCTTCGC
AGATGCTGAGAACCGGCAACAACTTCCAGTTTACTTACACCTTCGAGG
ACGTGCCTTTCCACAGCAGCTACGCCCACAGCCAGAGCTTGGACCGGC
TGATGAATCCTCTGATTGACCAGTACCTGTACTACTTGTCTCGGACTCA
AACAACAGGAGGCACGGCAAATACGCAGACTCTGGGCTTCAGCCAAGG
TGGGCCTAATACAATGGCCAATCAGGCAAAGAACTGGCTGCCAGGACC
CTGTTACCGCCAACAACGCGTCTCAACGACAACCGGGCAAAACAACAA
TAGCAACTTTGCCTGGACTGCTGGGACCAAATACCATCTGAATGGAAG
AAATTCATTGGCTAATCCTGGCATCGCTATGGCAACACACAAAGACGA
CGAGGAGCGTTTTTTTCCCAGTAACGGGATCCTGATTTTTGGCAAACAA
AATGCTGCCAGAGACAATGCGGATTACAGCGATGTCATGCTCACCAGC
GAGGAAGAAATCAAAACCACTAACCCTGTGGCTACAGAGGAATACGGT
ATCGTGGCAGATAACTTGCAGCAGCAAAACACGGCTCCTCAAATTGGA
ACTGTCAACAGCCAGGGGGCCTTACCCGGTATGGTCTGGCAGAACCGG
GACGTGTACCTGCAGGGTCCCATCTGGGCCAAGATTCCTCACACGGAC
GGCAACTTCCACCCGTCTCCGCTGATGGGCGGCTTTGGCCTGAAACATC
CTCCGCCTCAGATCCTGATCAAGAACACGCCTGTACCTGCGGATCCTCC
 
GACCACCTTCAACCAGTCAAAGCTGAACTCTTTCATCACGCAATACAGC
ACCGGACAGGTCAGCGTGGAAATTGAATGGGAGCTGCAGAAGGAAAA
CAGCAAGCGCTGGAACCCCGAGATCCAGTACACCTCCAACTACTACAA
ATCTACAAGTGTGGACTTTGCTGTTAATACAGAAGGCGTGTACTCTGAA
CCCCGCCCCATTGGCACCCGTTACCTCACCCGTAATCTGTAATTGCCTG
TTAATCAATAAACCGGTTGATTCGTTTCAGTTGAACTTTGGTCTCTGCG
AAGGGCGAATTCGTTTAAACCTGCAGGACTAGAGGTCCTGTATTAGAG
GTCACGTGAGTGTTTTGCGACATTTTGCGACACCATGTGGTCACGCTGG
GTATTTAAGCCCGAGTGAGCACGCAGGGTCTCCATTTTGAAGCGGGAG
GTTTGAACGCGCAGCCGCCAAGCCGAATTCTGCAGATATCCATCACACT
GGCGGCCGCTCGACTAGAGCGGCCGCCACCGCGGTGGAGCTCCAGCTT
TTGTTCCCTTTAGTGAGGGTTAATTGCGCGCTTGGCGTAATCATGGTCA
TAGCTGTTTCCTGTGTGAAATTGTTATCCGCTCACAATTCCACACAACA
TACGAGCCGGAAGCATAAAGTGTAAAGCCTGGGGTGCCTAATGAGTGA
GCTAACTCACATTAATTGCGTTGCGCTCACTGCCCGCTTTCCAGTCGGG
AAACCTGTCGTGCCAGCTGCATTAATGAATCGGCCAACGCGCGGGGAG
AGGCGGTTTGCGTATTGGGCGCTCTTCCGCTTCCTCGCTCACTGACTCG
CTGCGCTCGGTCGTTCGGCTGCGGCGAGCGGTATCAGCTCACTCAAAG
GCGGTAATACGGTTATCCACAGAATCAGGGGATAACGCAGGAAAGAAC
ATGTGAGCAAAAGGCCAGCAAAAGGCCAGGAACCGTAAAAAGGCCGC
GTTGCTGGCGTTTTTCCATAGGCTCCGCCCCCCTGACGAGCATCACAAA
AATCGACGCTCAAGTCAGAGGTGGCGAAACCCGACAGGACTATAAAGA
TACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGT
GGCGCTTTCTCATAGCTCACGCTGTAGGTATCTCAGTTCGGTGTAGGTC
GTTCGCTCCAAGCTGGGCTGTGTGCACGAACCCCCCGTTCAGCCCGACC
GCTGCGCCTTATCCGGTAACTATCGTCTTGAGTCCAACCCGGTAAGACA
CGACTTATCGCCACTGGCAGCAGCCACTGGTAACAGGATTAGCAGAGC
GAGGTATGTAGGCGGTGCTACAGAGTTCTTGAAGTGGTGGCCTAACTA
CGGCTACACTAGAAGAACAGTATTTGGTATCTGCGCTCTGCTGAAGCCA
GTTACCTTCGGAAAAAGAGTTGGTAGCTCTTGATCCGGCAAACAAACC
ACCGCTGGTAGCGGTGGTTTTTTTGTTTGCAAGCAGCAGATTACGCGCA
GAAAAAAAGGATCTCAAGAAGATCCTTTGATCTTTTCTACGGGGTCTGA
CGCTCAGTGGAACGAAAACTCACGTTAAGGGATTTTGGTCATGAGATT
ATCAAAAAGGATCTTCACCTAGATCCTTTTAAATTAAAAATGAAGTTTT
AAATCAATCTAAAGTATATATGAGTAAACTTGGTCTGACAGTTACCAAT
GCTTAATCAGTGAGGCACCTATCTCAGCGATCTGTCTATTTCGTTCATC
CATAGTTGCCTGACTCCCCGTCGTGTAGATAACTACGATACGGGAGGG
CTTACCATCTGGCCCCAGTGCTGCAATGATACCGCGAGACCCACGCTCA
CCGGCTCCAGATTTATCAGCAATAAACCAGCCAGCCGGAAGGGCCGAG
 
CGCAGAAGTGGTCCTGCAACTTTATCCGCCTCCATCCAGTCTATTAATT
GTTGCCGGGAAGCTAGAGTAAGTAGTTCGCCAGTTAATAGTTTGCGCA
ACGTTGTTGCCATTGCTACAGGCATCGTGGTGTCACGCTCGTCGTTTGG
TATGGCTTCATTCAGCTCCGGTTCCCAACGATCAAGGCGAGTTACATGA
TCCCCCATGTTGTGCAAAAAAGCGGTTAGCTCCTTCGGTCCTCCGATCG
TTGTCAGAAGTAAGTTGGCCGCAGTGTTATCACTCATGGTTATGGCAGC
ACTGCATAATTCTCTTACTGTCATGCCATCCGTAAGATGCTTTTCTGTGA
CTGGTGAGTACTCAACCAAGTCATTCTGAGAATAGTGTATGCGGCGAC
CGAGTTGCTCTTGCCCGGCGTCAATACGGGATAATACCGCGCCACATA
GCAGAACTTTAAAAGTGCTCATCATTGGAAAACGTTCTTCGGGGCGAA
AACTCTCAAGGATCTTACCGCTGTTGAGATCCAGTTCGATGTAACCCAC
TCGTGCACCCAACTGATCTTCAGCATCTTTTACTTTCACCAGCGTTTCTG
GGTGAGCAAAAACAGGAAGGCAAAATGCCGCAAAAAAGGGAATAAGG
GCGACACGGAAATGTTGAATACTCATACTCTTCCTTTTTCAATATTATT
GAAGCATTTATCAGGGTTATTGTCTCATGAGCGGATACATATTTGAATG
TATTTAGAAAAATAAACAAATAGGGGTTCCGCGCACATTTCCCCGAAA
AGTGCCACCTAAATTGTAAGCGTTAATATTTTGTTAAAATTCGCGTTAA
ATTTTTGTTAAATCAGCTCATTTTTTAACCAATAGGCCGAAATCGGCAA
AATCCCTTATAAATCAAAAGAATAGACCGAGATAGGGTTGAGTGTTGT
TCCAGTTTGGAACAAGAGTCCACTATTAAAGAACGTGGACTCCAACGT
CAAAGGGCGAAAAACCGTCTATCAGGGCGATGGCCCACTACGTGAACC
ATCACCCTAATCAAGTTTTTTGGGGTCGAGGTGCCGTAAAGCACTAAAT
CGGAACCCTAAAGGGAGCCCCCGATTTAGAGCTTGACGGGGAAAGCCG
GCGAACGTGGCGAGAAAGGAAGGGAAGAAAGCGAAAGGAGCGGGCG
CTAGGGCGCTGGCAAGTGTAGCGGTCACGCTGCGCGTAACCACCACAC
CCGCCGCGCTTAATGCGCCGCTACAGGGCGCGTCCCATTCGCCATTCAG
GCTGCGCAACTGTTGGGAAGGGCGATCGGTGCGGGCCTCTTCGCTATTA
CGCCAGCTGGCGAAAGGGGGATGTGCTGCAAGGCGATTAAGTTGGGTA
ACGCCAGGGTTTTCCCAGTCACGACGTTGTAAAACGACGGCCAGTGAG
CGCGCGTAATACGACTCACTATAGGGCGAATTGGGTACCGGGCCCCCC
CTCGATCGAGGTCGACGGTATCGGGGGAGCTCGCAGGGTCTCCATTTTG
AAGCGGGAGGTTTGAACGCGCAGCCGCC
   SEQ ID NO: 29

GGTACCCAACTCCATGCTTAACAGTCCCCAGGTACAGCCCACCCTGCGT
CGCAACCAGGAACAGCTCTACAGCTTCCTGGAGCGCCACTCGCCCTACT
TCCGCAGCCACAGTGCGCAGATTAGGAGCGCCACTTCTTTTTGTCACTT
GAAAAACATGTAAAAATAATGTACTAGGAGACACTTTCAATAAAGGCA
AATGTTTTTATTTGTACACTCTCGGGTGATTATTTACCCCCCACCCTTGC
CGTCTGCGCCGTTTAAAAATCAAAGGGGTTCTGCCGCGCATCGCTATGC
GCCACTGGCAGGGACACGTTGCGATACTGGTGTTTAGTGCTCCACTTAA

ACTCAGGCACAACCATCCGCGGCAGCTCGGTGAAGTTTTCACTCCACA
GGCTGCGCACCATCACCAACGCGTTTAGCAGGTCGGGCGCCGATATCTT
GAAGTCGCAGTTGGGGCCTCCGCCCTGCGCGCGCGAGTTGCGATACAC
AGGGTTGCAGCACTGGAACACTATCAGCGCCGGGTGGTGCACGCTGGC
CAGCACGCTCTTGTCGGAGATCAGATCCGCGTCCAGGTCCTCCGCGTTG
CTCAGGGCGAACGGAGTCAACTTTGGTAGCTGCCTTCCCAAAAAGGGT
GCATGCCCAGGCTTTGAGTTGCACTCGCACCGTAGTGGCATCAGAAGG
TGACCGTGCCCGGTCTGGGCGTTAGGATACAGCGCCTGCATGAAAGCC
TTGATCTGCTTAAAAGCCACCTGAGCCTTTGCGCCTTCAGAGAAGAACA
TGCCGCAAGACTTGCCGGAAAACTGATTGGCCGGACAGGCCGCGTCAT
GCACGCAGCACCTTGCGTCGGTGTTGGAGATCTGCACCACATTTCGGCC
CCACCGGTTCTTCACGATCTTGGCCTTGCTAGACTGCTCCTTCAGCGCG
CGCTGCCCGTTTTCGCTCGTCACATCCATTTCAATCACGTGCTCCTTATT
TATCATAATGCTCCCGTGTAGACACTTAAGCTCGCCTTCGATCTCAGCG
CAGCGGTGCAGCCACAACGCGCAGCCCGTGGGCTCGTGGTGCTTGTAG
GTTACCTCTGCAAACGACTGCAGGTACGCCTGCAGGAATCGCCCCATC
ATCGTCACAAAGGTCTTGTTGCTGGTGAAGGTCAGCTGCAACCCGCGGT
GCTCCTCGTTTAGCCAGGTCTTGCATACGGCCGCCAGAGCTTCCACTTG
GTCAGGCAGTAGCTTGAAGTTTGCCTTTAGATCGTTATCCACGTGGTAC
TTGTCCATCAACGCGCGCGCAGCCTCCATGCCCTTCTCCCACGCAGACA
CGATCGGCAGGCTCAGCGGGTTTATCACCGTGCTTTCACTTTCCGCTTC
ACTGGACTCTTCCTTTTCCTCTTGCGTCCGCATACCCCGCGCCACTGGGT
CGTCTTCATTCAGCCGCCGCACCGTGCGCTTACCTCCCTTGCCGTGCTTG
ATTAGCACCGGTGGGTTGCTGAAACCCACCATTTGTAGCGCCACATCTT
CTCTTTCTTCCTCGCTGTCCACGATCACCTCTGGGGATGGCGGGCGCTC
GGGCTTGGGAGAGGGGCGCTTCTTTTTCTTTTTGGACGCAATGGCCAAA
TCCGCCGTCGAGGTCGATGGCCGCGGGCTGGGTGTGCGCGGCACCAGC
GCATCTTGTGACGAGTCTTCTTCGTCCTCGGACTCGAGACGCCGCCTCA
GCCGCTTTTTTGGGGGCGCGCGGGGAGGCGGCGGCGACGGCGACGGGG
ACGACACGTCCTCCATGGTTGGTGGACGTCGCGCCGCACCGCGTCCGC
GCTCGGGGGTGGTTTCGCGCTGCTCCTCTTCCCGACTGGCCATTTCCTTC
TCCTATAGGCAGAAAAAGATCATGGAGTCAGTCGAGAAGGAGGACAG
CCTAACCGCCCCCTTTGAGTTCGCCACCACCGCCTCCACCGATGCCGCC
AACGCGCCTACCACCTTCCCCGTCGAGGCACCCCCGCTTGAGGAGGAG
GAAGTGATTATCGAGCAGGACCCAGGTTTTGTAAGCGAAGACGACGAG
GATCGCTCAGTACCAACAGAGGATAAAAAGCAAGACCAGGACGACGC
AGAGGCAAACGAGGAACAAGTCGGGCGGGGGGACCAAAGGCATGGCG
ACTACCTAGATGTGGGAGACGACGTGCTGTTGAAGCATCTGCAGCGCC
AGTGCGCCATTATCTGCGACGCGTTGCAAGAGCGCAGCGATGTGCCCC
TCGCCATAGCGGATGTCAGCCTTGCCTACGAACGCCACCTGTTCTCACC

GCGCGTACCCCCCAAACGCCAAGAAAACGGCACATGCGAGCCCAACCC
GCGCCTCAACTTCTACCCCGTATTTGCCGTGCCAGAGGTGCTTGCCACC
TATCACATCTTTTTCCAAAACTGCAAGATACCCCTATCCTGCCGTGCCA
ACCGCAGCCGAGCGGACAAGCAGCTGGCCTTGCGGCAGGGCGCTGTCA
TACCTGATATCGCCTCGCTCGACGAAGTGCCAAAAATCTTTGAGGGTCT
TGGACGCGACGAGAAACGCGCGGCAAACGCTCTGCAACAAGAAAACA
GCGAAAATGAAAGTCACTGTGGAGTGCTGGTGGAACTTGAGGGTGACA
ACGCGCGCCTAGCCGTGCTGAAACGCAGCATCGAGGTCACCCACTTTG
CCTACCCGGCACTTAACCTACCCCCCAAGGTTATGAGCACAGTCATGAG
CGAGCTGATCGTGCGCCGTGCACGACCCCTGGAGAGGGATGCAAACTT
GCAAGAACAAACCGAGGAGGGCCTACCCGCAGTTGGCGATGAGCAGCT
GGCGCGCTGGCTTGAGACGCGCGAGCCTGCCGACTTGGAGGAGCGACG
CAAGCTAATGATGGCCGCAGTGCTTGTTACCGTGGAGCTTGAGTGCATG
CAGCGGTTCTTTGCTGACCCGGAGATGCAGCGCAAGCTAGAGGAAACG
TTGCACTACACCTTTCGCCAGGGCTACGTGCGCCAGGCCTGCAAAATTT
CCAACGTGGAGCTCTGCAACCTGGTCTCCTACCTTGGAATTTTGCACGA
AAACCGCCTCGGGCAAAACGTGCTTCATTCCACGCTCAAGGGCGAGGC
GCGCCGCGACTACGTCCGCGACTGCGTTTACTTATTTCTGTGCTACACC
TGGCAAACGGCCATGGGCGTGTGGCAGCAATGCCTGGAGGAGCGCAAC
CTAAAGGAGCTGCAGAAGCTGCTAAAGCAAAACTTGAAGGACCTATGG
ACGGCCTTCAACGAGCGCTCCGTGGCCGCGCACCTGGCGGACATTATCT
TCCCCGAACGCCTGCTTAAAACCCTGCAACAGGGTCTGCCAGACTTCAC
CAGTCAAAGCATGTTGCAAAACTTTAGGAACTTTATCCTAGAGCGTTCA
GGAATTCTGCCCGCCACCTGCTGTGCGCTTCCTAGCGACTTTGTGCCCA
TTAAGTACCGTGAATGCCCTCCGCCGCTTTGGGGTCACTGCTACCTTCT
GCAGCTAGCCAACTACCTTGCCTACCACTCCGACATCATGGAAGACGT
GAGCGGTGACGGCCTACTGGAGTGTCACTGTCGCTGCAACCTATGCAC
CCCGCACCGCTCCCTGGTCTGCAATTCGCAACTGCTTAGCGAAAGTCAA
ATTATCGGTACCTTTGAGCTGCAGGGTCCCTCGCCTGACGAAAAGTCCG
CGGCTCCGGGGTTGAAACTCACTCCGGGGCTGTGGACGTCGGCTTACCT
TCGCAAATTTGTACCTGAGGACTACCACGCCCACGAGATTAGGTTCTAC
GAAGACCAATCCCGCCCGCCAAATGCGGAGCTTACCGCCTGCGTCATT
ACCCAGGGCCACATCCTTGGCCAATTGCAAGCCATCAACAAAGCCCGC
CAAGAGTTTCTGCTACGAAAGGGACGGGGGGTTTACCTGGACCCCCAG
TCCGGCGAGGAGCTCAACCCAATCCCCCCGCCGCCGCAGCCCTATCAG
CAGCCGCGGGCCCTTGCTTCCCAGGATGGCACCCAAAAAGAAGCTGCA
GCTGCCGCCGCCGCCACCCACGGACGAGGAGGAATACTGGGACAGTCA
GGCAGAGGAGGTTTTGGACGAGGAGGAGGAGATGATGGAAGACTGGG
ACAGCCTAGACGAAGCTTCCGAGGCCGAAGAGGTGTCAGACGAAACAC
CGTCACCCTCGGTCGCATTCCCCTCGCCGGCGCCCCAGAAATTGGCAAC

CGTTCCCAGCATCGCTACAACCTCCGCTCCTCAGGCGCCGCCGGCACTG
CCTGTTCGCCGACCCAACCGTAGATGGGACACCACTGGAACCAGGGCC
GGTAAGTCTAAGCAGCCGCCGCCGTTAGCCCAAGAGCAACAACAGCGC
CAAGGCTACCGCTCGTGGCGCGGGCACAAGAACGCCATAGTTGCTTGC
TTGCAAGACTGTGGGGGCAACATCTCCTTCGCCCGCCGCTTTCTTCTCT
ACCATCACGGCGTGGCCTTCCCCCGTAACATCCTGCATTACTACCGTCA
TCTCTACAGCCCCTACTGCACCGGCGGCAGCGGCAGCGGCAGCAACAG
CAGCGGTCACACAGAAGCAAAGGCGACCGGATAGCAAGACTCTGACA
AAGCCCAAGAAATCCACAGCGGCGGCAGCAGCAGGAGGAGGAGCGCT
GCGTCTGGCGCCCAACGAACCCGTATCGACCCGCGAGCTTAGAAATAG
GATTTTTCCCACTCTGTATGCTATATTTCAACAAAGCAGGGGCCAAGAA
CAAGAGCTGAAAATAAAAAACAGGTCTCTGCGCTCCCTCACCCGCAGC
TGCCTGTATCACAAAAGCGAAGATCAGCTTCGGCGCACGCTGGAAGAC
GCGGAGGCTCTCTTCAGCAAATACTGCGCGCTGACTCTTAAGGACTAGT
TTCGCGCCCTTTCTCAAATTTAAGCGCGAAAACTACGTCATCTCCAGCG
GCCACACCCGGCGCCAGCACCTGTCGTCAGCGCCATTATGAGCAAGGA
AATTCCCACGCCCTACATGTGGAGTTACCAGCCACAAATGGGACTTGC
GGCTGGAGCTGCCCAAGACTACTCAACCCGAATAAACTACATGAGCGC
GGGACCCCACATGATATCCCGGGTCAACGGAATCCGCGCCCACCGAAA
CCGAATTCTCCTCGAACAGGCGGCTATTACCACCACACCTCGTAATAAC
CTTAATCCCCGTAGTTGGCCCGCTGCCCTGGTGTACCAGGAAAGTCCCG
CTCCCACCACTGTGGTACTTCCCAGAGACGCCCAGGCCGAAGTTCAGAT
GACTAACTCAGGGGCGCAGCTTGCGGGCGGCTTTCGTCACAGGGTGCG
GTCGCCCGGGCGTTTTAGGGCGGAGTAACTTGCATGTATTGGGAATTGT
AGTTTTTTTAAAATGGGAAGTGACGTATCGTGGGAAAACGGAAGTGAA
GATTTGAGGAAGTTGTGGGTTTTTTGGCTTTCGTTTCTGGGCGTAGGTTC
GCGTGCGGTTTTCTGGGTGTTTTTTGTGGACTTTAACCGTTACGTCATTT
TTTAGTCCTATATATACTCGCTCTGTACTTGGCCCTTTTTACACTGTGAC
TGATTGAGCTGGTGCCGTGTCGAGTGGTGTTTTTTAATAGGTTTTTTTAC
TGGTAAGGCTGACTGTTATGGCTGCCGCTGTGGAAGCGCTGTATGTTGT
TCTGGAGCGGGAGGGTGCTATTTTGCCTAGGCAGGAGGGTTTTTCAGGT
GTTTATGTGTTTTTCTCTCCTATTAATTTTGTTATACCTCCTATGGGGGC
TGTAATGTTGTCTCTACGCCTGCGGGTATGTATTCCCCCGGGCTATTTCG
GTCGCTTTTTAGCACTGACCGATGTTAACCAACCTGATGTGTTTACCGA
GTCTTACATTATGACTCCGGACATGACCGAGGAACTGTCGGTGGTGCTT
TTTAATCACGGTGACCAGTTTTTTTACGGTCACGCCGGCATGGCCGTAG
TCCGTCTTATGCTTATAAGGGTTGTTTTTCCTGTTGTAAGACAGGCTTCT
AATGTTTAAATGTTTTTTTTTTTGTTATTTTATTTTGTGTTTAATGCAGGA
ACCCGCAGACATGTTTGAGAGAAAAATGGTGTCTTTTTCTGTGGTGGTT
CCGGAACTTACCTGCCTTTATCTGCATGAGCATGACTACGATGTGCTTG

CTTTTTTGCGCGAGGCTTTGCCTGATTTTTTGAGCAGCACCTTGCATTTT
ATATCGCCGCCCATGCAACAAGCTTACATAGGGGCTACGCTGGTTAGC
ATAGCTCCGAGTATGCGTGTCATAATCAGTGTGGGTTCTTTTGTCATGG
TTCCTGGCGGGGAAGTGGCCGCGCTGGTCCGTGCAGACCTGCACGATT
ATGTTCAGCTGGCCCTGCGAAGGGACCTACGGGATCGCGGTATTTTTGT
TAATGTTCCGCTTTTGAATCTTATACAGGTCTGTGAGGAACCTGAATTT
TTGCAATCATGATTCGCTGCTTGAGGCTGAAGGTGGAGGGCGCTCTGG
AGCAGATTTTTACAATGGCCGGACTTAATATTCGGGATTTGCTTAGAGA
CATATTGATAAGGTGGCGAGATGAAAATTATTTGGGCATGGTTGAAGG
TGCTGGAATGTTTATAGAGGAGATTCACCCTGAAGGGTTTAGCCTTTAC
GTCCACTTGGACGTGAGGGCAGTTTGCCTTTTGGAAGCCATTGTGCAAC
ATCTTACAAATGCCATTATCTGTTCTTTGGCTGTAGAGTTTGACCACGC
CACCGGAGGGGAGCGCGTTCACTTAATAGATCTTCATTTTGAGGTTTTG
GATAATCTTTTGGAATAAAAAAAAAAAAACATGGTTCTTCCAGCTCTTC
CCGCTCCTCCCGTGTGTGACTCGCAGAACGAATGTGTAGGTTGGCTGGG
TGTGGCTTATTCTGCGGTGGTGGATGTTATCAGGGCAGCGGCGCATGAA
GGAGTTTACATAGAACCCGAAGCCAGGGGGCGCCTGGATGCTTTGAGA
GAGTGGATATACTACAACTACTACACAGAGCGAGCTAAGCGACGAGAC
CGGAGACGCAGATCTGTTTGTCACGCCCGCACCTGGTTTTGCTTCAGGA
AATATGACTACGTCCGGCGTTCCATTTGGCATGACACTACGACCAACAC
GATCTCGGTTGTCTCGGCGCACTCCGTACAGTAGGGATCGCCTACCTCC
TTTTGAGACAGAGACCCGCGCTACCATACTGGAGGATCATCCGCTGCTG
CCCGAATGTAACACTTTGACAATGCACAACGTGAGTTACGTGCGAGGT
CTTCCCTGCAGTGTGGGATTTACGCTGATTCAGGAATGGGTTGTTCCCT
GGGATATGGTTCTGACGCGGGAGGAGCTTGTAATCCTGAGGAAGTGTA
TGCACGTGTGCCTGTGTTGTGCCAACATTGATATCATGACGAGCATGAT
GATCCATGGTTACGAGTCCTGGGCTCTCCACTGTCATTGTTCCAGTCCC
GGTTCCCTGCAGTGCATAGCCGGCGGGCAGGTTTTGGCCAGCTGGTTTA
GGATGGTGGTGGATGGCGCCATGTTTAATCAGAGGTTTATATGGTACCG
GGAGGTGGTGAATTACAACATGCCAAAAGAGGTAATGTTTATGTCCAG
CGTGTTTATGAGGGGTCGCCACTTAATCTACCTGCGCTTGTGGTATGAT
GGCCACGTGGGTTCTGTGGTCCCCGCCATGAGCTTTGGATACAGCGCCT
TGCACTGTGGGATTTTGAACAATATTGTGGTGCTGTGCTGCAGTTACTG
TGCTGATTTAAGTGAGATCAGGGTGCGCTGCTGTGCCCGGAGGACAAG
GCGTCTCATGCTGCGGGCGGTGCGAATCATCGCTGAGGAGACCACTGC
CATGTTGTATTCCTGCAGGACGGAGCGGCGGCGGCAGCAGTTTATTCGC
GCGCTGCTGCAGCACCACCGCCCTATCCTGATGCACGATTATGACTCTA
CCCCCATGTAGGCGTGGACTTCCCCTTCGCCGCCCGTTGAGCAACCGCA
AGTTGGACAGCAGCCTGTGGCTCAGCAGCTGGACAGCGACATGAACTT
AAGCGAGCTGCCCGGGGAGTTTATTAATATCACTGATGAGCGTTTGGCT

CGACAGGAAACCGTGTGGAATATAACACCTAAGAATATGTCTGTTACC
CATGATATGATGCTTTTTAAGGCCAGCCGGGGAGAAAGGACTGTGTAC
TCTGTGTGTTGGGAGGGAGGTGGCAGGTTGAATACTAGGGTTCTGTGA
GTTTGATTAAGGTACGGTGATCAATATAAGCTATGTGGTGGGGCTA
TACTACTGAATGAAAAATGACTTGAAATTTTCTGCAATTGAAAAATAA
ACACGTTGAAACATAACATGCAACAGGTTCACGATTCTTTATTCCTGGG
CAATGTAGGAGAAGGTGTAAGAGTTGGTAGCAAAAGTTTCAGTGGTGT
ATTTTCCACTTTCCCAGGACCATGTAAAAGACATAGAGTAAGTGCTTAC
CTCGCTAGTTTCTGTGGATTCACTAGAATCGATGTAGGATGTTGCCCCT
CCTGACGCGGTAGGAGAAGGGGAGGGTGCCCTGCATGTCTGCCGCTGC
TCTTGCTCTTGCCGCTGCTGAGGAGGGGGGCGCATCTGCCGCAGCACCG
GATGCATCTGGGAAAAGCAAAAAAGGGGCTCGTCCCTGTTTCCGGAGG
AATTTGCAAGCGGGGTCTTGCATGACGGGGAGGCAAACCCCCGTTCGC
CGCAGTCCGGCCGGCCCGAGACTCGAACCGGGGGTCCTGCGACTCAAC
CCTTGGAAAATAACCCTCCGGCTACAGGGAGCGAGCCACTTAATGCTTT
CGCTTTCCAGCCTAACCGCTTACGCCGCGCGCGGCCAGTGGCCAAAAA
AGCTAGCGCAGCAGCCGCCGCGCCTGGAAGGAAGCCAAAAGGAGCGC
TCCCCCGTTGTCTGACGTCGCACACCTGGGTTCGACACGCGGGCGGTAA
CCGCATGGATCACGGCGGACGGCCGGATCCGGGGTTCGAACCCCGGTC
GTCCGCCATGATACCCTTGCGAATTTATCCACCAGACCACGGAAGAGT
GCCCGCTTACAGGCTCTCCTTTTGCACGGTCTAGAGCGTCAACGACTGC
GCACGCCTCACCGGCCAGAGCGTCCCGACCATGGAGCACTTTTTGCCGC
TGCGCAACATCTGGAACCGCGTCCGCGACTTTCCGCGCGCCTCCACCAC
CGCCGCCGGCATCACCTGGATGTCCAGGTACATCTACGGATTACGTCGA
CGTTTAAACCATATGATCAGCTCACTCAAAGGCGGTAATACGGTTATCC
ACAGAATCAGGGGATAACGCAGGAAAGAACATGTGAGCAAAAGGCCA
GCAAAAGGCCAGGAACCGTAAAAAGGCCGCGTTGCTGGCGTTTTTCCA
TAGGCTCCGCCCCCCTGACGAGCATCACAAAAATCGACGCTCAAGTCA
GAGGTGGCGAAACCCGACAGGACTATAAAGATACCAGGCGTTTCCCCC
TGGAAGCTCCCTCGTGCGCTCTCCTGTTCCGACCCTGCCGCTTACCGGA
TACCTGTCCGCCTTTCTCCCTTCGGGAAGCGTGGCGCTTTCTCATAGCTC
ACGCTGTAGGTATCTCAGTTCGGTGTAGGTCGTTCGCTCCAAGCTGGGC
TGTGTGCACGAACCCCCCGTTCAGCCCGACCGCTGCGCCTTATCCGGTA
ACTATCGTCTTGAGTCCAACCCGGTAAGACACGACTTATCGCCACTGGC
AGCAGCCACTGGTAACAGGATTAGCAGAGCGAGGTATGTAGGCGGTGC
TACAGAGTTCTTGAAGTGGTGGCCTAACTACGGCTACACTAGAAGAAC
AGTATTTGGTATCTGCGCTCTGCTGAAGCCAGTTACCTTCGGAAAAAGA
GTTGGTAGCTCTTGATCCGGCAAACAAACCACCGCTGGTAGCGGTGGTT
TTTTTGTTTGCAAGCAGCAGATTACGCGCAGAAAAAAAGGATCTCAAG
AAGATCCTTTGATCTTTTCTACGGGGTCTGACGCTCAGTGGAACGAAAA

CTCACGTTAAGGGATTTTGGTCATGAGATTATCAAAAAGGATCTTCACC
TAGATCCTTTTAAATTAAAAATGAAGTTTTAAATCAATCTAAAGTATAT
ATGAGTAAACTTGGTCTGACAGTTACCAATGCTTAATCAGTGAGGCACC
TATCTCAGCGATCTGTCTATTTCGTTCATCCATAGTTGCCTGACTCCCCG
TCGTGTAGATAACTACGATACGGGAGGGCTTACCATCTGGCCCCAGTG
CTGCAATGATACCGCGAGACCCACGCTCACCGGCTCCAGATTTATCAGC
AATAAACCAGCCAGCCGGAAGGGCCGAGCGCAGAAGTGGTCCTGCAA
CTTTATCCGCCTCCATCCAGTCTATTAATTGTTGCCGGGAAGCTAGAGT
AAGTAGTTCGCCAGTTAATAGTTTGCGCAACGTTGTTGCCATTGCTACA
GGCATCGTGGTGTCACGCTCGTCGTTTGGTATGGCTTCATTCAGCTCCG
GTTCCCAACGATCAAGGCGAGTTACATGATCCCCCATGTTGTGCAAAA
AAGCGGTTAGCTCCTTCGGTCCTCCGATCGTTGTCAGAAGTAAGTTGGC
CGCAGTGTTATCACTCATGGTTATGGCAGCACTGCATAATTCTCTTACT
GTCATGCCATCCGTAAGATGCTTTTCTGTGACTGGTGAGTACTCAACCA
AGTCATTCTGAGAATAGTGTATGCGGCGACCGAGTTGCTCTTGCCCGGC
GTCAATACGGGATAATACCGCGCCACATAGCAGAACTTTAAAAGTGCT
CATCATTGGAAAACGTTCTTCGGGGCGAAAACTCTCAAGGATCTTACCG
CTGTTGAGATCCAGTTCGATGTAACCCACTCGTGCACCCAACTGATCTT
CAGCATCTTTTACTTTCACCAGCGTTTCTGGGTGAGCAAAAACAGGAAG
GCAAAATGCCGCAAAAAAGGGAATAAGGGCGACACGGAAATGTTGAA
TACTCATACTCTTCCTTTTTCAATATTATTGAAGCATTTATCAGGGTTAT
TGTCTCATGAGCGGATACATATTTGAATGTATTTAGAAAAATAAACAA
ATAGGGGTTCCGCGCACATTTCCCCGAAAAGTGCCACCTAAATTGTAA
GCGTTAATATTTTGTTAAAATTCGCGTTAAATTTTTGTTAAATCAGCTCA
TTTTTTAACCAATAGGCCGAAATCGGCAAAATCCCTTATAAATCAAAAG
AATAGACCGAGATAGGGTTGAGTGTTGTTCCAGTTTGGAACAAGAGTC
CACTATTAAAGAACGTGGACTCCAACGTCAAAGGGCGAAAAACCGTCT
ATCAGGGCGATGGCCCACTACGTGAACCATCACCCTAATCAAGTTTTTT
GGGGTCGAGGTGCCGTAAAGCACTAAATCGGAACCCTAAAGGGAGCCC
CCGATTTAGAGCTTGACGGGGAAAGCCGGCGAACGTGGCGAGAAAGG
AAGGGAAGAAAGCGAAAGGAGCGGGCGCTAGGGCGCTGGCAAGTGTA
GCGGTCACGCTGCGCGTAACCACCACACCCGCCGCGCTTAATGCGCCG
CTACAGGGCGCGATGGATCC

## Claims

1. A gene sequence construct for gene therapy of HIV infection, comprising one or more gene coding sequences of an antibody molecule with the ability to inhibit HIV infection and one or more gene coding sequences of a polypeptide (consisting of 2-50 amino acid residues) with the ability to inhibit HIV infection, to achieve the expression of a fusion protein molecule comprising the antibody molecule and the polypeptide against HIV infection encoded by a single gene, which has two or more than two target sites.

2. The gene sequence construct according to claim 1, wherein the antibody molecule comprises a heavy chain constant region and/or a light chain constant region.

3. The gene sequence construct according to claim 2, wherein the heavy chain constant region comprises a heavy chain constant region of IgG1, IgG2, IgG3 or IgG4.

4. The gene sequence construct according to claim 2 or 3, wherein the light chain constant region comprises a light chain constant region of a kappa or lambda light chain.

5. The gene sequence construct according to any one of claims 1-4, comprising two or more than two gene coding sequences of the antibody molecule with the ability to inhibit HIV infection.

6. The gene sequence construct according to any one of claims 1-5, comprising three or more than three gene coding sequences of the antibody molecule with the ability to inhibit HIV infection.

7. The gene sequence construct according to any one of claims 1-6, comprising four or more than four gene coding sequences of the antibody molecule with the ability to inhibit HIV infection.

8. The gene sequence construct according to any one of claims 1-7, comprising two or more than two gene coding sequences of the polypeptide with the ability to inhibit HIV infection.

9. The gene sequence construct according to any one of claims 1-8, comprising three or more than three gene coding sequences of the polypeptide with the ability to inhibit HIV infection.

10. The gene sequence construct according to any one of claims 1-9, comprising four or more than four gene coding sequences of the polypeptide with the ability to inhibit HIV infection.

11. The gene sequence construct according to any one of claims 1-10, wherein the fusion protein molecule comprising the antibody molecule and the polypeptide against HIV infection encoded by the single gene has three or more than three target sites.

12. The gene sequence construct according to any one of claims 1-11, wherein the fusion protein molecule comprising the antibody molecule and the polypeptide against HIV infection encoded by the single gene has four or more than four target sites.

13. The gene sequence construct according to any one of claims 1-12, wherein the fusion protein molecule comprising the antibody molecule and the polypeptide against HIV infection encoded by the single gene has five or more than five target sites.

14. The gene sequence construct according to any one of claims 1-13, wherein the fusion protein molecule comprising the antibody molecule and the polypeptide against HIV infection encoded by the single gene has six or more than six target sites.

15. The gene sequence construct according to any one of claims 1-14, comprising two or more than two gene coding sequences of the antibody molecule with the ability to inhibit HIV infection and one or more gene coding sequences of the polypeptide with the ability to inhibit HIV infection.

16. The gene sequence construct according to claim 15, wherein the fusion protein molecule comprising the antibody molecule and the polypeptide against HIV infection encoded by the single gene has three or more than three target sites.

17. The gene sequence construct according to any one of claims 1-16, wherein the gene coding sequence of the antibody molecule with the ability to inhibit HIV infection and the gene coding sequence of the polypeptide with the ability to inhibit HIV infection are directly or indirectly concatenated via a coding sequence of a linker polypeptide.

18. The gene sequence construct according to any one of claims 1-17, comprising two or more than two gene coding sequences of antibody molecules with the ability to inhibit HIV infection, wherein the gene coding sequences of the antibody molecules are directly or indirectly concatenated via a coding sequence of a linker polypeptide.

19. The gene sequence construct according to any one of claims 1-18, comprising two or more than two gene coding sequences of polypeptides with the ability to inhibit HIV infection, wherein the gene coding sequences of the polypeptides are directly or indirectly concatenated via a coding sequence of a linker polypeptide.

20. The gene sequence construct according to any one of claims 1-19, wherein the one or more gene coding sequence of the antibody molecule with the ability to inhibit HIV infection comprises a gene coding sequence of an anti-HIV-1-gp160 (or its cleavage products gp120 and gp41) antibody molecule.

21. The gene sequence construct according to any one of claims 1-20, wherein the one or more gene coding sequence of the antibody molecule with the ability to inhibit HIV infection comprises a gene coding sequence of an antibody molecule that binds to human CD4 receptor site.

22. The gene sequence construct according to any one of claims 1-21, wherein the one or more gene coding sequence of the polypeptide with the ability to inhibit HIV infection comprises a gene coding sequence of a polypeptide that inhibits the fusion of HIV and CD4+ T cell membranes.

23. The gene sequence construct according to any one of claims 1-21, comprising two or more than two gene coding sequences of the antibody molecule with the ability to inhibit HIV infection and one or more gene coding sequences of the polypeptide with the ability to inhibit HIV infection, wherein the two or more than two gene coding sequences of the antibody molecules with the ability to inhibit HIV infection comprise a gene coding sequence of an antibody molecule against HIV-1-gp160 (or its cleavage products gp120 and gp41) and a gene coding sequence of an antibody molecule that binds to human CD4 receptor site, and wherein the one or more gene coding sequences of the polypeptide with the ability to inhibit HIV infection comprise a gene coding sequence of a polypeptide that inhibits the fusion of HIV and CD4+ T cell membranes.

24. The gene sequence construct according to any one of claims 1-23, comprising (i) gene coding sequences of light chain complementarity determining regions (LCDR1, LCDR2 and LCDR3) and heavy chain complementarity determining regions (HCDR1, HCDR2 and HCDR3) of an anti-HIV-1-gp 160 (or its cleavage products gp120 and gp41) monoclonal antibody, (ii) gene coding sequences of light chain complementarity determining regions (LCDR1, LCDR2 and LCDR3) and heavy chain complementarity determining regions (HCDR1, HCDR2 and HCDR3) of a monoclonal antibody that binds to human CD4 receptor site, (iii) a gene coding sequence of human IgG Fc fragment, and (iv) a gene coding sequence of a polypeptide that inhibits the fusion of HIV and CD4+ T cell membranes, wherein the coding sequences of the antibodies can be directly or indirectly concatenated via a coding sequence of a linker polypeptide in any order.

25. The gene sequence construct according to any one of claims 1-24, comprising (i) gene coding sequences of light chain variable region (VL) and heavy chain variable region (VH) of an anti-HIV-1-gp160 (including its cleavage products gp120 and gp41) monoclonal antibody, (ii) gene coding sequences of light chain variable region (VL) and heavy chain variable region (VH) of a monoclonal antibody that binds to human CD4 receptor site, (iii) a gene coding sequence of human IgG Fc fragment, and (iv) a gene coding sequence of a polypeptide that inhibits the fusion of HIV and CD4+ T cell membranes, wherein the coding sequences of the antibodies can be directly or indirectly concatenated via a coding sequence of a linker polypeptide in any order.

26. The gene sequence construct according to any one of claims 1-25, further comprising a promoter located upstream of the gene coding sequence of the antibody molecule with the ability to inhibit HIV infection and the gene coding sequence of the polypeptide with the ability to inhibit HIV infection.

27. The gene sequence construct according to any one of claims 1-26, further comprising a secretion signal peptide coding sequence located upstream of the gene coding sequence of the antibody molecule with the ability to inhibit HIV infection and the gene coding sequence of the polypeptide with the ability to inhibit HIV infection.

28. The gene sequence construct according to any one of claims 1-27, comprising a first gene coding sequence of an antibody molecule with the ability to inhibit HIV infection, a second gene coding sequence of an antibody molecule with the ability to inhibit HIV infection, and a gene coding sequence of a polypeptide with the ability to inhibit HIV infection, and is selected from:
VL2-linker-VH2-linker-VL1-linker-VH1-linker-CH2-CH3; or
VL2-linker-VH2-linker-VL1-linker-VH1-linker-CH2-CH3-linker-peptide inhibitor; or
other constructs that are constructed by arranging the combination of VL2 and VH2 or VL1 and VH1 in different orders in a construct;
wherein VL2 and VH2 are the variable region fragments of light chain and heavy chain of the first antibody molecule respectively; VL1 and VH1 are the variable region fragments of light chain and heavy chain of the second antibody molecule respectively; CH2-CH3 is Fc fragment of human IgG constant region, and the linker is a linker polypeptide; the peptide inhibitor is a polypeptide that inhibits HIV infection (for example, a polypeptide that inhibits the fusion of HIV and CD4+ T cell membranes).

29. The gene sequence construct according to claim 28, which is VL2-linker-VH2-linker-VL1-linker-VH1-linker-CH2-CH3, or a construct in which the combinations of VL2 and VH2 or VL1 and VH1 are arranged in different orders.

30. The gene sequence construct according to claim 28, which is VL2-linker-VH2-linker-VL1-linker-VH1-linker-CH2-CH3-linker-peptide inhibitor, or a construct in which the combinations of VL2 and VH2 or VL1 and VH1 are arranged in different orders.

31. The gene sequence construct according to claims 28, 29 and 30, wherein the protein sequences of VL2 and VH2 include SEQ ID NO: 2, or a functional fragment thereof, or a homologous sequence that is at least 75%, 80%, 85%, 90%, 95%, 98%, or 99% identical thereto.

32. The gene sequence construct according to claims 28, 29 and 30, wherein the protein sequences of VL1 and VH1 include SEQ ID NO: 3, or a functional fragment thereof, or a homologous sequence that is at least 75%, 80%, 85%, 90%, 95%, 98%, or 99% identical thereto.

33. The gene sequence construct according to claims 28, 29 and 30, wherein the sequence of the linker polypeptide (linker) is selected from: GGGGS, (GGGGS)₂, (GGGGS)₃, (GGGGS)₄, (GGGGS)₅, (GGGGS)₆ and (GGGGS)₇, or other optional linker polypeptide sequences.

34. The gene sequence construct according to claims 28 and 30, wherein the polypeptide (peptide inhibitor) that inhibits the fusion of HIV and CD4+ T cell membranes can be selected from the group consisting of membrane fusion inhibitory polypeptides P52, C34, T20, etc.

35. The gene sequence construct according to claim 34, wherein the sequence of the membrane fusion inhibitory polypeptide P52 includes SEQ ID NO: 5 or a homologous sequence that is at least 75%, 80%, 85%, 90%, 95%, 98%, or 99% identical thereto; the polypeptide sequence of C34 includes SEQ ID NO: 6 or a homologous sequence that is at least 75%, 80%, 85%, 90%, 95%, 98%, or 99% identical thereto; the polypeptide sequence of T20 includes SEQ ID NO: 7 or a homologous sequence that is at least 75%, 80%, 85%, 90%, 95%, 98%, or 99% identical thereto.

36. A viral vector genome comprising the construct of any one of the preceding claims.

37. A viral vector system comprising the genome according to claim 36.

38. The viral vector system according to claim 37, which is a lentiviral vector system or an adeno-associated virus vector system.

39. The lentiviral vector system according to claim 38, comprising the genome according to claim 36 and other nucleotide sequences encoding and expressing packaging components required for the production of lentivirus, which are introduced into production cells to produce a lentiviral particle containing the genome according to claim 36.

40. The adeno-associated virus vector system according to claim 38, comprising the genome according to claim 36 and other nucleotide sequences encoding and expressing packaging components required for the production of adeno-associated virus, which are introduced into production cells to produce an adeno-associated virus particle containing the genome according to claim 36.

41. A viral particle comprising the genome of the construct of any one of claims 1-35.

42. A pharmaceutical composition, comprising the viral particle according to claim 41, and a pharmaceutically acceptable carrier or diluent, or cells transduced by the lentiviral particle according to claim 39 in vitro, including but not limited to transduced muscle cells, liver cells, or CD4+ T cells.

43. The virus particle according to claims 39-41 or the pharmaceutical composition according to claim 42, for use in injection into the body to express an antibody-like molecule protein with two or more than two target sites, and the mature molecule is a dimer formed by disulfide bonds, which can effectively and broadly block infection of HIV against human CD4+ T cells by binding to multiple binding sites involved in different steps of HIV infection against human CD4+ T cells, and can be used for gene therapy of HIV infection, thereby achieving long-term treatment for a HIV-infected individual.

44. A method of inhibiting HIV infection, comprising administering to cells the viral particle or the pharmaceutical composition according to claims 39-42.

45. The method according to claim 44, wherein the cells comprise muscle cells, liver cells, or CD4+ T cells.

46. The method according to claim 44, wherein the method comprises transducing the cells of claim 45 in vitro or in vivo with the viral particle or the pharmaceutical composition according to claims 39-42.

47. A method of treating HIV infection in a subject in need thereof, wherein the method comprises administering to the subject a therapeutically effective dose of the viral particle or the pharmaceutical composition of claims 39-42.

48. The method according to claim 47, wherein the subject includes an earlystage HIV infector, a HIV-infected individual who is already received cocktail drug therapy, or a HIV-infected individual who is resistant to cocktail drug therapy.

49. The method according to claim 43, wherein the viral particle or the pharmaceutical composition according to claims 39-42 is injected intramuscularly.

50. The method according to claim 43, wherein the viral particle or the pharmaceutical composition according to claims 39-42 or CD4+ T cells transduced thereby is injected intravenously.

51. The virus particle and the pharmaceutical composition injected into the body according to the method of claims 49 and 50, which can express anti-HIV protein molecules with multiple targets sites and secrete them into blood, which can act on multiple nodes of HIV infection, and can effectively block HIV infection path and effectively avoid the loss of the ability to inhibit HIV infection due to escape mutations of HIV, thereby achieving a long-term (for example, therapeutically effective lasts for one or several years) or even permanent therapeutic effect on HIV infection with a single injection.
